# EUROPEAN PATENT SPECIFICATION

(11) **EP 0 556 171 B1**
(45) Date of publication and mention of the grant of the patent: **09.08.2000**
(21) Application number: 90917592.9
(22) Date of filing: 12.10.1990
(51) Int. Cl.: C12N 5/00, C12N 15/18, A61K 38/27, A61K 48/00, C07K 14/00

(54) **GROWTH HORMONE ANTAGONISTS**
ANTAGONISTEN VON WACHSTUMSHORMONEN
ANTAGONISTES D'HORMONES DE CROISSANCE

(30) Priority: 12.10.1989 US 419561
(43) Date of publication of application: 25.08.1993
(73) Proprietor: OHIO UNIVERSITY, Athens, OH 45701 (US)
(72) Inventor: KOPCHICK, John, J., Athens, OH 45701 (US); CHEN, Wen, Y., Athens, OH 45701 (US)
(74) Representative: Plougmann, Vingtoft & Partners A/S
(86) International application number: US9005874
(87) International publication number: WO9105853

(56) References cited:
- WO-A-91/00870
- PROTEIN ENGINEERING, 1987, Alan R. Liss Inc., New York, NY (US); pp. 193-199
- SCIENCE, vol. 243, 10 March 1989; CUNNINGHAM et al., pp. 1330-1336
- NATURE, vol. 315, 20 June 1985; HAMMER et al., pp. 680-683
- PROCEEDINGS OF THE NATL. ACADEMY OF SCIENCES USA, vol. 87, July 1990, Washington, DC (US); CHEN et al., pp. 5061-5065
- PROCEEDINGS OF THE NATL. ACADEMY OF SCIENCES USA, vol. 85, May 1988, Washington, DC (US); BREMS et al., pp. 3367-3371
- BIOCHEMISTRY, vol. 26, no. 24, 1987; BREMS et al., 7774-7778
- BIOCHEMICAL & BIOPHYSICAL RESERAHC COMMUNICATIONS, vol. 139, no. 2, 16 September 1986; TOU et al., pp. 763-770
- NATURE, vol. 300, 16 December 1982; PALMITER et al., pp. 611-615
- SCIENCE, vol. 222, 18 November 1983; PALMITER et al., pp. 809-814

## Description

### BACKGROUND OF THE INVENTION

### Field of the Invention

This invention relates to muteins of growth hormone, especially bovine growth hormone, which muteins inhibit the growth of animals. These analogues may be expressed in transgenic animals which thereby acquire a "reduced growth" phenotype.

### Information Disclosure Statement

Bovine, growth hormone (GH) is a protein of 191 amino acids that is naturally synthesized in the anterior pituitary. The molecular weight of the mature protein is about 22,000 daltons, but it is initially made as a pre-growth hormone with an extra 26 amino acids on the amino terminus. This leader (or signal peptide) is normally cleaved during secretion of the hormone by bovine pituitary cells. Several forms of the mature protein have been found in nature. The N-terminus can vary (due to variation in the site of cleavage during secretion) so that the mature protein begins with either NH₂-Ala-Phe-Pro or NH₂-Phe-Pro. Additionally, the amino acid at position 126 may be either leucine or valine, apparently as a result of allelic variation in the bovine population.

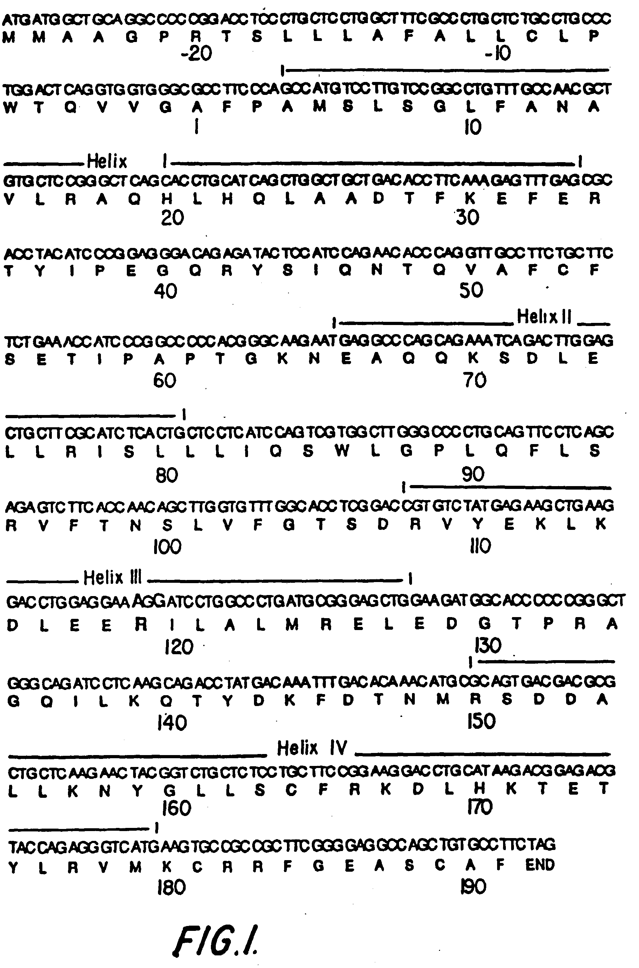

Bovine growth hormone (bGH) as referred to herein with reference to the present invention is the naturally occurring 191 amino acid form of bovine growth hormone whose amino acid sequence is identical to that of the bovine growth hormone mutant set forth in Figure 1 but for the presence of a glycine residue instead of the arginine residue shown at position 119 of the mutant sequence.

Exogenous administration of bGH to cattle increases milk production, feed efficiency, growth rate, and the lean-to-fat ratio, and decreases fattening time.

bGH has been produced by recombinant DNA techniques, see e.g., Fraser, U.S. 4,443,539 (yeast); Buell, EP Appl. 103,395 (bacteria); Krivl, EP Appl. 193,515 (bacteria); Kopchick, EP Appl. 161,640 (encapsulated mouse cells implanted into animals); DeBoer, EP Appl. 75,444 (bacteria; gene modified to eliminate harmful secondary structure) and this has facilitated the production of analogues of bGH by site-specific mutagenesis. Thus, Aviv, GB 2,073,245 describes production of Met Pro (des Ala) bGH, Met Arg (des Ala) bGH, Met-Glu-Gly (des Ala) bGH, and des (Ala¹-Phe²-Pro³-Ala⁴) bGH in E. coli. Brems, et al., PNAS (USA) 85:3367-71 (1988) reported preparation of the bGH mutant K112L, which extended the hydrophobic face of the third alpha helix of bGH. The 96-133 fragment of this mutant was also prepared. The biological activity of proteolytic fragments of bGH has also been studied. Brems, et al., Biochemistry, 26:7774 (1987); Swislocki, et al., Endocrinology, 87:900 (1970); Paladini, et al., TIBS, 256 (Nov. 1979). The fragment of bGH containing amino acids 96-133 is superior in growth promoting assays to bGH 1-95 and bGH 151-191. Hara, et al., Biochemistry, 17:550 (1978); Sonenberg, U.S. Patent Nos. 3,664,925 and 4,056,520; Chen and Sonenberg, J. Biol. Chem., 250:2510-14 (1977).

Similar results were observed with the fragment bGH (96-133). Graf, et al., Eur. J. Biochem., 64:333-340 (1976); Hara, et al., Biochem., 17:550-56 (1978). The information obtained through analyses of peptide fragments is of uncertain value since the native conformation of the molecule may be dramatically disrupted.

Analogues of bGH have varied in growth-promoting activity, as have the known analogues of other growth hormones. However, a growth hormone analogue having growth-inhibitory activity has not previously been reported.

A variety of transgenic animals have been produced. Hammer, et al., Nature, 315:680-683 (1985) (rabbits, sheep and pigs). Certain of these animals have been caused to express a growth hormone, and increased growth of such transgenic animals has been reported. Palmiter, et al., Nature 300:611 (1982) microinjected the male pronucleus of fertilized mouse eggs with a DNA fragment containing the promoter of the mouse metallothionein-I gene fused to the structural gene of rat growth hormone. Several of the transgenic mice developed from the genetically modified zygote exhibited a growth rate substantially higher than that of control mice. (In effect, the genetically modified mouse serves as a test environment for determining the effect of the hormone on animal growth). Later, Palmiter, et al., Science, 222:809 (1983) demonstrated that a similar enhancement of growth could be obtained in transgenic mice bearing an expressible human growth hormone gene. A like effect is observed when human growth hormone releasing factor is expressed in transgenic mice. Hammer, et al., Nature, 315:413 (1985).

Bovine growth hormone has also been expressed in transgenic animals. McGrane, et al. J. Biol. Chem., 263:11443-51 (1988); Kopchick, et al., Brazil. J. Genetics, 12:37-54 (1989).

However, transgenic animals characterized by an exogenous gene which confers a reduced growth phenotype were hitherto unknown.

Researchers have attempted to predict the secondary structure of a protein from its primary amino acid sequence Chou and Fasman, Biochemistry, 13:222 (1974). The secondary structure of a polypeptide is a regular arrangement of a linear segment of the polypeptide chain. The most commonly encountered secondary structures are the beta-sheets and the alpha-helices. Helices may be described by the rise per element d, the number of elements per turn n, and the distance r of a marker point on each element (e.g., the C_{α} atom) from the helix axis. The mean values of these parameters for alpha helices in polypeptides are as follows:
n, 3.6 residues/turn,
d, 1.5 angstroms/residue, and
r, 2.3 angstroms.

See Schulz and Schimer, Principles of Protein Structure 69 (Springer-Verlag: 1979). The alpha helix is stabilized by hydrogen bonding between peptide amide and carbonyl groups of residues separated by a single turn of the helix.

Secondary structure predictions are based on observation of the frequency of occurrence of the amino acid in a beta-sheet, alpha-helix, etc. in a protein having a known three dimensional structure.

### SUMMARY OF THE INVENTION

The present invention relates to proteins which are substantially homologous with a vertebrate growth hormone but have growth-inhibitory activity.

We have discovered that mutation of Gly¹¹⁹ in bGH to Arg ("G119R"), Pro ("G119P"), Lys ("G119K"), Trp ("G119W") and Leu ("G119L") and the mutation of Ala¹²² to Asp ("A122D") results in a mutein (mutant protein or peptide fragment thereof) which has growth-inhibitory activity in vertebrates, especially mammals. This novel hormone may be administered to mammals (or other vertebrates), in particular bovines, when growth inhibition is desirable. In one embodiment of the invention, the hormone is produced exogenously and administered to the subject. In view of the size of the hormone, it is preferably produced by expression in a suitable host of a gene coding for it. Such a gene is most readily prepared by site-specific mutagenesis of a bGH gene. However, the hormone may also be produced by other techniques, such as by condensation of fragments of native bGH with a synthetic peptide carrying the replacement amino acid.

Accordingly the present invention provides an isolated peptide or protein of at least 11 amino acids having growth-inhibitory activity in a vertebrate and comprising a mutant alpha helix which is substantially homologous with but not identical to the third alpha helix of a vertebrate growth hormone, the said third alpha helix corresponding to the alpha helix formed by the amino acids at positions 109 to 126 of the naturally occuring form of bovine growth hormone (bGH), said naturally occuring form of bovine growth hormone being identical to the bovine growth hormone mutant sequence set forth in Figure 1 except for the presence of a glycine instead of the arginine shown at position 119 of the mutant sequence, wherein the mutant alpha helix differs from the third alpha helix of the vertebrate growth hormone at least at the position corresponding to position 119 of said naturally occurring form of bovine growth hormone, with the proviso that the protein or peptide is neither (a) the bovine somatotropin having the amino acid sequence of Chart 1 of WO 91/00870 (=D1), but having glycine at position 119 replaced by proline; nor (b) a mutant, hPRL(111-129), of full length native human growth hormone ( hGH) having the sequence shown in Fig. 1 of Cunningham et at, Science 243:1330-36, 1989 (=D7), which mutant differs therefrom solely by the 12 substitutions as indicated for hPRL(111-129) in Table 1 of D7.

Also provided are a recombinant DNA molecule comprising a gene including a sequence encoding such a protein or peptide, a cell comprising such a DNA molecule and a method of producing such a protein or peptide.

Further provided is use of a peptide or protein of at least 11 amino acids, comprising an alpha helix which is substantially homologous with but not identical to that alpha helix of a mammalian or other vertebrate growth hormone that corresponds to the alpha helix that comprises the amino acids at positions 109 to 126 of the naturally occurring form of bovine growth hormone (bGH) that is identical to the bovine growth hormone mutant sequence set forth in Figure 1 but for the presence of a glycine instead of the arginine shown at position 119 of the mutant sequence, the alpha helix of said peptide or protein differing from that vertebrate growth hormone at least at the amino acid corresponding to the glycine at position 119 of said naturally occurring form of bovine growth hormone, in the manufacture of a medicament for the treatment of a condition improvable by inhibiting or reducing growth hormone activity.

In a further embodiment of the invention, this gene is introduced into a prenatal form of a mammal by known techniques, and the prenatal form is developed into a transgenic mammal which expresses a reduced growth phenotype. A mammal could be genetically modified after birth, i.e., "gene therapy".

The present invention therefore provides a recombinant DNA molecule comprising a gene including a sequence encoding a peptide or protein of at least 11 amino acids, comprising an alpha helix which is substantially homologous with but not identical to that alpha helix of a mammalian or other vertebrate growth hormone that corresponds to the alpha helix comprising the amino acids at positions 109 to 126 of the naturally occurring form of bovine growth hormone (bGH) that is identical to the bovine growth hormone mutant sequence set forth in Figure 1 but for the presence of a glycine instead of the arginine shown at position 119 of the mutant sequence, wherein the alpha helix of the peptide or protein differs from the vertebrate alpha helix of the growth hormone at least at the amino acid corresponding to the glycine at position 119 of said naturally occurring form of bovine growth hormone for use as a medicament, especially for inhibiting or reducing growth hormone activity in a human or animal subject.

The invention also provides use of such a recombinant DNA molecule in the manufacture of a composition for the treatment of a condition improvable by inhibiting or reducing growth hormone activity.

Thus, growth-inhibited animals may be produced either by administration of the growth inhibitory hormone of this invention in pharmaceutical form, or by genetic transformation of a prenatal or postnatal form of the animal.

The hormone, or the gene encoding it, is useful in the production of small animals for use in research facilities where space is restricted, as pets for pet lovers with limited quarters, and as livestock for farmers having small tracts. The hormone may also be useful in the treatment of human giantism, and in research on giantism and dwarfism.

In the course of our work, we have discovered a correlation between the ability of mouse L cells to secrete the protein and the protein having an effect (positive or negative) on growth rate in a transgenic animal. An L cell secretion assay may be used to identify growth-modulating proteins.

The appended claims are hereby incorporated by reference as a further enumeration of the preferred embodiments. All patents and publications cited herein are incorporated by reference to the extent pertinent.

### BRIEF DESCRIPTION OF THE DRAWINGS

Figure 1 Amino acid sequence of bGH (G119R) and nucleotide sequence of the gene encoding this analogue. The alpha helices are marked and the amino acids are numbered, with number 1 being the first amino acid of the mature protein. The boldfaced bases and amino acids are those mutagenized in the G119R mutant.

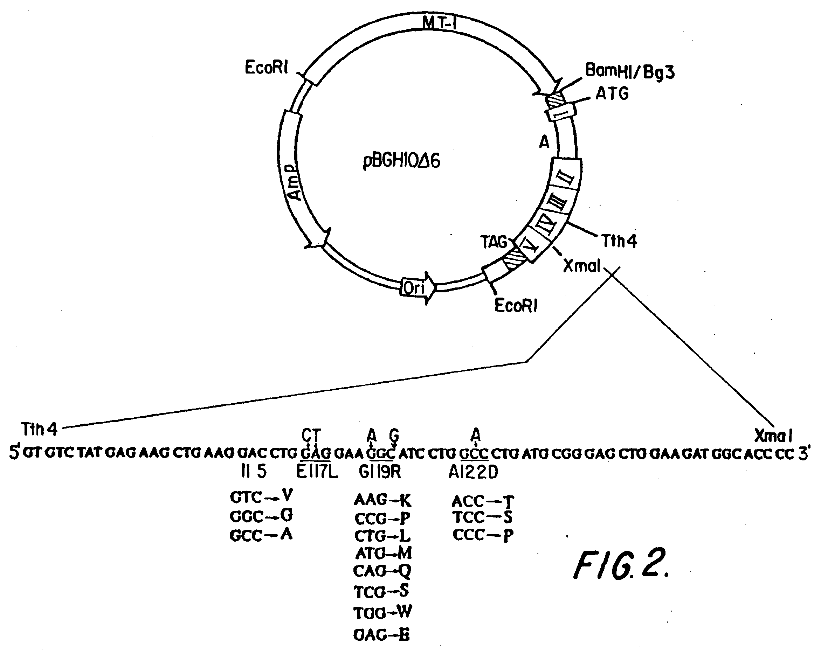

Figure 2 General strategy of oligonucleotide-directed mutagenesis. pBGH10Δ6 was used as the parental vector. It contains mouse metallothionein I transcriptional regulatory sequences (MT-1) fused to the bGH gene (BamHI joint with BglII) which contains five exons (shaded boxes I-V) and intron A. This fusion gene was incorporated into pBR322 at the EcoRI site. The pBR322 origin of replication (ORI), ampicillin resistant gene (Amp), as well as the bGH translation start (ATG) and stop (TAG) codons are indicated. 5' and 3' non-translated regions are shown in hatching. The nucleotide sequence between restriction sites Tth111I and XmaI is shown. Substitution mutations are indicated. One silent situation is also indicated (*) which created a unique BamHI site. The position of the principal amino acid residues targeted for change (115, 117, 119, 122) are indicated.
Figure 3 is an idealized surface net (cylindrical plot) representation of most of the third alpha helix of bovine growth hormone. The surface net is produced by projection of the helix onto a coaxial cylindrical sheet of paper, cutting this paper parallel to the helical axis and flattening it. The volumes of the amino acids are given in parentheses. A dashed line indicates the cleft or depression formed by Ala122-Gly11a-Asp115.

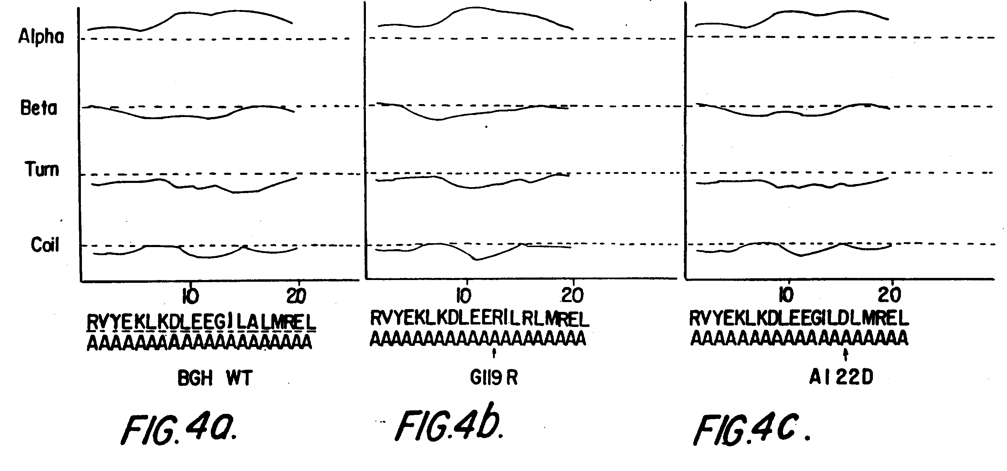

Figure 4 is a plot of the secondary structure prediction (alpha-helix, beta-sheet, reverse turn, random coil) for amino acids 108-127 of bovine growth hormone (a) wild-type (b) the mutant G119R and (c) the mutant A122D. These plots were generated by the "Micro-Genie" program.

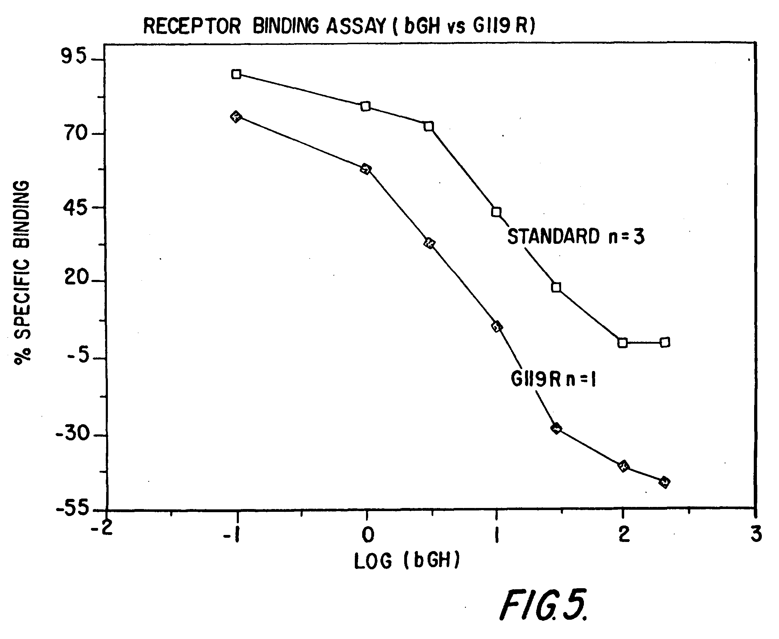

Figure 5 depicts a receptor binding assay, (wild-type) bGH versus mutant bGH G119R). This figure shows that the mutant bGH (G119R) has a greater affinity for the growth hormone receptor of mouse liver membrane preparations than wild type bGH.

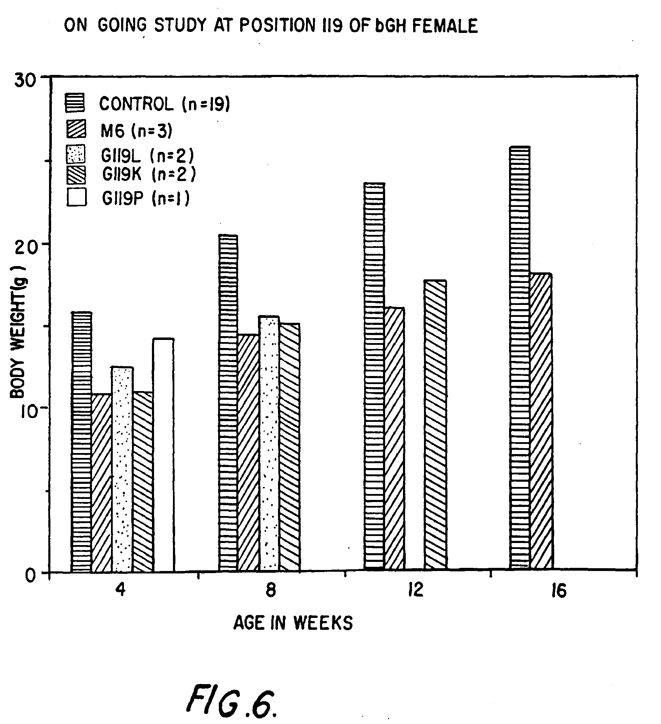

Figure 6 provides a growth rate comparison among control (non-transgenic), G119R, G119L, G119K and G119P mice, illustrating the growth-inhibitory effect of these mutants.

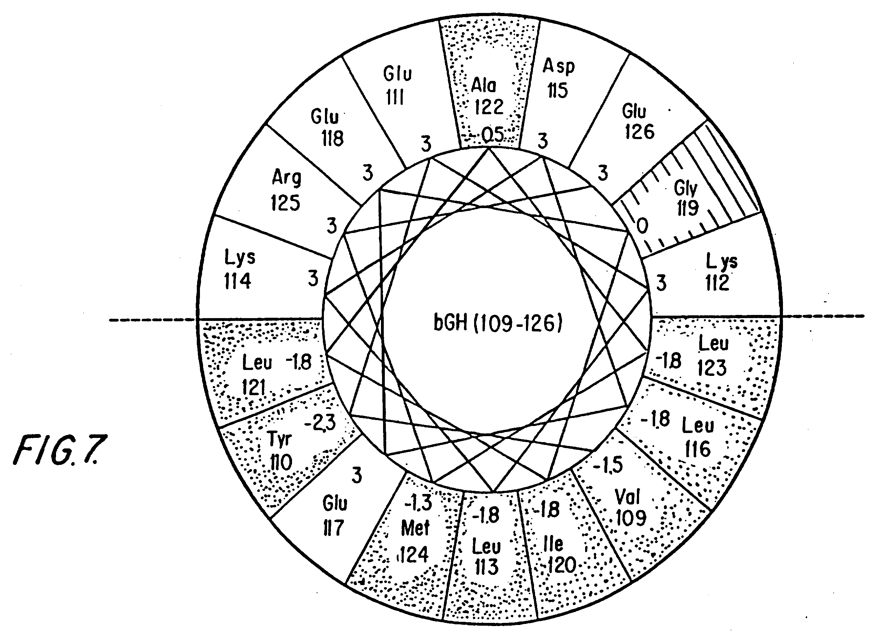

Figure 7 presents an axial view of the third alpha helix (109-126) of bGH, showing its amphipathic tendencies. Hydrophobic amino acid sectors are shaded by dots; the glycine sector, a neutral amino acid, by slanted lines. The residue numbers and hydrophilicity values (Hopp and Wood scale) are given.

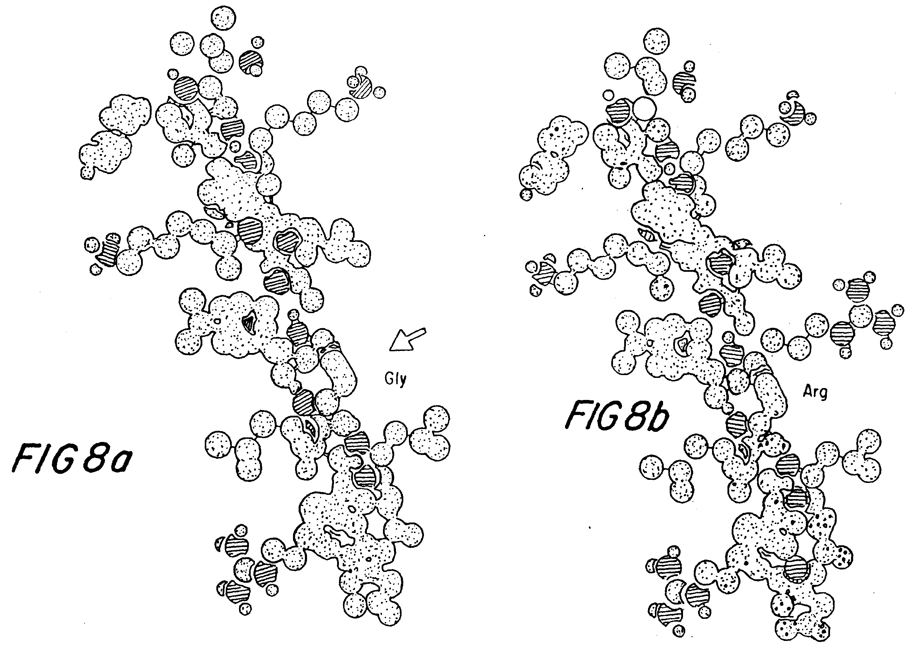

Figure 8 presents side views of the third alpha helix of wild type (left) and G119R mutant (right) bGHs projected on the plane in which the side chain of the Arginine-119 of the mutant G119R lies. The bottom of the cleft is indicated by an arrow.

The views were prepared by use of molecular modelling software (QUANTA and CHARMm, Polygene, Waltham, Massachusetts, USA).

### DETAILED DESCRIPTION OF THE PREFERRED EMBODIMENTS

The three-dimensional structure of porcine growth hormone has been determined by X-ray diffraction and compared to that of other growth hormones. Abdel-Meguid, et al., Proc. Nat. Acad. Sci., 84:6434 (1987). Like the other growth hormones thus studied, it is a single domain protein arranged as a four helix bundle with the helices in an antiparallel relationship. Its four helixes are made up of residues 7-34, 75-87, 106-127 and 152-183.

Bovine growth hormone is 93% homologous at the amino acid sequence level with porcine growth hormone, and bGH's structure has been deduced by study of two sequences and of the structure of porcine growth hormone. Its four alpha helixes have been reported to be assumed by amino acids 4-33, 66-80, 108-127 and 150-179, though for purposes of the present invention, the third alpha helix of bGH is defined as amino acids 109-126. The conformation is reasonably consistent with the predictions made by Chen and Sonenberg, Biochemistry, 16:2110 (1977) using the method of Chou and Fasman, supra (10-34, 66-87, 111-127, 186-191).

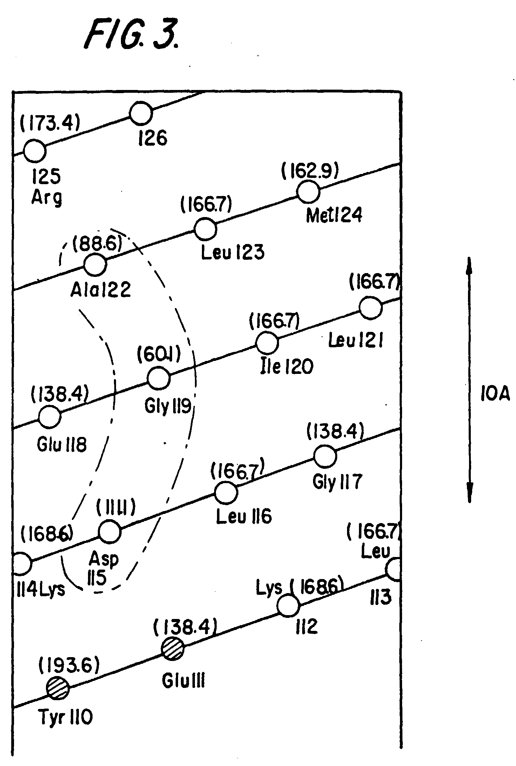

The amino acid sequence of the growth hormones isolated from various vertebrate species are highly conserved. In a comparison of flounder growth hormone with other growth hormones, including bGH, Watahiki, et al., J. Biol. Chem., 264:312 (1989) identified five conserved regions. Watahiki's conserved region GD4 comprises the stretch LKDLEEGILALMRELED of bovine growth hormone, i.e., residues 113 to 129. Watahiki's Figure 3 identifies residues conserved among the GHs and residues predicted to be important for the manifestation of growth-promoting activity.

It has been shown that a recombinant molecule containing a hGH-(1-134) fragment linked to a human placental lactogen-(14-191) fragment retained full hGH immunological activity and binding affinity to GH receptors isolated from rabbit liver. Russell, et al., J. Biol. Chem., 256:296-300 (1981). By using the homolog-scanning mutagenesis technique, gene fragments of homologous hormones -i.e., human placental lactogen or human prolactin - were systematically substituted throughout the hGH gene, thus producing various chimeric hormones. Cunningham, et al., Science, 243:1330-36 (1989). A comparison of the binding affinities of these mutant GHs and wild-type hGH to a cloned liver hGH receptor led to the conclusion that there were three discontinuous polypeptide determinants in hGH involved in receptor binding. They were located at the NH₂ terminus, COOH terminus, and within a loop between amino acid residues 54 and 74. These putative binding domains were further analyzed by an alanine-scanning mutagenesis technique in which alanine residues were systematically substituted throughout those regions. Amino acid residues at positions 10, 58, 64, 68, 172, 174, 175 and 176 of hGH were shown to be important for GH receptor binding. Cunningham, et al., Science, 244:1081-85 (1989).

The present invention relates to growth-inhibitory compounds (peptides or proteins) having a similarity in sequence and secondary structure to a vertebrate growth hormone, including but not limited to mammalian growth hormones, especially bovine growth hormone. Preferably, the compound comprises an alpha helix having an amino acid sequence homology of at least about 50% with the third alpha helix of a vertebrate growth hormone, especially bovine growth hormone. Other alpha helices of the native hormone may be omitted if this can be done without loss of growth-inhibitory activity.

The overall percentage homology of bovine growth hormone with other mammalian growth hormones is high: porcine (92%), ovine (99%), human (66%), and rat (87%). Insofar as the third alpha helix (amino acid sequence homologous to bGH 109-126) is concerned, the percentage homology is comparable to the overall figure: porcine (94%), ovine (94%), human (66%) and rat (94%).

The present invention is not limited to the mutation of the third alpha helix of bovine growth hormone. Rather, it encompasses the mutation of the third alpha helix of any mammalian or other vertebrate growth hormone, including, but not limited to, the growth hormones whose sequences are given in Watahiki (1989): flounder, yellowtail, tuna, salmon, chicken, rat, porcine, ovine, bovine and human growth hormones. Expression of mutants of other growth hormones is facilitated by the availability of genes encoding the latter. See, e.g., Goeddel, Nature, 281:544-683 (1979) (hGH).

The concept of a polypeptide which is substantially homologous to bovine growth hormone is deemed to include (but is not limited to) any polypeptide which differs from bovine growth hormone by (a) a substitution at an amino acid corresponding to amino acid 119 and, optionally, also at 115 and/or 122 of bovine growth hormone, and, optionally (b) a substitution at an amino acid corresponding to an amino acid of bovine growth hormone which is not conserved among the vertebrate growth hormones, and/or truncation of amino acids 1-95 and/or 134-191. (Conserved amino acids are identified in Watahiki, et al, 1989.) Thus, all non-bovine vertebrate growth hormones are "substantially homologous" with bovine growth hormone.

The compound is considered to be growth-inhibitory if the growth of test animals of at least one vertebrate species which are treated with the compound (or which have been genetically engineered to express it themselves) is significantly slower than the growth of control animals (the term "significant" being used in its statistical sense). Preferably, it is growth-inhibitory in a plurality of species. Growth hormones have considerable interspecies cross-reactivity. Gill, et al., Biotechnology, 3:643 (1985) reported that recombinant chicken and bovine growth hormones accelerate growth in juvenile pacific salmon.

It is known that certain fragments of growth hormones also have growth-promoting activity, and it is expected that the growth-inhibitory peptides (the term is used hereafter to include proteins) of the present invention need not be as large as bGH. Preferably, the peptides are at least 11 amino acids long (three turns of an alpha helix) and more preferably at least 50 amino acids long. These peptides may retain the growth inhibiting action of, e.g., bGH (G119R), yet lack other, undesirable biological activities of the native size mutant. They may also have more desirable pharmacokinetic characteristics.

Accordingly, one aspect of the present invention is a peptide or protein of at least 11 amino acids, comprising an alpha helix which is substantially homologous with but not identical to that alpha helix of a mammalian or other vertebrate growth hormone that corresponds to the alpha helix that comprises the amino acids at positions 109 to 126 of the naturally occurring form of bovine growth hormone (bGH) that is identical to the bovine growth hormone mutant sequence set forth in Figure 1 but for the presence of a glycine instead of the arginine shown at position 119 of the mutant sequence, the alpha helix of said peptide or protein differing from that alpha helix of the vertebrate growth hormone at least at the amino acid corresponding to the glycine at position 119 of said naturally occurring form of bovine growth hormone (bGH), with the above-mentioned proviso, said peptide or protein corresponding to a fragment of said vertebrate growth hormone and said peptide or protein having growth-inhibitory activity in a vertebrate.

The growth inhibitory peptides of the present invention may also be larger than bGH, provided, that the additional amino acids do not result in the compound being unable to reduce the growth rate of a vertebrate.

While the mechanism of action of applicant's growth-inhibitory peptides is not known, it is believed that they function as antagonists to wild-type growth hormones endogenously produced by the target animal. We have shown that, e.g., bGH (G119R) and bGH (G119R, E117L, A122D), both competitively inhibit the binding of wild type bGH to liver membrane preparations. Thus, it is believed that the compound has a net result of inhibiting growth because its growth-promoting activity is substantially less than that of wild type growth hormones (and perhaps is negligible) yet it can displace from growth hormone receptor sites the endogenous native growth hormone (whose stimulation of growth would have been more pronounced). However, applicants are not bound by this theory. Preferably, the compounds of the present invention have ED50 which is less than about 10 times the ED50 of wild type bGH in an assay of the ability of the compound to displace radiolabeled wild type bGH from a liver membrane preparation made as described below. More preferably, the compounds have an ED50 at least comparable to that of wild type bGH. Most preferably, the compounds have a higher affinity for growth hormone receptors than does the growth hormone native to the animal receiving the compound. For purification and characterization of a human growth hormone receptor, see Leung, et al., Nature, 330:537-43 (1987).

The preferred growth-inhibitory peptides are characterized by a modification of the surface topography of the third alpha helix. It will be seen from Figure 3 that in third alpha helix of "wild-type" bovine growth hormone, there is a surface cleft or depression beginning, at the Alanine-122, deepening at the Glycine-119, and ending with the Aspartate-115. All of the mutants prepared so far, both those which retain the wild-type growth-promoting activity and those which do not, are consistent with the theory that growth-promoting activity requires the presence of this cleft or depression and that, if it is "filled in" by substitution of amino acids with bulkier side chains, the mutein inhibits the growth of the subject.

Mutations which substantially destabilize the alpha-helix are undesirable since they may result in the loss of all growth-related activity. We have observed such loss in the case of several mutations which were expected to disrupt the alpha helix.

For a discussion of alpha helix formers and breakers, see Chou and Fasman, supra. Glu, Ala and Leu are the preferred alpha formers while Pro and Gly are characterized as strong helix breakers. Substitutions which introduce strong alpha helix breakers are less desirable, but may be tolerated in a particular case, such as the end of the helix. The secondary structures of our analogues have been predicted using the "Micro Genie" computer program, which uses the algorithm of Garnier, et al., J. Biol. Chem., 120:97-120 (1978).

With respect to amino acid 119, glycine is both the smallest amino acid residue and the one least favorable to alpha-helix formation. Thus, it is believed that any other amino acid may be substituted for it without destabilizing the alpha helix, while at the same time filling in the aforementioned cleft. It is possible that G119A will confer the "small animal" phenotype, particularly in conjunction with other mutations, and such mutation is in that case within the scope of the present invention. However, alanine is less favored than other amino acids, because it is the next smallest amino acid and since alanine is the native amino acid at position 122. More preferably, the new amino acid is larger than alanine.

With regard to position 122, the alanine may be changed to any amino acid other than glycine (which has the disadvantages already mentioned), provided that the mutation does not destroy the alpha helix. Proline is not favored because mutations K114P, E118P and L121P are believed to destroy the alpha-helix. Tyrosine has a higher alpha-helical propensity than proline, but still lower than that of the wild-type alanine. However, since the alanine at position 122 is flanked on both sides by leucine, a strong alpha-helix former, it is possible that the substitution of tyrosine or even proline would be tolerated.

The modification of position 115 is suggested by our "cleft" theory. The aspartate at position 115 may be replaced by a bulkier amino acid which does not destroy the alpha helix. Preferably, the replacement amino acid has a size greater than that of glutamate. The amino acids histidine, methionine, isoleucine, leucine, lysine, arginine, phenylalanine, tyrosine and tryptophan are substantially larger than glutamate. Of these, His, Met, Leu and Trp are more preferred because they combine the advantages of bulk with a reasonably strong alpha-helical propensity. Note, however, that the wild-type Glu is the strongest alpha-helix former of all of the amino acids.

The peptides and proteins of the present invention comprise an altered amino acid at position 119. The amino acids at positions 122 and 115 may also be altered if desired, either singly or in combination. It is also possible to alter another amino acid in the alpha helix provided that the substitution does not destroy the alpha helix. Preferably, such alterations replace an amino acid with one of similar size and polarity. It may be advantageous to modify amino acids flanking the primary mutation site 119 and 122 and/or 115 in order to increase the alpha-helical propensities of the sequence, particularly if the mutation at 119, and any at 122 and/or 115, is one expected to destabilize the helix.

The present invention provides especially for a protein selected from the group consisting of
bGH (G119R),
bGH (G119R, E117L, A122D),
bGH (G119K),
bGH (G119L), and
fragments thereof, and
fragments of bGH (G119P)
having growth-inhibitory activity in a vertebrate,
   wherein bGH is the naturally occurring form of bovine growth hormone (bGH) that is identical to the bovine growth hormone mutant sequence set forth in Figure 1 but for the presence of a glycine instead of the arginine shown at position 119 of the mutant sequence.

The present invention is not limited to any particular method of producing the desired bGH antagonists. Preferably, these antagonists are produced by first altering a gene encoding a BGH having the "native" third alpha helix by site-specific mutagenesis, and then cloning and expressing the altered gene in a suitable host. The gene may be of genomic origin, it may be cDNA prepared from bGH messenger RNA, it may be synthetic, or it may be a combination thereof. For the amino acid sequence of bGH and for the cDNA sequence of the bGH gene, see Miller, et al., J. Biol. Chem., 255:7521-24 (1980). For the genomic sequence, see Woychick, et al., Nucleic Acids Res., 10:7197-7210 (1982).

Accordingly, the present invention provides a method of producing a growth inhibitory peptide or protein which comprises providing cells bearing a gene including a sequence encoding a peptide or protein according to the invention with conditions conducive to the expression of said gene, whereby said protein is produced in a usable or recoverable form.

The host may be any convenient organism, including a bacterial, yeast, or mammalian cell. The gene is operably linked to a promoter functional in the host. A constitutive promoter would activate gene expression in a general manner, i.e., in many tissue and at all times during development. A regulatable promoter may be activated in a tissue or cell specific manner, at precise time during development, or in response to changes in the environment. A constitutive promoter is usually employed when larger amounts of gene product (usually protein) is required or when the gene product is required in many cells of many tissues. A regulatable promoter is utilized when one gene product is required in a small number of cells of a particular tissue or at a given time during development.

The expression system may be engineered so that the antagonist is secreted into the culture medium, or the host cells may be grown to a high cell density and then lysed to release the compound.

One method suitable for the purification of bGH (G119R) and the like is described in Leung, et al., Endocrinology, 119:1489-1496 (1986). Essentially, this procedure involves purification by (a) ammonium sulfate precipitation, (b) fractionation on DEAE-cellulose (or any equivalent ion-exchange column), and (c) gel filtration (e.g., on a Sephadex G-25 and/or Sephacryl S-200 column). Other procedures applicable to purification of growth hormone-related compounds are set forth in Reichert, Jr., "Purification of Anterior Pituitary Hormones: Bovine, Rat and Rabbit," Meth. Enzymol., 37:360 et seq. (Academic Press, N.Y.:1975). Polyclonal or monoclonal antibodies which specifically recognize the protein of interest may also be used in the purification process.

The present invention provides a protein or peptide according to the present invention for use as a medicament and, also, a pharmaceutical composition comprising a protein or peptide according to the present invention and a pharmaceutically acceptable carrier.

The purified antagonist may then be combined with compatible, nontoxic pharmaceutical excipients and administered to an animal or human, e.g. to treat a condition characterized by an excessive growth rate. In the case of administration to animals, it may be preferable to incorporate the drug into the animal's feed, possibly in a prepared combination of drug and nutritional material ready for use by the farmer. An effective dosage and treatment protocol may be determined by conventional means, starting with a low dose in laboratory animals and then increasing the dosage while monitoring the effects, and systematically varying the dosage regimen as well. The trial dosages would be chosen after consideration of the clinical literature with respect to administration of growth hormones, and of somatostatin (a growth hormone release inhibitor).

In another embodiment, the gene is introduced into a host cell which is developed into genetically transformed cells of a transgenic animal. Linearized DNA bearing the growth hormone antagonist gene may be microinjected into a gamete, into the pronuclei of fertilized eggs, into the cytoplasm, into the nuclei of two-cell embryos, into individual cells of a blastocyst, or into the blastocoel cavity. (Some of these targets may be reached by electroporation instead of microinjection.) Alternatively, a retrovirus bearing the gene may be constructed and used to infect preimplantation embryos or tissue culture cells (e.g., embryonic stem cells) which may be aggregated with such embryos. In either case, the genetically modified zygote, after a brief in vitro cultivation, is implanted into a foster mother and carried to term. For "gene therapy" post partum, see Cline, et al., Nature, 284:422-425 (1980); Williamson, Nature, 298:416-18 (1982). Again, the gene is operably linked to a promoter functional in the host, and the promoter may be constitutive or regulatable. Preferably, expression is regulated so abnormal embryonic or fetal development is avoided.

Accordingly, the present invention provides a non-human transgenic animal comprising a plurality of cells comprising a gene operably linked to a promoter, the gene including a sequence encoding a peptide or protein of at least 11 amino acids comprising an alpha helix which is substantially homologous with but not identical to that alpha helix of a mammalian or other vertebrate growth hormone that corresponds to the alpha helix comprising the amino acids at positions 109 to 126 of the naturally occurring form of bovine growth hormone (bGH) that is identical to the bovine growth hormone mutant sequence set forth in Figure 1 but for the presence of a glycine instead of the arginine shown at position 119 of the mutant sequence, the alpha helix of said peptide or protein differing from that alpha helix of the vertebrate growth hormone at least at the amino acid corresponding to the glycine at position 119 of said naturally occurring form of bovine growth hormone.

The invention is further illustrated, without limitation, by the following examples.

### Example 1: Generation of Mutations Conferring the Reduced Growth Phenotype

### MATERIALS AND METHODS

The plasmid, pBGH-10delta6, was derived from pBGH-10 and contains the complete coding region of bGH and intron A. Bovine growth hormone introns B, C and D are absent (Figure 1). This plasmid encodes "wild type" bGH, and its expression is controlled by a 1700 base pair segment of the mouse metallothionein I transcriptional regulatory sequence.

Plasmids pBGH-10delta6-G¹¹⁹R and pBGH-10delta6-E¹¹⁷L, G¹¹⁹R, A¹²²D were derived from pBGH-10delta6 and were generated by segment-directed mutagenesis using complementary oligonucleotides to replace the DNA between the Tth111I site (found near the 3' end of Exon IV) and the Xma I site (located near the 5' end of Exon V). The other mutations described herein were generated similarly.

The complementary oligonucleotides used for pBGH10 delta 6-G¹¹⁹R were: 
5'GTGTCTATGAGAAGCTGAAGGACCTGGAGGAAAGGATCCTGGCCTGATGCGGGAGCTGGA
AGATGGCACCCC 3'; 73-MER) and (5'CCGGGGGGTGCCATCTTCCAGCTCCCGCAT
CAGGGCCAGGATCCTTTCCTCCAGGTCCTTCAGCTTCTCATAGACA 3'; 76-MER).
 The complementary oligonucleotides used for pBGH10delta6-E¹¹⁷L, G¹¹⁹R, A¹²²D were: 
(5'GTGTCTATGAGAAGCTGAAGGACCTGCTGGAAAGGATCCTGGACCTGATGCGGGAGCTG
GAAGATGGCACCCC 3'; 73-mer) and 5' CCGGGGGGTGCCATCTTCCAGCTCCCGC
ATCAGGTCCAGGATCCTTTCCAGCAGGTCCTTCAGCTTCTCATAGACA 3'; 76-mer).
 These oligonucleotides hybridize as follows:

### G119R

 GT GTC TAT GAG AAG CTG AAG GAC CTG GAG GAA AGG ATC CTG GCC
ACA CAG ATA CTC TTC GAC TTC CTG GAC CTC CTT TCC TAG GAC CGG
Arg Val Tyr Glu Lys Leu Lys Asp Leu Glu Glu Arg Ile Leu Ala
CTG ATG CGG GAG CTG GAA GAT GGC ACC CC
GAC TAC GCC CTC GAC CTT CTA CCG TGG GGG GCC
Leu Met Arg Glu Leu

### E117L, G119R, A122D

GT GTC TAT GAG AAG CTG AAG GAC CTG CTG GAA AGG ATC CTG GAC
ACA CAG ATA CTC TTC GAC TTC CTG GAC GTC CTT TCC TAG GAC CGG
Arg Val Tyr Glu Lys Leu Lys Asp Leu Leu Glu Arg lle Leu Asp

CTG ATG CGG GAG CTG GAA GAT GGC ACC CC
GAC TAC GCC CTC GAC CTT CTA CCG TGG GGG GCC
Leu Met Arg Glu Leu

These oligonucleotides encode DNA changes which result in the substitutions of arginine for glycine at position 119 in pBGH-10delta6-G119R; and leucine for glutamate at position 117, arginine for glycine at position 119 and aspartate for alanine at position 122 in pBGH-10delta6-E¹¹⁷L, G¹¹⁹R, and A¹²²D. These amino acids were chosen because they have hydrophilic (arginine and aspartic acid) or hydrophobic (leucine) character [See Hopp and Woods, PNAS (USA), 78:3824-28 (1981)], positively (arginine) or negatively (aspartic acid) charged side chains (See Kaiser and Kezdy, Science acid) 223:249-55 (1984)], and high α-helical-forming potential [See Chou and Fasman, Ann. Rev. Biochem., 47:251-76 (1978)] furthering generation of an idealized amphiphilic α-helix [See Margalit, et al., J. Immunol., 138:2213-29 (1987); Brems, et al., Biochemistry 26:7774-78 (1987); Kaiser and Kezdy, supra; Chen, et al., PNAS (USA), 87:5061-65 (July 1990). In addition, these oligonucleotide duplexes encode a silent base-pair change designed to create a unique BamHI restriction site which simplified screening procedures. The oligonucleotides were annealed and subcloned between the Tth111I and XmaI sites using standard procedures (Maniatis et al., Molecular Cloning (Cold Spring Harbor: (1982)). Mutant plasmid DNA's were identified by digestion: with BamH1 restriction site which simplified screening procedures. The oligonucleotides were annealed and subcloned between the Tth111I and XmaI sites using standard procedures (Maniatis et al., Molecular Cloning (Cold Spring Harbor: 1982). Mutant plasmid DNA's were identified by digestion with BamH1.

The nucleotide sequence of the mutated bovine growth hormone target regions were determined by using the dideoxy chain-termination method with modified T7 DNA polymerase (Sequenase, United States Biochemical; Sanger et al., PNAS (USA), 74:5463-67 (1977)). Oligonucleotide primers for manual DNA sequencing were synthesized using the DuPont Coder #300 DNA synthesizer and purified by denaturing polyacrylamide gel electrophoresis, passive elution and concentration by ethanol precipitation. The oligonucleotide primers used for the direct sequencing analysis of the two mutants was the following: 18-mer (5'AAATTTGTCATAGGTCTG 3'). Briefly, 1-3µg of double-stranded plasmid DNA was denatured in the presence of 0.2N NaOH, and 10-20 pmoles of oligonucleotide primer was allowed to anneal (65°C, 2 min. followed by 30 min. slow cool) to the denatured template. A two-step polymerization was performed by using the modified T7 DNA polymerase which extends the oligonucleotide-primed chain in the presence of dNTP's and deoxyadenosine 5'-[α-³⁵S] triotriphosphate (>1000 Ci/mmole, Amersham) followed by transfer of equal aliquots into each of four specific dideoxynucleotide mixes which randomly terminate chain elongation. Following addition of a formamide termination buffer to each reaction, the samples were incubated at 80°C for 2 min. and the DNA sequence was determined after size fractionation of the four sets of fragments by 10% polyacrylamide/8M urea electrophoresis and autoradiography.

### Example 2: Expression in Mammalian Cells in Culture

Using the in vitro mutagenesis protocols described above, two mutant bGH genes were generated initially: one converts glycine¹¹⁹ to arginine ("G119R") and the second converts glutamate¹¹⁷ to leucine, glycine¹¹⁹ to arginine, and alanine¹²² to aspartate (E117L, G119R, A122D).

The plasmids encoding these mutations as well as wild type bGH DNA (pBGH10delta) were transiently introduced into cultured mouse L cells, which were subsequently analyzed for bGH expression. Following "western analysis", protein bands of approximately 22,000 daltons were observed for wild type bGH and bGH derived from the two mutant genes.

Mouse L cells were maintained in DMEM (Gibco) plus 10% calf serum and 25µg/ml gentamicin (Gibco). In this study, a modification of a previously described transfection procedure was employed (Lopata et al., Nucleic Acids Res., 12:5707-5717 (1984)). Briefly, 2µg of plasmid DNA was added to 1.0ml of DMEM containing 0.2mg DEAE-dextran. This solution was added to approximately 10⁶ cells in a 35-mm tissue culture plate which had been washed previously with 2.0ml of DMEM. Following incubation of the cells for 1 hour at 37°C, the DNA-DEAE-dextran solution was removed and the cells "shocked" for 90 seconds with 2.0ml of 10% DMSO in Hepes buffered saline, at room temperature. Subsequently, the "shock" solution was removed and cells washed with 2.0ml DMEM. Media containing 10% Nu-Serum (Collaborative Research) plus 50µg/ml gentamicin were changed daily. Culture fluids were stored at -20°C. For bGH binding assays, transfected cells were incubated in DMEM minus serum for 16 hours, after which the culture fluids were removed and frozen at -20°C.

Sodium dodecyl sulfate (SDS) PAGE analyses of secreted bGH have been described (Kopchick et al., DNA, 4:23-31 (1985); Kelder et al., Gene, 76:75-80 (1989). In this study, we used a polyclonal anti-bGH serum for "western" analysis.

### Example 3: Growth Hormone Receptor Binding Studies

Culture fluids lacking serum were collected from cells transfected by pBGH-10delta6 (wild type bGH) and the mutant bGH genes. Following lyophilization of the culture media and bGH concentration determinations, competitive membrane binding studies were carried out as previously described (Smith & Talamants, J. Biol. Chem., 262:2213-19 (1987)). Liver membrane preparations from C57BL/6JxSJL hybrid mice of either sex (60-120 days old) were homogenized with a Brinkman Polytron in 4 volumes (w/v) of 0.3M sucrose, 10mM EDTA, 50mM Hepes, 0.1mM TPCK and 1mM PMSF at pH 8.0. The above step and all the following protocols were carried out at 4°C. The homogenate was centrifuged at 20,000xg for 30 min. and the supernatant was centrifuged at 100,000xg for 1 hour. The pellets were washed once with 10mM Hepes, pH 8.0 and recentrifuged. These pellets were resuspended in 10mM Hepes, pH 8.0, to a protein concentration of approximately 50mg/ml. The membranes were aliquoted, frozen on dry ice, and stored at -20°C. Membrane protein concentrations were determined by the Lowry protein assay (Lowry et al., J. Biol. Chem., 193:265-275 (1951)).

Competitive binding assays were performed using the following protocol. Microsomal membranes corresponding to three mgs. protein were incubated with 30,000 cpm/tube ¹²⁵I bGH (Cambridge Medical Diagnostics) and unlabeled bGH ranging from 0.3ml assay buffer (20mM Hepes, 10mM CaCl₂ 0.1% BSA, and 0.05% NaN₃ pH 8.0). All assays were performed in triplicate. After overnight incubation at room temperature, membrane bound hormone was separated from free hormone by the addition of 1 ml of ice cold assay buffer followed by centrifugation at 10,000xg for 20 min. Membrane pellets were then assayed for radioactivity. Specifically bound radioactivity was determined by subtraction from the value produced by incubation of membranes with 5µg unlabeled bGH (Smith and Talamants, 1987).

Effective doses which resulted in 50% displacement (ED50) of ¹²⁵I-bGH from the membrane preparations were determined. Mutant bGH encoded by pBGH-10delta6-G¹¹⁹R and pBGH10delta 6-E¹¹⁷L, G¹¹⁹R, A¹²²D revealed an ED50 value similar to (the triple mutant) or higher than (G119R) wild type bGH.

### Example 4: Transgenic Mouse Production

A series of transgenic mouse lines which contain wild type and mutant bGH genes were produced by standard microinjection techniques (McGrane et al., 1988). DNA extraction from mouse tails, dot blots, and serum determinations was as described (McGrane et al., 1988).

The genes contain the transcriptional regulatory sequences of the mouse metallothionein I promoter which has been shown to be active in liver tissue as well as other tissues of the transgenic mouse (Palmiter et al., Nature, 300:611-615 (1982)). Offspring generated by the microinjection procedure were assayed for bGH DNA by slot blot hybridization analysis. Mouse lines were generated which contain approximately one copy of the recombinant bGH DNA sequences derived from pBGH-10delta6, (wild type), pBGH-10delta6-G¹¹⁹R, and pBGH10delta6-E^{117L}, G¹¹⁹R, A¹²²D. Serum from transgenic animals were assayed for bGH levels by the Western technique. All mice which expressed the wild type bGH transgene in serum also possessed a corresponding enhanced growth rate. Mice which expressed mutant bGH (G¹¹⁹R or E¹¹⁷L, G¹¹⁹R, A¹²²D) in serum were dramatically and significantly smaller. The growth rate for wild type bGH transgenic mice relative to control littermates was 1.5 while the ratio for the two bGH mutant mice to control littermates was ^{∼}0.6. In the case of the triple mutant, we have generated 10 founder mice that express the mutated bGH gene. The growth ratio between the transgenic and nontransgenic littermates ranged from 0.58 to 1.00. The degree of suppression of growth was directly related to the serum levels of the mutated bGH. Three founders have been bred that pass the trait to offspring; ≈50% of these offspring are positive for the gene and possess the corresponding small phenotype.

Since there was no change in binding affinity of bGH-M8 (the triple mutant) to mouse liver membranes when compared with wild-type bGH, it is possible that bGH contains distinct growth-promoting and receptor-binding domain(s). The three substitution mutations in bGH (bGH-M8) located within the third α-helix may have altered either one or more critical amino acids in, or the local conformation of, this potential growth-promoting domain, subsequently resulting in a reduction in bGH growth promoting activity. Amino acid sequence comparison among GHs and products of other members of the gene family revealed that Glu-117, Gly-119, and Ala-122 are relatively conserved in products of the GH gene family, (See Watahiki, et al., (1989) supra). Ala-122 is conserved only in GHs from nonprimate mammals as well as chickens, whereas Glu-117 is only conserved in GHs from mammals. Gly-119 is conserved among products of all members of the GH gene family, which includes prolactins and placental lactogens. Therefore, these amino acids may be important for the biological activities of GHs.

It has been demonstrated that many activities of GH are mediated through a family of peptides known as insulin-like growth factors (IGF), in particular IGF-1, which is believed to be produced primarily in the liver following GH binding to its receptor(s). (See Truesch, et al., Ann. Rev. Physiol., 47:443-67 (1985); Zapt, et al.; Harm. Res., 24:121-130 (1986)). IGF-1 has been shown to decrease GH production in the pituitary by a classical negative feedback mechanism. (Leung, et al., Endocrinology, 119:1489-96 (1986)). One hypothesis to explain the growth suppression in pBGH10Δ6-M8 transgenic mice is that bGH-M8 is active as an in vivo antagonist to mouse GH (mGH), thereby suppressing mouse IGF-1 production. If this is true, then one would expect not only a reduction in serum mouse IGF-1 levels in bGH M8 transgenic mice but also an increase in mGH production in the pituitary. Preliminary results from immunoblot analysis of whole pituitary glands taken from bGH-M8 transgenic mice, bGH transgenic mice, and their nontransgenic littermates suggest that the pituitary glands in those growth-suppressed mice contain higher levels of mGH relative to their nontransgenic littermates. In contrast, mGH levels in bGH transgenic mice were largely depressed because mouse IGF-1 levels in serum of bGH transgenic mice increased up to twice as much as levels in serum of their nontransgenic littermates. Palmiter, et al., Science, 222:809-14 (1983). If our hypothesis were true, it would be the first example to our knowledge of an in vivo growth hormone antagonist and the first example of uncoupling of growth-promoting and receptor-binding activities of GHs.

### Example 5: Screening of other Muteins of bGH

### Examples and Comparative Examples

By similar procedures, muteins of bGH with alterations in the third alpha helix have been prepared and tested for secretion in L cells, and, in selected cases, their effect on the growth of transgenic mice, with the following results.

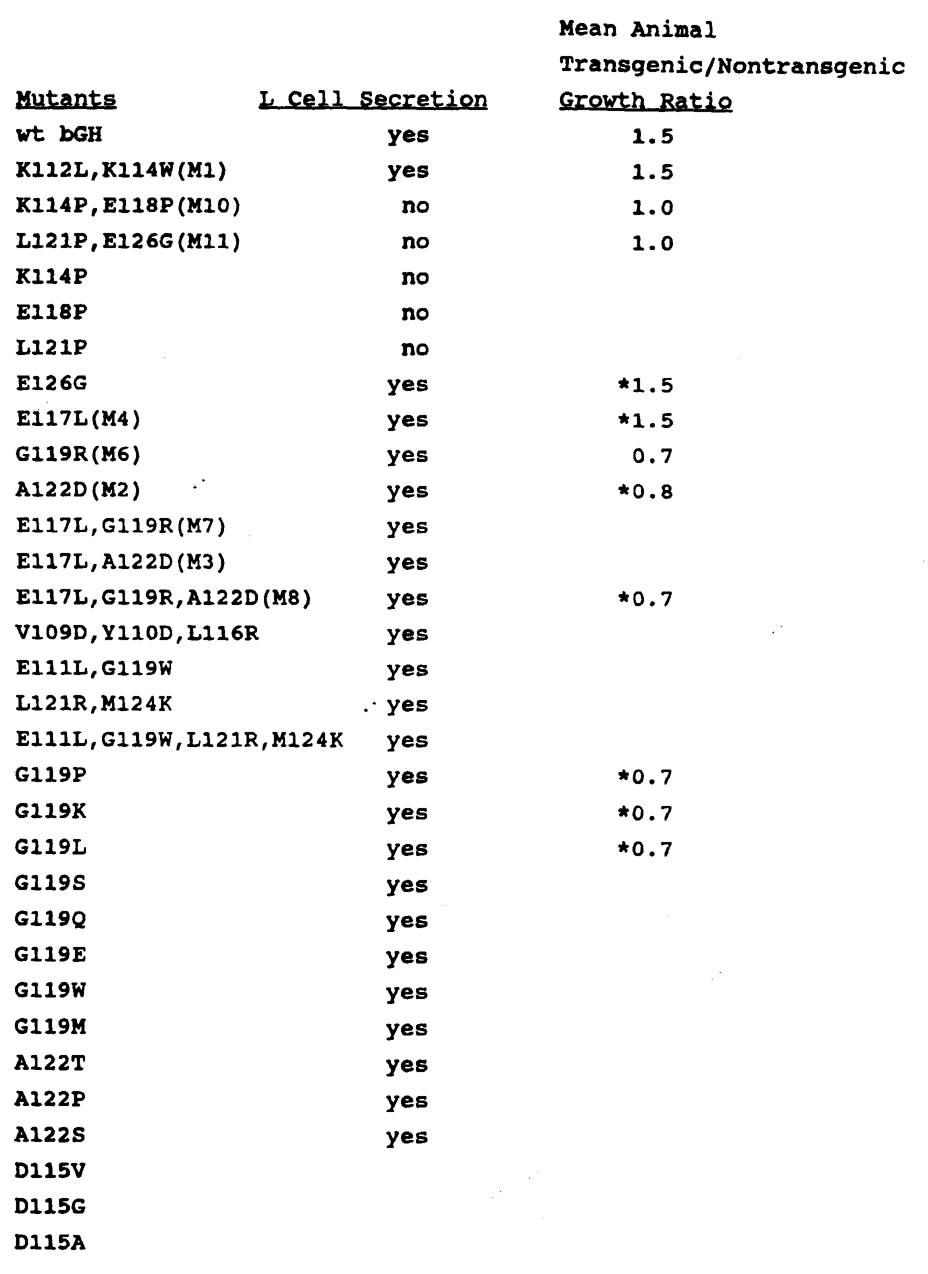

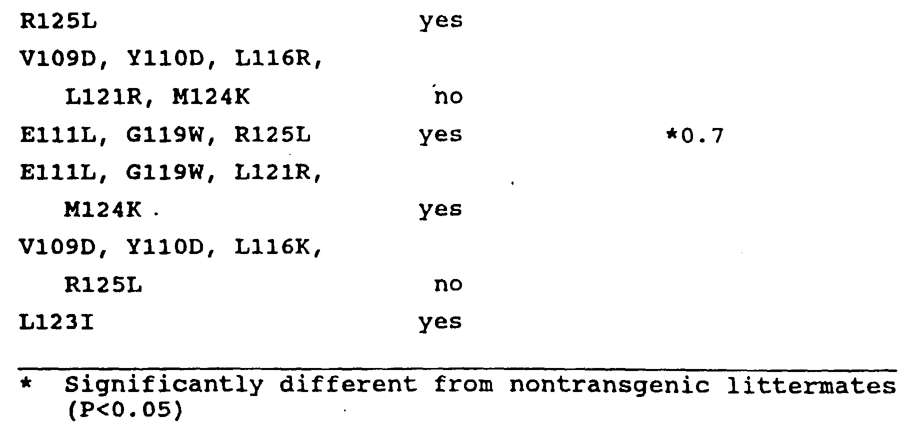

The mutants are described by giving the original amino acid, its position in the amino acid sequence of bGH, and the replacement amino acid, with the amino acids set forth according to the internationally accepted single letter code. George, et al., Protein Seq. Data Anal., 1:27-39 (1987).

The mutant K112L, K114W shows the effect of expanding the hydrophobic face of the helix. This mutant affects animal growth much as does wild type growth hormone.

The mutations K114P, E118P and L121P (and various combinations thereof) apparently destroy the alpha helix (Proline is a strong alpha helix breaker.) The growth-related biological activity is abolished. The mutation E126G is a special case; glycine is a helix breaker, but position 126 is at the end of the helix so the normal biological activity is retained. So, too is G119P; one strong helix breaker was substituted for an even stronger one.

The third alpha helix of wild type growth hormone diverges from a perfect amphiphilic alpha helix at three positions. First, at 117, Glu is a hydrophilic amino acid in the hydrophobic face. Second, at 119, Gly is a neutral amino acid in the hydrophilic face. Finally, at 122, Ala is a hydrophobic amino acid in the hydrophilic face. The mutations E117L, G119R and A122D, separately or in combination, increase the amphiphilic character of the helix. G119R additionally increases the alpha-helical tendencies of the sequence.

Our initial hypothesis was that the growth-inhibitory activity of the mutants G119R and E117L/G119R/A122D was associated with the increased amphipathicity of the third alpha helix. We have since developed evidence that the amphipathicity of the third alpha helix is largely irrelevant to that activity.
(1) The single E117L, like wt bGH, produced large animals.
(2) Mutant G119P produced the small animal phenotype even though praline is as hydrophilic as glycine.
(3) Mutant G119L produced the small animal phenotype even though leucine is hydrophobic and therefore disrupts the hydrophilic face of the helix.
(54) Mutant E111L/G119W/R125L produced the small animal phenotype even though all three mutations disrupt the hydrophilic face of the helix.

Thus, in one embodiment, the present invention relates to mutations of the third alpha helix which result in growth-inhibitory activity yet reduce or leave unchanged the amphiphilic character of the helix.

To summarize, residues 119 and 122 are important to the growth response, but alterations in these residues apparently did not affect receptor binding. Destruction of the helix by insertion of a helix-breaker at residues 114, 118 and 121 abolished both growth-affecting activity and, in cultured mouse cells, secretory activity. Residues 112, 114, 117 and 121 do not appear to be important in the growth response, at least so long as the helix is not destroyed.

Additional growth hormone antagonists may be identified by systematically varying the codon corresponding to G119 in bGH, so as to express the 18 other mutants having a single amino acid change at this position. This is readily accomplished by synthesizing oligonucleotides differing from those set forth in Example 1 at codon 119 so as to encode the desired alternative amino acid. By similar means, variations of the codons corresponding to amino acids 115 and 122 of bGH, or still other amino acids, are investigated.

The following table may be helpful in identifying candidate mutants:

| AA | Volume (angstrom³) | Alpha Helicity | Notes |
|---|---|---|---|
| Gly(G) | 60.1 | 0.53 | E126G(big), L121P/E126G(null) |
| Ala(A) | 88.6 | 1.45 | |
| Ser(S) | 89.0 | 0.79 | |
| Cys(C) | 108.5 | 0.77 | |
| Asp(D) | 111.1 | 0.98 | A122D(sm) |
| Thr(T) | 116.1 | 0.82 | |
| Asn(N) | 117.7 | 0.73 | |
| Pro(P) | 122.7 | 0.59 | G119P(sm), K114P/E118P(null), L121P/E126G(null) |
| Glu(E) | 138.4 | 1.53 | |
| Val(V) | 140.0 | 1.14 | |
| Gln(G) | 143.9 | 1.17 | |
| His(H) | 153.2 | 1.24 | |
| Met(M) | 162.9 | 1.20 | |
| Ile(I) | 166.7 | 1.00 | |
| Leu(L) | 166.7 | 1.34 | G119L(sm), E117L(big), E117L/G119R/A122D(sm), E111L/G119W/E125L (sm) |
| Lys(K) | 168.6 | 1.07 | G119K(sm) |
| Arg(R) | 173.4 | 0.79 | G119R(sm) |
| Phe(F) | 189.9 | 1.12 | |
| Tyr(Y) | 193.6 | 0.61 | |
| Trp(W) | 227.8 | 1.14 | E111L/G119W/R125L (sm) |

See Schulz and Schirmer, Principles of Protein Structure, Tables 6-1 (1979), for alpha-helicity and see Creighton, Proteins: Structures and Molecular Properties, Table 1-1 (1983) for volume of amino acids. Table 1-2 of Creighton also gives references for the detailed geometries of the amino acids. In notes, "sm" = small animal, "big" = big animal, "null" = no effect on growth.

## Claims (Claims for the following Contracting State(s): AT, BE, CH, LI, DE, DK, GB, IT, LU, NL, SE)

1. An isolated peptide or protein of at least 11 amino acids having growth-inhibitory activity in a vertebrate and comprising a mutant alpha helix which is substantially homologous with but not identical to the third alpha helix of a vertebrate growth hormone, the said third alpha helix corresponding to the alpha helix formed by the amino acids at positions 109 to 126 of the naturally occuring form of bovine growth hormone (bGH), said naturally occuring form of bovine growth hormone being identical to the bovine growth hormone mutant sequence set forth in Figure 1 except for the presence of a glycine instead of the arginine shown at position 119 of the mutant sequence,
wherein the mutant alpha helix differs from the third alpha helix of the vertebrate growth hormone at least at the position corresponding to position 119 of said naturally occurring form of bovine growth hormone,
with the proviso that the protein or peptide is neither
(a) the bovine somatotropin having the amino acid sequence of Chart 1 of WO 91/00870 (=D1), but having glycine at position 119 replaced by proline; nor
(b) a mutant, hPRL(111-129), of full length native human growth hormone ( hGH) having the sequence shown in Fig. 1 of Cunningham et al., Science 243:1330-36, 1989 (=D7), which mutant differs therefrom solely by the 12 substitutions as indicated for hPRL(111-129) in Table 1 of D7.

2. A peptide or protein of claim 1 which is the same size as or l arger than said vertebrate growth hormone.

3. An isolated peptide or protein of at least 11 amino acids which is a fragment of a protein having growth-inhibitory activity in a vertebrate and comprising a mutant alpha helix which is substantially homologous with but not identical to the third alpha helix of a vertebrate growth hormone, the said third alpha helix corresponding to the alpha helix formed by the amino acids at positions 109 to 126 of the naturally occuring form of bovine growth hormone (bGH), said naturally occuring form of bovine growth hormone being identical to the bovine growth hormone mutant sequence set forth in Figure 1 except for the presence of a glycine instead of the arginine shown at position 119 of the mutant sequence,
wherein the mutant alpha helix differs from the third alpha helix of the vertebrate growth hormone at at least at the position corresponding to position 119 of said naturally occurring form of bovine growth hormone,
and wherein said isolated peptide or protein has an amino acid sequence which is truncated relative to that of said vertebrate growth hormone.

4. A peptide or protein according to any one of claims 1 to 3 wherein the vertebrate growth hormone is a mammalian growth hormone.

5. A peptide or protein according to any one of claims 1 to 4, wherein the vertebrate growth hormone is bovine growth hormone.

6. A peptide or protein according to any one of claims 1 to 4, wherein the vertebrate growth hormone is human growth hormone.

7. A peptide or protein according to any one of claims 1 to 3, wherein the vertebrate growth hormone is chicken growth hormone.

8. A peptide or protein according to any one of claims 1 to 4, wherein the vertebrate growth hormone is rat, porcine or ovine growth hormone.

9. A peptide or protein according to any one of claims 1 to 3, wherein the vertebrate growth hormone is flounder, yellowtail, tuna or salmon growth hormone.

10. A peptide or protein according to any one of claims 1 to 3 which is substantially homologous with said naturally occurring form of bovine growth hormone.

11. A peptide or protein of any of claims 1 to 3 which is substantially homologous with a vertebrate growth hormone.

12. A peptide or protein of any of claims 1 to 3 which is substantially homologous with a mammalian growth hormone.

13. A peptide or protein according to any one of claims 1 to 12, wherein the amino acid at the position corresponding to position 119 of said naturally occurring form of bovine growth hormone is not alanine.

14. A peptide or protein according to any of claims 1 to 13 wherein the amino acid at the position corresponding to position 119 of said naturally occurring form of bovine growth hormone is arginine.

15. A peptide or protein according to any of claims 1 to 13 wherein the amino acid at the position corresponding to position 119 of said naturally occurring form of bovine growth hormone is proline.

16. A peptide or protein according to any of claims 1 to 13 wherein the amino acid at the position corresponding to position 119 of said naturally occurring form of bovine growth hormone is lysine.

17. A peptide or protein according to any of claims 1 to 13 wherein the amino acid at the position corresponding to position 119 of said naturally occurring form of bovine growth hormone is leucine.

18. A peptide or protein according to any of claims 1 to 13 wherein the amino acid at the position corresponding to position 119 of said naturally occurring form of bovine growth hormone is serine.

19. A peptide or protein according to any of claims 1 to 13 wherein the amino acid at the position corresponding to position 119 of said naturally occurring form of bovine growth hormone is glutamine.

20. A peptide or protein according to any of claims 1 to 13 wherein the amino acid at the position corresponding to position 119 of said naturally occurring form of bovine growth hormone is glutamic acid.

21. A peptide or protein according to any of claims 1 to 13 wherein the amino acid at the position corresponding to position 119 of said naturally occurring form of bovine growth hormone is tryptophan.

22. A peptide or protein according to any of claims 1 to 13 wherein the amino acid at the position corresponding to position 119 of said naturally occurring form of bovine growth hormone is methionine.

23. An isolated protein selected from the group consisting of
bGH (G119R),
bGH (G119R, E117L, A122D),
bGH (G119K),
bGH (G119L),
fragments of those proteins, and
fragments of bGH (G119P)
having growth-inhibitory activity in a vertebrate, wherein bGH is the naturally occurring form of bovine growth hormone (bGH) that is identical to the bovine growth hormone mutant sequence set forth in Figure 1 but for the presence of a glycine instead of the arginine shown at position 119 of the mutant sequence.

24. A peptide or protein of claim 3 which lacks at least one alpha helix selected from the group consisting of helices corresponding to the first, second or fourth helixes of said naturally occurring form of bovine growth hormone.

25. A peptide or protein of any of claims 1 to 24 which further differs from said vertebrate growth hormone by one or more substitutions at amino acids corresponding to amino acids 115 or 122 of said naturally occurring bovine growth hormone, and/or to amino acids of said naturally occurring bovine growth hormone which are not conserved among vertebrate growth hormones, and/by truncation of amino acids corresponding to amino acids 1-95 and 134-191 of said naturally occurring bovine growth hormone.

26. A peptide or protein of any of claims 1 to 25, wherein the peptide or protein has an ED50 which is less than about 10 times the ED50 of wild type growth hormone in an assay of the ability of the peptide or protein to displace radio labelled wild type growth hormone from a mouse liver membrane receptor.

27. A peptide or protein of any of claims 1 to 26, wherein the peptide or protein has growth-inhibitory activity in a mammal.

28. A recombinant DNA molecule comprising a gene including a sequence encoding the peptide or protein of any of claims 1 to 27.

29. A molecule of claim 28, further comprising a promoter operably linked to said gene.

30. A molecule of claim 29, where said promoter is not derived from the promoter of a growth hormone gene.

31. A cell comprising the recombinant DNA molecule of any one of claims 28 to 30.

32. A cell of claim 31, where said cell is one which does express a chromosomal growth hormone gene.

33. A non-human transgenic animal comprising a plurality of cells comprising a gene operably linked to a promoter, the gene including a sequence encoding a peptide or protein of at least 11 amino acids having growth-inhibitory activity in a vertebrate and comprising a mutant alpha helix which is substantially homologous with but not identical to the third alpha helix of a vertebrate growth hormone, the said third alpha helix corresponding to the alpha helix formed by the amino acids at positions 109 to 126 of the naturally occuring form of bovine growth hormone (bGH), said naturally occuring form of bovine growth hormone being identical to the bovine growth hormone mutant sequence set forth in Figure 1 except for the presence of a glycine instead of the arginine shown at position 119 of the mutant sequence,
wherein the mutant alpha helix differs from the third alpha helix of the vertebrate growth hormone at least at the position corresponding to position 119 of said naturally occurring form of bovine growth hormone.

34. A non-human transgenic animal as claimed in claim 33, wherein a plurality of cells comprise a gene including a sequence encoding a protein or peptide according to any one of claims 1 to 27.

35. A non-human animal of claim 33 or claim 34, which exhibits a reduced growth rate as compared to control animals.

36. A method of producing a growth inhibitory peptide or protein which comprises providing cells bearing a gene including a sequence encoding a peptide or protein according to any one of claims 1 to 27 with conditions conducive to the expression of said gene, whereby said protein is produced in a usable or recoverable form.

37. A pharmaceutical composition comprising the peptide or protein of any of claims 1 to 27 and a pharmaceutically acceptable carrier.

38. A peptide or protein of at least 11 amino acids having growth-inhibitory activity in a vertebrate and comprising a mutant alpha helix which is substantially homologous with but not identical to the third alpha helix of a vertebrate growth hormone, the said third alpha helix corresponding to the alpha helix formed by the amino acids at positions 109 to 126 of the naturally occuring form of bovine growth hormone (bGH), said naturally occuring form of bovine growth hormone being identical to the bovine growth hormone mutant sequence set forth in Figure 1 except for the presence of a glycine instead of the arginine shown at position 119 of the mutant sequence,
wherein the mutant alpha helix differs from the third alpha helix of the vertebrate growth hormone at least at the position corresponding to position 119 of said naturally occurring form of bovine growth hormone,
for use as a medicament,
with the proviso that the protein or peptide is neither
(a) the bovine somatotropin having the amino acid sequence of Chart 1 of WO 91/00870 (=D1), but having glycine at position 119 replaced by proline; nor
(b) a mutant, hPRL(111-129), of full length native human growth hormone (hGH) having the sequence shown in Fig. 1 of Cunningham et al., Science 243:1330-36, 1989 (=D7), which mutant differs therefrom solely by the 12 substitutions as indicated for hPRL(111-129) in Table 1 of D7.

39. A peptide or protein according to any one of claims 1 to 27, or a peptide or protein according to claim 38 having the properties first recited in any one of claims 2 to 27, for use as a medicament.

40. A recombinant DNA molecule comprising a gene including a sequence encoding a peptide or protein of at least 11 amino acids having growth-inhibitory activity in a vertebrate and comprising a mutant alpha helix which is substantially homologous with but not identical to the third alpha helix of a vertebrate growth hormone, the said third alpha helix corresponding to the alpha helix formed by the amino acids at positions 109 to 126 of the naturally occuring form of bovine growth hormone (bGH), said naturally occuring form of bovine growth hormone being identical to the bovine growth hormone mutant sequence set forth in Figure 1 except for the presence of a glycine instead of the arginine shown at position 119 of the mutant sequence,
wherein the mutant alpha helix differs from the third alpha helix of the vertebrate growth hormone at least at the position corresponding to position 119 of said naturally occurring form of bovine growth hormone,
for use as a medicament.

41. A recombinant DNA molecule as claimed in any one of claims 28 to 30, or a recombinant DNA molecule according to claim 40 encoding a peptide or protein having the properties first recited in any one of claims 2 to 27, for use as a medicament.

42. A recombinant molecule as claimed in claim 40 or 41 f or inhibiting or reducing growth hormone activity in a human or animal subject.

43. A DNA molecule according to any one of claims 40 to 42, whereby a resulting genetically modified human or animal subject is capable of expressing the protein or peptide according to any of claims 1 to 27 or 38 at a growth inhibitory level.

44. Use of a peptide or protein of at least 11 amino acids having growth-inhibitory activity in a vertebrate and comprising a mutant alpha helix which is substantially homologous with but not identical to the third alpha helix of a vertebrate growth hormone, the said third alpha helix corresponding to the alpha helix formed by the amino acids at positions 109 to 126 of the naturally occuring form of bovine growth hormone (bGH), said naturally occuring form of bovine growth hormone being identical to the bovine growth hormone mutant sequence set forth in Figure 1 except for the presence of a glycine instead of the arginine shown at position 119 of the mutant sequence, wherein the mutant alpha helix differs from the third alpha helix of the vertebrate growth hormone at least at the position corresponding to position 119 of said naturally occurring form of bovine growth hormone,
in the manufacture of a medicament for the treatment of a condition improvable by inhibiting or reducing growth hormone activity.

45. Use of a peptide or protein of any one of claims 1 to 27, or use of a peptide or protein according to claim 44 having the properties first recited in any one of claims 2 to 27, in the manufacture of a medicament for the treatment of a condition improvable by inhibiting or reducing growth hormone activity.

46. Use of a recombinant DNA molecule comprising a gene including a sequence encoding a peptide or protein of at least 11 amino acids having growth-inhibitory activity in a vertebrate and comprising a mutant alpha helix which is substantially homologous with but not identical to the third alpha helix of a vertebrate growth hormone, the said third alpha helix corresponding to the alpha helix formed by the amino acids at positions 109 to 126 of the naturally occuring form of bovine growth hormone (bGH), said naturally occuring form of bovine growth hormone being identical to the bovine growth hormone mutant sequence set forth in Figure 1 except for the presence of a glycine instead of the arginine shown at position 119 of the mutant sequence,
wherein the mutant alpha helix differs from the third alpha helix of the vertebrate growth hormone at least at the position corresponding to position 119 of said naturally occurring form of bovine growth hormone,
in the manufacture of a composition for the treatment of a condition improvable by inhibiting or reducing growth hormone activity.

47. Use of a recombinant DNA molecule according to any one of claims 28 to 30, or use of a recombinant DNA molecule according to claim 46 encoding a peptide or protein having the properties first recited in any one of claims 2 to 27, in the manufacture of a composition for the treatment of a condition improvable by inhibiting or reducing growth hormone activity.

48. The peptide or protein of claim 3 which is of at least 50 amino acids.

49. The peptide or protein of any of claims 1 to 27 which has growth-inhibitory activity in a human.

50. The peptide or protein of claim 1, wherein the amino acid at the position corresponding to position 119 of said naturally occurring form of bovine growth hormone is a hydrophobic amino acid, according to the scale of Hopp and Woods, PNAS (USA) 78: 3824-28 (1981).

51. The peptide or protein of claim 1, wherein the amphiphilic character of the mutant alpha helix, as determined by the method of Margalit et al., J. Immunol. 138: 2213-29 (1987), is reduced or unchanged relative to the amphiphilic character of the third alpha helix at positions 109 to 126 of said bovine growth hormone.

52. A peptide or protein according to claim 50 or 51 for use as a medicament.

53. Use of the peptide or protein of claim 50 or 51 in the manufacture of a medicament for the treatment of a condition improvable by inhibiting or reducing growth hormone activity.

## Claims (Claims for the following Contracting State(s): GR)

1. A recombinant DNA molecule comprising a gene including a sequence encoding a peptide or protein of at least 11 amino acids having growth-inhibitory activity in a vertebrate and comprising a mutant alpha helix which is substantially homologous with but not identical to the third alpha helix of a vertebrate growth hormone, the said third alpha helix corresponding to the alpha helix formed by the amino acids at positions 109 to 126 of the naturally occuring form of bovine growth hormone (bGH), said naturally occuring form of bovine growth hormone being identical to the bovine growth hormone mutant sequence set forth in Figure 1 except for the presence of a glycine instead of the arginine shown at position 119 of the mutant sequence,
wherein the mutant alpha helix differs from the third alpha helix of the vertebrate growth hormone at least at the position corresponding to position 119 of said naturally occurring form of bovine growth hormone,
with the proviso that the protein or peptide is neither
(a) the bovine somatotropin having the amino acid sequence of Chart 1 of WO 91/00870 (=D1), but having glycine at position 119 replaced by proline; nor
(b) a mutant, hPRL(111-129), of full length native human growth hormone (hGH) having the sequence shown in Fig. 1 of Cunningham et al., Science 243:1330-36, 1989 (=D7), which mutant differs therefrom solely by the 12 substitutions as indicated for hPRL(111-129) in Table 1 of D7.

2. A molecule according to claim 1 which is the same size as or larger than said vertebrate growth hormone.

3. A recombinant DNA molecule comprising a gene including a sequence encoding a peptide or protein of at least 11 amino acids which is a fragment of a protein having growth-inhibitory activity in a vertebrate and comprising a mutant alpha helix which is substantially homologous with but not identical to the third alpha helix of a vertebrate growth hormone, the said third alpha helix corresponding to the alpha helix formed by the amino acids at positions 109 to 126 of the naturally occuring form of bovine growth hormone (bGH), said naturally occuring form of bovine growth hormone being identical to the bovine growth hormone mutant sequence set forth in Figure 1 except for the presence of a glycine instead of the arginine shown at position 119 of the mutant sequence,

4. A molecule according to any one of claims 1 to 3 wherein the vertebrate growth hormone is a mammalian growth hormone.

5. A molecule according to any one of claims 1 to 4, wherein the vertebrate growth hormone is bovine growth hormone.

6. A molecule according to any one of claims 1 to 4, wherein the vertebrate growth hormone is human growth hormone.

7. A molecule according to any one of claims 1 to 3, wherein the vertebrate growth hormone is chicken growth hormone.

8. A molecule according to any one of claims 1 to 4, wherein the vertebrate growth hormone is rat, porcine or ovine growth hormone.

9. A molecule according to any one of claims 1 to 3, wherein the vertebrate growth hormone is flounder, yellowtail, tuna or salmon growth hormone.

10. A molecule according to any one of claims 1 to 3, wherein the peptide or protein is substantially homologous with said naturally occurring form of bovine growth hormone.

11. A molecule according to any of claims 1 to 3, wherein the peptide or protein is substantially homologous with a vertebrate growth hormone.

12. A molecule according to any of claims 1 to 3, wherein the peptide or protein is substantially homologous with a mammalian growth hormone.

13. A molecule according to any one of claims 1 to 12, wherein the amino acid encoded at the position corresponding to position 119 of said naturally occurring form of bovine growth hormone is not alanine.

14. A molecule according to any of claims 1 to 13 wherein the amino acid encoded at the position corresponding to position 119 of said naturally occurring form of bovine growth hormone is arginine.

15. A molecule according to any of claims 1 to 13 wherein the amino acid encoded at the position corresponding to position 119 of said naturally occurring form of bovine growth hormone is proline.

16. A molecule according to any of claims 1 to 13 wherein the amino acid encoded at the position corresponding to position 119 of said naturally occurring form of bovine growth hormone is lysine.

17. A molecule according to any of claims 1 to 13 wherein the amino acid encoded at the position corresponding to position 119 of said naturally occurring form of bovine growth hormone is leucine.

18. A molecule according to any of claims 1 to 13 wherein the amino acid encoded at the position corresponding to position 119 of said naturally occurring form of bovine growth hormone is serine.

19. A molecule according to any of claims 1 to 13 wherein the amino acid encoded at the position corresponding to position 119 of said naturally occurring form of bovine growth hormone is glutamine.

20. A molecule according to any of claims 1 to 13 wherein the amino acid encoded at the position corresponding to position 119 of said naturally occurring form of bovine growth hormone is glutamic acid.

21. A molecule according to any of claims 1 to 13 wherein the amino acid encoded at the position corresponding to position 119 of said naturally occurring form of bovine growth hormone is tryptophan.

22. A molecule according to any of claims 1 to 13 wherein the amino acid encoded at the position corresponding to position 119 of said naturally occurring form of bovine growth hormone is methionine.

23. A molecule according to claim 1, wherein the peptide or protein is selected from the group consisting of
bGH (G119R),
bGH (G119R, E117L, A122D),
bGH (G119K),
bGH (G119L),
fragments of those proteins, and
fragments of bGH(G119P)
having growth-inhibitory activity in a vertebrate, wherein bGH is the naturally occurring form of bovine growth hormone (bGH) that is identical to the bovine growth hormone mutant sequence set forth in Figure 1 but for the presence of a glycine instead of the arginine shown at position 119 of the mutant sequence.

24. A molecule according to claim 3, wherein the peptide or protein lacks at least one alpha helix selected from the group consisting of helices corresponding to the first, second or fourth helixes of said naturally occurring form of bovine growth hormone.

25. A molecule according to any of claims 1-24, wherein the peptide or protein further differs from said vertebrate growth hormone by one or more substitutions at amino acids corresponding to amino acids 115 or 122 of said naturally occurring bovine growth hormone, and/or to amino acids of said naturally occurring bovine growth hormone which are not conserved among vertebrate growth hormones, and/by truncation of amino acids corresponding to amino acids 1-95 and 134-191 of said naturally occurring bovine growth hormone.

26. A molecule according to any of claims 1 to 25, wherein the peptide or protein has an ED50 which is less than about 10 times the ED50 of wild type growth hormone in an assay of the ability of the peptide or protein to displace radio labelled wild type growth hormone from a mouse liver membrane receptor.

27. A molecule according to any of claims 1 to 26, wherein the peptide or protein has growth-inhibitory activity in a mammal.

28. A molecule according to any of claims 1 to 27, further comprising a promoter operably linked to said gene.

29. A molecule according to claim 28, where said promoter is not derived from the promoter of a growth hormone gene.

30. A cell comprising the recombinant DNA molecule of any one of claims 1 to 29.

31. A cell according to claim 30, where said cell is one which does express a chromosomal growth hormone gene.

32. An isolated peptide or protein of at least 11 amino acids having growth-inhibitory activity in a vertebrate and comprising a mutant alpha helix which is substantially homologous with but not identical to the third alpha helix of a vertebrate growth hormone, the said third alpha helix corresponding to the alpha helix formed by the amino acids at positions 109 to 126 of the naturally occuring form of bovine growth hormone (bGH), said naturally occuring form of bovine growth hormone being identical to the bovine growth hormone mutant sequence set forth in Figure 1 except for the presence of a glycine instead of the arginine shown at position 119 of the mutant sequence,
wherein the mutant alpha helix differs from the third alpha helix of the vertebrate growth hormone at least at the position corresponding to position 119 of said naturally occurring form of bovine growth hormone,
with the proviso that the protein or peptide is neither
(a) the bovine somatotropin having the amino acid sequence of Chart 1 of WO 91/00870 (=D1), but having glycine at position 119 replaced by proline; nor
(b) a mutant, hPRL(111-129), of full length native human growth hormone (hGH) having the sequence shown in Fig. 1 of Cunningham et al., Science 243:1330-36, 1989 (=D7), which mutant differs therefrom solely by the 12 substitutions as indicated for hPRL(111-129) in Table 1 of D7.

33. A peptide or protein according to claim 32, wherein the peptide or protein has properties as first recited in any one of claims 2-27.

34. A non-human transgenic animal comprising a plurality of cells comprising a gene operably linked to a promoter, the gene including a sequence encoding a peptide or protein of at least 11 amino acids having growth-inhibitory activity in a vertebrate and comprising a mutant alpha helix which is substantially homologous with but not identical to the third alpha helix of a vertebrate growth hormone, the said third alpha helix corresponding to the alpha helix formed by the amino acids at positions 109 to 126 of the naturally occuring form of bovine growth hormone (bGH), said naturally occuring form of bovine growth hormone being identical to the bovine growth hormone mutant sequence set forth in Figure 1 except for the presence of a glycine instead of the arginine shown at position 119 of the mutant sequence, wherein the mutant alpha helix differs from the third alpha helix of the vertebrate growth hormone at least at the position corresponding to position 119 of said naturally occurring form of bovine growth hormone.

35. A non-human transgenic animal as claimed in claim 34, wherein a plurality of cells comprise a recombinant DNA molecule according to any one of claims 1 to 29.

36. A non-human animal of claim 34 or claim 35, which exhibits a reduced growth rate as compared to control animals.

37. A method of producing a growth inhibitory peptide or protein which comprises providing cells bearing a gene including a sequence encoding a peptide or protein according to claim 32 or 33 with conditions conducive to the expression of said gene, whereby said protein is produced in a usable or recoverable form.

38. A pharmaceutical composition comprising the peptide or protein of claim 32 or 33 and a pharmaceutically acceptable carrier.

39. A process for making a pharmaceutical composition which comprises bringing into admixture a peptide or protein as defined in claim 32 or 33 and a pharmaceutically acceptable carrier.

40. A recombinant DNA molecule comprising a gene including a sequence encoding a peptide or protein of at least 11 amino acids having growth-inhibitory activity in a vertebrate and comprising a mutant alpha helix which is substantially homologous with but not identical to the third alpha helix of a vertebrate growth hormone, the said third alpha helix corresponding to the alpha helix formed by the amino acids at positions 109 to 126 of the naturally occuring form of bovine growth hormone (bGH), said naturally occuring form of bovine growth hormone being identical to the bovine growth hormone mutant sequence set forth in Figure 1 except for the presence of a glycine instead of the arginine shown at position 119 of the mutant sequence,
wherein the mutant alpha helix differs from the third alpha helix of the vertebrate growth hormone at least at the position corresponding to position 119 of said naturally occurring form of bovine growth hormone.
for use as a medicament.

41. A recombinant DNA molecule as claimed in any one of claims 1 to 29 for use as a medicament.

42. A recombinant DNA molecule as claimed in claim 40 or 41 for inhibiting or reducing growth hormone activity in a human or animal subject.

43. A recombinant DNA molecule according to any one of claims 40 to 42, whereby a resulting genetically modified human or animal subject is capable of expressing the protein or peptide according to any of claims 1 to 27 at a growth inhibitory level.

44. Use of a peptide or protein of at least 11 amino acids having growth-inhibitory activity in a vertebrate and comprising a mutant alpha helix which is substantially homologous with but not identical to the third alpha helix of a vertebrate growth hormone, the said third alpha helix corresponding to the alpha helix formed by the amino acids at positions 109 to 126 of the naturally occuring form of bovine growth hormone (bGH), said naturally occuring form of bovine growth hormone being identical to the bovine growth hormone mutant sequence set forth in Figure 1 except for the presence of a glycine instead of the arginine shown at position 119 of the mutant sequence,
wherein the mutant alpha helix differs from the third alpha helix of the vertebrate growth hormone at least at the position corresponding to position 119 of said naturally occurring form of bovine growth hormone,
in the manufacture of a medicament for the treatment of a condition improvable by inhibiting or reducing growth hormone activity.

45. Use of a peptide or protein as defined in any one of claims 1 to 27 in the manufacture of a medicament for the treatment of a condition improvable by inhibiting or reducing growth hormone activity.

46. Use of a recombinant DNA molecule comprising a gene including a sequence encoding a peptide or protein of at least 11 amino acids having growth-inhibitory activity in a vertebrate and comprising a mutant alpha helix which is substantially homologous with but not identical to the third alpha helix of a vertebrate growth hormone, the said third alpha helix corresponding to the alpha helix formed by the amino acids at positions 109 to 126 of the naturally occuring form of bovine growth hormone (bGH), said naturally occuring form of bovine growth hormone being identical to the bovine growth hormone mutant sequence set forth in Figure 1 except for the presence of a glycine instead of the arginine shown at position 119 of the mutant sequence,
wherein the mutant alpha helix differs from the third alpha helix of the vertebrate growth hormone at least at the position corresponding to position 119 of said naturally occurring form of bovine growth hormone,
in the manufacture of a composition for the treatment of a condition improvable by inhibiting or reducing growth hormone activity.

47. Use of a recombinant DNA molecule according to any one of claims 1 to 29 in the manufacture of a composition for the treatment of a condition improvable by inhibiting or reducing growth hormone activity.

48. A peptide or protein as defined in claim 3 which is of at least 50 amino acids.

49. A peptide or protein as defined in any of claims 1 to 27 which has growth-inhibitory activity in a human.

50. The peptide or protein of claim 1, wherein the amino acid at the position corresponding to position 119 of said naturally occurring form of bovine growth hormone is a hydrophobic amino acid, according to the scale of Hopp and Woods, PNAS (USA) 78: 3824-28 (1981).

51. The peptide or protein of claim 1, wherein the amphiphilic character of the mutant alpha helix, as determined by the method of Margalit et al., J. Immunol. 138: 2213-29 (1987), is reduced or unchanged relative to the amphiphilic character of the third alpha helix at positions 109 to 126 of said bovine growth hormone.

52. A peptide or protein according to claim 50 or 51 for use as a medicament.

53. Use of the peptide or protein of claim 50 or 51 in the manufacture of a medicament for the treatment of a condition improvable by inhibiting or reducing growth hormone activity.

54. A process for making a pharmaceutical composition which comprises bringing into admixture a peptide or protein as defined in claim 50 or 51 and a pharmaceutically acceptable carrier.

## Claims (Claims for the following Contracting State(s): ES)

1. A method of producing a growth inhibitory peptide or protein which comprises providing cells bearing a gene, which gene includes a sequence encoding a peptide or protein of at least 11 amino acids having growth-inhibitory activity in a vertebrate and comprising a mutant alpha helix which is substantially homologous with but not identical to the third alpha helix of a vertebrate growth hormone, the said third alpha helix corresponding to the alpha helix formed by the amino acids at positions 109 to 126 of the naturally occuring form of bovine growth hormone (bGH), said naturally occuring form of bovine growth hormone being identical to the bovine growth hormone mutant sequence set forth in Figure 1 except for the presence of a glycine instead of the arginine shown at position 119 of the mutant sequence,
wherein the mutant alpha helix differs from the third alpha helix of the vertebrate growth hormone at least at the position corresponding to position 119 of said naturally occurring form of bovine growth hormone,
with the proviso that the protein or peptide is neither
(a) the bovine somatotropin having the amino acid sequence of Chart 1 of WO 91/00870 (=D1), but having glycine at position 119 replaced by proline; nor
(b) a mutant, hPRL(111-129), of full length native human growth hormone (hGH) having the sequence shown in Fig. 1 of Cunningham et al., Science 243:1330-36, 1989 (=D7), which mutant differs therefrom solely by the 12 substitutions as indicated for hPRL(111-129) in Table 1 of D7,
with conditions conducive to the expression of said gene, whereby said protein is produced in a usable or recoverable form.

2. A method according to claim 1, which further includes the step of preparing the cells by transformation of host cells to cause them to bear said gene.

3. A method according to claim 1 or 2, wherein the peptide or protein is the same size as or larger than said vertebrate growth hormone.

4. A method of producing a growth inhibitory peptide or protein which comprises providing cells bearing a gene, which gene includes a sequence encoding a peptide or protein of at least 11 amino acids which is a fragment of a protein having growth-inhibitory activity in a vertebrate and comprising a mutant alpha helix which is substantially homologous with but not identical to the third alpha helix of a vertebrate growth hormone, the said third alpha helix corresponding to the alpha helix formed by the amino acids at positions 109 to 126 of the naturally occuring form of bovine growth hormone (bGH), said naturally occuring form of bovine growth hormone being identical to the bovine growth hormone mutant sequence set forth in Figure 1 except for the presence of a glycine instead of the arginine shown at position 119 of the mutant sequence,
with conditions conducive to the expression of said gene, whereby said protein is produced in a usable or recoverable form.

5. A method according to any one of claims 1 to 4, wherein the vertebrate growth hormone is a mammalian growth hormone.

6. A method according to any one of claims 1 to 5, wherein the vertebrate growth hormone is bovine growth hormone.

7. A method according to any one of claims 1 to 5, wherein the vertebrate growth hormone is human growth hormone.

8. A method according to any one of claims 1 to 4, wherein the vertebrate growth hormone is chicken growth hormone.

9. A method according to any one of claims 1 to 5, wherein the vertebrate growth hormone is rat, porcine or ovine growth hormone.

10. A method according to any one of claims 1 to 4, wherein the vertebrate growth hormone is flounder, yellowtail, tuna or salmon growth hormone.

11. A method according to any one of claims 1 to 4, wherein the peptide or protein is substantially homologous with said naturally occurring form of bovine growth hormone.

12. A method according to any of claims 1 to 4, wherein the peptide or protein is substantially homologous with a vertebrate growth hormone.

13. A method according to any of claims 1 to 4, wherein the peptide or protein is substantially homologous with a mammalian growth hormone.

14. A method according to any one of claims 1 to 13, wherein the amino acid encoded at the position corresponding to position 119 of said naturally occurring form of bovine growth hormone is not alanine.

15. A method according to any of claims 1 to 14 wherein the amino acid encoded at the position corresponding to position 119 of said naturally occurring form of bovine growth hormone is arginine.

16. A method according to any of claims 1 to 14 wherein the amino acid encoded at the position corresponding to position 119 of said naturally occurring form of bovine growth hormone is proline.

17. A method according to any of claims 1 to 14 wherein the amino acid encoded at the position corresponding to position 119 of said naturally occurring form of bovine growth hormone is lysine.

18. A method according to any of claims 1 to 14 wherein the amino acid encoded at the position corresponding to position 119 of said naturally occurring form of bovine growth hormone is leucine.

19. A method according to any of claims 1 to 14 wherein the amino acid encoded at the position corresponding to position 119 of said naturally occurring form of bovine growth hormone is serine.

20. A method according to any of claims 1 to 14 wherein the amino acid encoded at the position corresponding to position 119 of said naturally occurring form of bovine growth hormone is glutamine.

21. A method according to any of claims 1 to 14 wherein the amino acid encoded at the position corresponding to position 119 of said naturally occurring form of bovine growth hormone is glutamic acid.

22. A method according to any of claims 1 to 14 wherein the amino acid encoded at the position corresponding to position 119 of said naturally occurring form of bovine growth hormone is tryptophan.

23. A method according to any of claims 1 to 14 wherein the amino acid encoded at the position corresponding to position 119 of said naturally occurring form of bovine growth hormone is methionine.

24. A method according to claim 1, wherein the peptide or protein is selected from the group consisting of
bGH (G119R),
bGH (G119R, E117L, A122D),
bGH (G119K),
bGH (G119L),
fragments of those proteins, and
fragments of bGH (G119P)
having growth-inhibitory activity in a vertebrate, wherein bGH is the naturally occurring form of bovine growth hormone (bGH) that is identical to the bovine growth hormone mutant sequence set forth in Figure 1 but for the presence of a glycine instead of the arginine shown at position 119 of the mutant sequence.

25. A method according to claim 4, wherein the peptide or protein lacks at least one alpha helix selected from the group consisting of helices corresponding to the first, second or fourth helixes of said naturally occurring form of bovine growth hormone.

26. A method according to any of claims 1 to 25, wherein the peptide or protein further differs from said vertebrate growth hormone by one or more substitutions at amino acids corresponding to amino acids 115 or 122 of said naturally occurring bovine growth hormone, and/or to amino acids of said naturally occurring bovine growth hormone which are not conserved among vertebrate growth hormones, and/by truncation of amino acids corresponding to amino acids 1-95 and 134-191 of said naturally occurring bovine growth hormone.

27. A method according to any of claims 1 to 26, wherein the peptide or protein has an ED50 which is less than about 10 times the ED50 of wild type growth hormone in an assay of the ability of the peptide or protein to displace radio labelled wild type growth hormone from a mouse liver membrane receptor.

28. A method according to any of claims 1 to 27, wherein the peptide or protein has growth-inhibitory activity in a mammal.

29. A process for making a pharmaceutical composition which comprises bringing into admixture a peptide or protein as defined in any one of claims 1 to 28 and a pharmaceutically acceptable carrier.

30. A recombinant DNA molecule comprising a gene including a sequence encoding a peptide or protein as defined in any one of claims 1 to 20.

31. A molecule according to claim 30, further comprising a promoter operably linked to said gene.

32. A molecule according to claim 31, where said promoter is not derived from the promoter of a growth hormone gene.

33. A cell comprising the recombinant DNA molecule according to any one of claims 30 to 32.

34. A cell according to claim 33, where said cell is one which does express a chromosomal growth hormone gene.

35. A non-human transgenic animal comprising a plurality of cells comprising a gene operably linked to a promoter, the gene including a sequence encoding a peptide or protein of at least 11 amino acids having growth-inhibitory activity in a vertebrate and comprising a mutant alpha helix which is substantially homologous with but not identical to the third alpha helix of a vertebrate growth hormone, the said third alpha helix corresponding to the alpha helix formed by the amino acids at positions 109 to 126 of the naturally occuring form of bovine growth hormone (bGH), said naturally occuring form of bovine growth hormone being identical to the bovine growth hormone mutant sequence set forth in Figure 1 except for the presence of a glycine instead of the arginine shown at position 119 of the mutant sequence, wherein the mutant alpha helix differs from the third alpha helix of the vertebrate growth hormone at least at the position corresponding to position 119 of said naturally occurring form of bovine growth hormone.

36. A non-human transgenic animal as claimed in claim 35, wherein a plurality of cells comprise a recombinant DNA molecule according to any one of claims 30 to 32.

37. A non-human animal according to claim 35 or claim 36, which exhibits a reduced growth rate as compared to control animals.

38. A pharmaceutical composition comprising a peptide or protein as defined in any one of claims 1 to 28 and a pharmaceutically acceptable carrier.

39. A peptide or protein as defined in any one of claims 1 to 29 for use as a medicament.

40. A recombinant DNA molecule comprising a gene including a sequence encoding a peptide or protein of at least 11 amino acids having growth-inhibitory activity in a vertebrate and comprising a mutant alpha helix which is substantially homologous with but not identical to the third alpha helix of a vertebrate growth hormone, the said third alpha helix corresponding to the alpha helix formed by the amino acids at positions 109 to 126 of the naturally occuring form of bovine growth hormone (bGH), said naturally occuring form of bovine growth hormone being identical to the bovine growth hormone mutant sequence set forth in Figure 1 except for the presence of a glycine instead of the arginine shown at position 119 of the mutant sequence,
wherein the mutant alpha helix differs from the third alpha helix of the vertebrate growth hormone at least at the position corresponding to position 119 of said naturally occurring form of bovine growth hormone,
for use as a medicament.

41. A recombinant DNA molecule as claimed in any one of claims 30 to 32 for use as a medicament.

42. A recombinant DNA molecule as claimed in claim 40 or 41 for inhibiting or reducing growth hormone activity in a human or animal subject.

43. A recombinant DNA molecule according to any one of claims 40 to 42, whereby a resulting genetically modified human or animal subject is capable of expressing the protein or peptide according to any of claims 1 to 27 at a growth inhibitory level.

44. A method for manufacturing a medicament for the treatment of a condition improvable by inhibiting or reducing growth hormone activity, characterised in the use of a peptide or protein of at least 11 amino acids having growth-inhibitory activity in a vertebrate and comprising a mutant alpha helix which is substantially homologous with but not identical to the third alpha helix of a vertebrate growth hormone, the said third alpha helix corresponding to the alpha helix formed by the amino acids at positions 109 to 126 of the naturally occuring form of bovine growth hormone (bGH), said naturally occuring form of bovine growth hormone being identical to the bovine growth hormone mutant sequence set forth in Figure 1 except for the presence of a glycine instead of the arginine shown at position 119 of the mutant sequence, wherein the mutant alpha helix differs from the third alpha helix of the vertebrate growth hormone at least at the position corresponding to position 119 of said naturally occurring form of bovine growth hormone.

45. A method for manufacturing a medicament for the treatment of a condition improvable by inhibiting or reducing growth hormone activity, characterised in the use of a peptide or protein as defined in any one of claims 1 to 28.

46. Use of a peptide or protein of at least 11 amino acids having growth-inhibitory activity in a vertebrate and comprising a mutant alpha helix which is substantially homologous with but not identical to the third alpha helix of a vertebrate growth hormone, the said third alpha helix corresponding to the alpha helix formed by the amino acids at positions 109 to 126 of the naturally occuring form of bovine growth hormone (bGH), said naturally occuring form of bovine growth hormone being identical to the bovine growth hormone mutant sequence set forth in Figure 1 except for the presence of a glycine instead of the arginine shown at position 119 of the mutant sequence,
wherein the mutant alpha helix differs from the third alpha helix of the vertebrate growth hormone at least at the position corresponding to position 119 of said naturally occurring form of bovine growth hormone,
in the manufacture of a medicament for the treatment of a condition improvable by inhibiting or reducing growth hormone activity.

47. A method for manufacturing a composition for the treatment of a condition improvable by inhibiting or reducing growth hormone activity, characterised in the use of a recombinant DNA molecule according to any one of claims 30 to 32.

48. Use of a recombinant DNA molecule comprising a gene including a sequence encoding a peptide or protein of at least 11 amino acids having growth-inhibitory activity in a vertebrate and comprising a mutant alpha helix which is substantially homologous with but not identical to the third alpha helix of a vertebrate growth hormone, the said third alpha helix corresponding to the alpha helix formed by the amino acids at positions 109 to 126 of the naturally occuring form of bovine growth hormone (bGH), said naturally occuring form of bovine growth hormone being identical to the bovine growth hormone mutant sequence set forth in Figure 1 except for the presence of a glycine instead of the arginine shown at position 119 of the mutant sequence,
wherein the mutant alpha helix differs from the third alpha helix of the vertebrate growth hormone at least at the position corresponding to position 119 of said naturally occurring form of bovine growth hormone,
in the manufacture of a composition for the treatment of a condition improvable by inhibiting or reducing growth hormone activity.

49. An isolated peptide or protein of at least 11 amino acids having growth-inhibitory activity in a vertebrate and comprising a mutant alpha helix which is substantially homologous with but not identical to the third alpha helix of a vertebrate growth hormone, the said third alpha helix corresponding to the alpha helix formed by the amino acids at positions 109 to 126 of the naturally occuring form of bovine growth hormone (bGH), said naturally occuring form of bovine growth hormone being identical to the bovine growth hormone mutant sequence set forth in Figure 1 except for the presence of a glycine instead of the arginine shown at position 119 of the mutant sequence,
wherein the mutant alpha helix differs from the third alpha helix of the vertebrate growth hormone at least at the position corresponding to position 119 of said naturally occurring form of bovine growth hormone,
with the proviso that the protein or peptide is neither
(a) the bovine somatotropin having the amino acid sequence of Chart 1 of WO 91/00870 (=D1), but having glycine at position 119 replaced by proline; nor
(b) a mutant, hPRL(111-129), of full length native human growth hormone (hGH) having the sequence shown in Fig. 1 of Cunningham et al., Science 243:1330-36, 1989 (=D7), which mutant differs therefrom solely by the 12 substitutions as indicated for hPRL(111-129) in Table 1 of D7.

50. A peptide or protein according to claim 49, wherein the peptide or protein has properties as first recited in any one of claims 2-28.

51. The peptide or protein of claim 1, wherein the amino acid at the position corresponding to position 119 of said naturally occurring form of bovine growth hormone is a hydrophobic amino acid, according to the scale of Hopp and Woods, PNAS (USA) 78: 3824-28 (1981).

52. The peptide or protein of claim 1, wherein the amphiphilic character of the mutant alpha helix, as determined by the method of Margalit et al., J. Immunol. 138: 2213-29 (1987), is reduced or unchanged relative to the amphiphilic character of the third alpha helix at positions 109 to 126 of said bovine growth hormone.

53. A peptide or protein according to claim 51 or 52 for use as a medicament.

54. Use of the peptide or protein of claim 51 or 52 in the manufacture of a medicament for the treatment of a condition improvable by inhibiting or reducing growth hormone activity.

55. A process for making a pharmaceutical composition which comprises bringing into admixture a peptide or protein as defined in claim 51 or 52 and a pharmaceutically acceptable carrier.

## Patentansprüche (Patentansprüche für folgende(n) Vertragsstaat(en): AT, BE, CH, LI, DE, DK, GB, IT, LU, NL, SE)

1. Isoliertes Peptid oder Protein mit mindestens 11 Aminosäuren, das Wachstumsinhibitoraktivität in einem Vertebraten aufweist und eine mutierte Alpha-Helix umfaßt, die im wesentlichen homolog, aber nicht identisch ist mit der dritten Alpha-Helix eines Vertebraten-Wachstumshormons, wobei die dritte Alpha-Helix der Alpha-Helix entspricht, die von den Aminosäuren an den Positionen 109 bis 126 der natürlich vorkommenden Form des Rinderwachstumshormons (bGH) gebildet wird, wobei die natürlich vorkommende Form des Rinderwachstumshormons identisch ist mit der Rinderwachstumshormon-Mutantensequenz, die in Figur 1 gezeigt ist, außer daß ein Glycin anstelle des Arginins, das in Position 119 der Mutantensequenz gezeigt ist, vorliegt, wobei die mutierte Alpha-Helix sich von der dritten Alpha-Helix des Vertebraten-Wachstumshormons mindestens in der Position unterscheidet, die der Position 119 der natürlich vorkommenden Form des Rinderwachstumshormons entspricht,
mit der Maßgabe, daß das Protein oder Peptid weder
(a) das Rindersomatotropin ist, das die Aminosäuresequenz von Diagramm 1 der WO 91/00870 (D1) hat, wobei Glycin an Position 119 durch Prolin ersetzt ist; noch
(b) eine Mutante, hPRL (111-129), des vollständigen nativen menschlichen Wachstumshormons (hGH) ist mit der Sequenz, die in Figur 1 von Cunningham et al., Science 243, 1330-36 (1989) (D7) gezeigt ist, wobei die Mutante sich davon nur durch 12 Substitutionen unterscheidet, wie sie für hPRL (111-129) in Tabelle 1 von D7 gezeigt sind.

2. Peptid oder Protein nach Anspruch 1, das die gleiche Größe hat wie oder größer ist als das Vertebraten-Wachstumshormon.

3. Isoliertes Peptid oder Protein mit mindestens 11 Aminosäuren, das ein Fragment eines Proteins ist, das Wachstumsinhibitoraktivität in einem Vertebraten aufweist und eine mutierte Alpha-Helix umfaßt, die im wesentlichen homolog, aber nicht identisch ist mit der dritten Alpha-Helix eines Vertebraten-Wachstumshormons,
wobei die dritte Alpha-Helix der Alpha-Helix entspricht, die von den Aminosäuren an den Positionen 109 bis 126 der natürlich vorkommenden Form von Rinderwachstumshormon (bGH) gebildet wird, wobei die natürlich vorkommende Form des Rinderwachstumshormons identisch ist mit der Rinderwachstumshormon-Mutantensequenz, die in Figur 1 gezeigt ist, außer daß ein Glycin anstelle des Arginins, das in Position 119 der Mutantensequenz gezeigt ist, vorliegt, wobei die mutierte Alpha-Helix sich von der dritten Alpha-Helix des Vertebraten-Wachstumshormons in mindestens der Position unterscheidet, die der Position 119 der natürlich vorkommenden Form des Rinderwachstumshormons entspricht,
und wobei das isolierte Peptid oder Protein eine Aminosäuresequenz aufweist, die relativ zu der des Vertebraten-Wachstumshormons verkürzt ist.

4. Peptid oder Protein nach einem der Ansprüche 1 bis 3, wobei das Vertebraten-Wachstumshormon ein Säugerwachstumshormon ist.

5. Peptid oder Protein nach einem der Ansprüche 1 bis 4, wobei das Vertebraten-Wachstumshormon Rinderwachstumshormon ist.

6. Peptid oder Protein nach einem der Ansprüche 1 bis 4, wobei das Vertebraten-Wachstumshormon menschliches Wachstumshormon ist.

7. Peptid oder Protein nach einem der Ansprüche 1 bis 3, wobei das Vertebraten-Wachstumshormon Hühnerwachstumshormon ist.

8. Peptid oder Protein nach einem der Ansprüche 1 bis 4, wobei das Vertebraten-Wachstumshormon Ratten-, Schweine- oder Schafwachstumshormon ist.

9. Peptid oder Protein nach einem der Ansprüche 1 bis 3, wobei das Vertebraten-Wachstumshormon Flunder-, Gelbschwanz-, Thunfisch- oder Lachswachstumshormon ist.

10. Peptid oder Protein nach einem der Ansprüche 1 bis 3, das im wesentlichen homolog mit der natürlich vorkommenden Form von Rinderwachstumshormon ist.

11. Peptid oder Protein nach einem der Ansprüche 1 bis 3, das im wesentlichen homolog mit einem Vertebraten-Wachstumshormon ist.

12. Peptid oder Protein nach einem der Ansprüche 1 bis 3, das im wesentlichen homolog mit einem Säugerwachstumshormon ist.

13. Peptid oder Protein nach einem der Ansprüche 1 bis 12, wobei die Aminosäure an der Position, die der Position 119 der natürlich vorkommenden Form des Rinderwachstumshormons entspricht, nicht Alanin ist.

14. Peptid oder Protein nach einem der Ansprüche 1 bis 13, wobei die Aminosäure an der Position, die der Position 119 der natürlich vorkommenden Form des Rinderwachstumshormons entspricht, Arginin ist.

15. Peptid oder Protein nach einem der Ansprüche 1 bis 13, wobei die Aminosäure an der Position, die der Position 119 der natürlich vorkommenden Form des Rinderwachstumshormons entspricht, Prolin ist.

16. Peptid oder Protein nach einem der Ansprüche 1 bis 13, wobei die Aminosäure an der Position, die der Position 119 der natürlich vorkommenden Form des Rinderwachstumshormons entspricht, Lysin ist.

17. Peptid oder Protein nach einem der Ansprüche 1 bis 13, wobei die Aminosäure an der Position, die der Position 119 der natürlich vorkommenden Form des Rinderwachstumshormons entspricht, Leucin ist.

18. Peptid oder Protein nach einem der Ansprüche 1 bis 13, wobei die Aminosäure an der Position, die der Position 119 der natürlich vorkommenden Form des Rinderwachstumshormons entspricht, Serin ist.

19. Peptid oder Protein nach einem der Ansprüche 1 bis 13, wobei die Aminosäure an der Position, die der Position 119 der natürlich vorkommenden Form des Rinderwachstumshormons entspricht, Glutamin ist.

20. Peptid oder Protein nach einem der Ansprüche 1 bis 13, wobei die Aminosäure an der Position, die der Position 119 der natürlich vorkommenden Form des Rinderwachstumshormons entspricht, Glutaminsäure ist.

21. Peptid oder Protein nach einem der Ansprüche 1 bis 13, wobei die Aminosäure an der Position, die der Position 119 der natürlich vorkommenden Form des Rinderwachstumshormons entspricht, Tryptophan ist.

22. Peptid oder Protein nach einem der Ansprüche 1 bis 13, wobei die Aminosäure an der Position, die der Position 119 der natürlich vorkommenden Form des Rinderwachstumshormons entspricht, Methionin ist.

23. Isoliertes Protein, ausgewählt aus der Gruppe
bGH (G119R),
bGH (G119R, E117L, A122D),
bGH (G119K),
bGH (G119L),
Fragmente dieser Proteine und
Fragmente von bGH (G119P),
die Wachstumsinhibitoraktivität in einem Vertebraten haben, wobei bGH die natürlich vorkommende Form von Rinderwachstumshormon (bGH) ist, das identisch ist mit der Rinderwachstumshormon-Mutantensequenz, die in Figur 1 gezeigt ist, bis auf das Vorhandensein eines Glycins anstelle des Arginins, das in Position 119 der Mutantensequenz gezeigt ist.

24. Peptid oder Protein nach Anspruch 3, dem mindestens eine Alpha-Helix fehlt, ausgewählt aus Helices, die den ersten, zweiten oder vierten Helices der natürlich vorkommenden Form des Rinderwachstumshormons entsprechen.

25. Peptid oder Protein nach einem der Ansprüche 1 bis 24, das sich weiterhin von dem Vertebraten-Wachstumshormon durch eine oder mehrere Substitutionen an Aminosäuren unterscheiden, die den Aminosäuren 115 oder 122 des natürlich vorkommenden Rinderwachstumshormons entsprechen, und/oder den Aminosäuren des natürlich vorkommenden Rinderwachstumshormons, die unter den Vertebraten-Wachstumshormonen nicht konserviert sind, und/oder durch Verkürzung der Aminosäuren, die den Aminosäuren 1 bis 95 und 134 bis 191 des natürlich vorkommenden Wachstumshormons entsprechen.

26. Peptid oder Protein nach einem der Ansprüche 1 bis 25, wobei das Peptid oder Protein einen ED50-Wert hat, der niedriger ist als etwa 10mal der ED50-Wert des Wildtyp-Wachstumshormons in einem Test auf die Fähigkeit des Peptids oder Proteins radioaktiv markiertes Wildtyp-Wachstumshormon von einem Mausleber-Membranrezeptor zu ersetzen.

27. Peptid oder Protein nach einem der Ansprüche 1 bis 26, wobei das Peptid oder Protein Wachstumsinhibitoraktivität in einem Säuger hat.

28. Rekombinantes DNA-Molekül, umfassend ein Gen, das eine Sequenz einschließt, die das Peptid oder Protein nach einem der Ansprüche 1 bis 27 codiert.

29. Molekül nach Anspruch 28, das weiterhin einen Promotor umfaßt, der funktionell mit dem Gen verknüpft ist.

30. Molekül nach Anspruch 29, wobei der Promotor nicht vom Promotor eines Wachstumshormongens abgeleitet ist.

31. Zelle, umfassend das rekombinante DNA-Molekül nach einem der Ansprüche 28 bis 30.

32. Zelle nach Anspruch 31, wobei die Zelle eine Zelle ist, die ein chromosomales Wachstumshormongen exprimiert.

33. Nicht-menschliches transgenes Tier, umfassend eine Vielzahl von Zellen, die ein Gen enthalten, das funktionell mit einem Promotor verknüpft ist, wobei das Gen eine Sequenz umfaßt, die ein Peptid oder Protein mit mindestens 11 Aminosäuren codiert, das Wachstumsinhibitoraktivität in einem Vertebraten aufweist und eine mutierte Alpha-Helix umfaßt, die im wesentlichen homolog, aber nicht identisch ist mit der dritten Alpha-Helix eines Vertebraten-Wachstumshormons, wobei die dritte Alpha-Helix der Alpha-Helix entspricht, die von den Aminosäuren an den Positionen 109 bis 126 der natürlich vorkommenden Form des Rinderwachstumshormons (bGH) gebildet wird,
wobei die natürlich vorkommende Form des Rinderwachstumshormons identisch ist mit der Rinderwachstumshormon-Mutantensequenz, die in Figur 1 gezeigt ist, außer daß ein Glycin anstelle des Arginins, das in Position 119 der Mutantensequenz gezeigt ist, vorliegt,
wobei die mutierte Alpha-Helix sich von der dritten Alpha-Helix des Vertebraten-Wachstumshormons mindestens in der Position unterscheidet, die der Position 119 der natürlich vorkommenden Form des Rinderwachstumshormons entspricht.

34. Nicht-menschliches transgenes Tier nach Anspruch 33, wobei eine Vielzahl von Zellen ein Gen umfassen, das eine Sequenz einschließt, die ein Protein oder Peptid nach einem der Ansprüche 1 bis 27 codiert.

35. Nicht-menschliches Tier nach Anspruch 33 oder 34, das eine reduzierte Wachstumsgeschwindigkeit verglichen mit Kontrolltieren zeigt.

36. Verfahren zur Herstellung eines Wachstumsinhibitorpeptids oder -Proteins, umfassend die Bereitstellung von Zellen, die ein Gen tragen, das eine Sequenz einschließt, die ein Peptid oder Protein nach einem der Ansprüche 1 bis 27 codiert, unter Bedingungen, die förderlich sind für die Expression des Gens, wobei das Protein in einer verwendbaren oder gewinnbaren Form produziert wird.

37. Arzneimittel, umfassend das Peptid oder Protein nach einem der Ansprüche 1 bis 27 und einen pharmazeutisch verträglichen Träger.

38. Peptid oder Protein mit mindestens 11 Aminosäuren, das Wachstumsinhibitoraktivität in einem Vertebraten aufweist und eine mutierte Alpha-Helix umfaßt, die im wesentlichen homolog, aber nicht identisch ist mit der dritten Alpha-Helix eines Vertebraten-Wachstumshormons, wobei die dritte Alpha-Helix der Alpha-Helix entspricht, die von den Aminosäuren an den Positionen 109 bis 126 der natürlich vorkommenden Form des Rinderwachstumshormons (bGH) gebildet wird, wobei die natürlich vorkommende Form des Rinderwachstumshormons identisch ist mit der Rinderwachstumshormon-Mutantensequenz, die in Figur 1 gezeigt ist, außer daß ein Glycin anstelle des Arginins, das an Position 119 der Mutantensequenz gezeigt ist, vorliegt,
wobei die mutierte Alpha-Helix sich von der dritten Alpha-Helix des Vertebraten-Wachstumshormons mindestens in der Position unterscheidet, die der Position 119 der natürlich vorkommenden Form des Rinderwachstumshormons entspricht, zur Verwendung als Medikament,
mit der Maßgabe, daß das Protein oder Peptid weder
(a) das Rindersomatotropin ist, das die Aminosäuresequenz von Diagramm 1 der WO 91/00870 (D1) hat, wobei Glycin an Position 119 durch Prolin ersetzt ist; noch
(b) eine Mutante, hPRL (111-129), des vollständigen nativen menschlichen Wachstumshormons (hGH) ist mit der Sequenz, die in Figur 1 von Cunningham et al., Science 243, 1330-36 (1989) (D7) gezeigt ist, wobei die Mutante sich davon nur durch 12 Substitutionen unterscheidet, wie sie für hPRL (111-129) in Tabelle 1 von D7 gezeigt sind.

39. Peptid oder Protein nach einem der Ansprüche 1 bis 27, oder Peptid oder Protein nach Anspruch 38 mit den Eigenschaften, die zum ersten Mal in einem der Ansprüche 2 bis 27 beschrieben sind, zur Verwendung als ein Medikament.

40. Rekombinantes DNA-Molekül, umfassend ein Gen, das eine Sequenz einschließt, die ein Peptid oder Protein mit mindestens 11 Aminosäuren codiert, das Wachstumsinhibitoraktivität in einem Vertebraten aufweist und eine mutierte Alpha-Helix umfaßt, die im wesentlichen homolog, aber nicht identisch ist mit der dritten Alpha-Helix eines Vertebraten-Wachstumshormons, wobei die dritte Alpha-Helix der Alpha-Helix entspricht, die von den Aminosäuren an den Positionen 109 bis 126 der natürlich vorkommenden Form des Rinderwachstumshormons (bGH) gebildet wird, wobei die natürlich vorkommende Form des Rinderwachstumshormons identisch ist mit der Rinderwachstumshormon-Mutantensequenz, die in Figur 1 gezeigt ist, außer daß ein Glycin anstelle des Arginins, das an Position 119 der Mutantensequenz gezeigt ist, vorliegt, wobei die mutierte Alpha-Helix sich von der dritten Alpha-Helix des Vertebraten-Wachstumshormons mindestens in der Position unterscheidet, die der Position 119 der natürlich vorkommenden Form des Rinderwachstumshormons entspricht, zur Verwendung als ein Medikament.

41. Rekombinantes DNA-Molekül nach einem der Ansprüche 28 bis 30 oder ein rekombinantes DNA-Molekül nach Anspruch 40, das ein Peptid oder Protein mit den Eigenschaften codiert, die zum ersten Mal in einem der Ansprüche 2 bis 27 beschrieben sind, zur Verwendung als ein Medikament.

42. Rekombinantes Molekül nach Anspruch 40 oder 41 zur Hemmung oder Verminderung der Wachstumshormonaktivität in einem menschlichen oder tierischen Individuum.

43. DNA-Molekül nach einem der Ansprüche 40 bis 42, wobei ein resultierendes genetisch modifiziertes menschliches oder tierisches Individuum fähig ist, das Protein oder Peptid nach einem der Ansprüche 1 bis 27 oder 38 auf einem Wachstumsinhibitor-Niveau zu exprimieren.

44. Verwendung eines Peptids oder Proteins mit mindestens 11 Aminosäuren, das Wachstumsinhibitoraktivität in einem Vertebraten aufweist und eine mutierte Alpha-Helix umfaßt, die im wesentlichen homolog, aber nicht identisch ist mit der dritten Alpha-Helix eines Vertebraten-Wachstumshormons, wobei die dritte Alpha-Helix der Alpha-Helix entspricht, die von den Aminosäuren an den Positionen 109 bis 126 der natürlich vorkommenden Form des Rinderwachstumshormons (bGH) gebildet wird, wobei die natürlich vorkommende Form des Rinderwachstumshormons identisch ist mit der Rinderwachstumshormon-Mutantensequenz, die in Figur 1 gezeigt ist, außer daß ein Glycin anstelle des Arginins, das an Position 119 der Mutantensequenz gezeigt ist, vorliegt, wobei die mutierte Alpha-Helix sich von der dritten Alpha-Helix des Vertebraten-Wachstumshormons mindestens in der Position unterscheidet, die der Position 119 der natürlich vorkommenden Form des Rinderwachstumshormons entspricht, für die Herstellung eines Medikaments zur Behandlung eines Zustandes, der durch Hemmung oder Verminderung der Wachstumshormonaktivität verbessert werden kann.

45. Verwendung eines Peptids oder Proteins nach einem der Ansprüche 1 bis 27 oder Verwendung eines Peptids oder Proteins nach Anspruch 44 mit den Eigenschaften, die in einem der Ansprüche 2 bis 27 zum ersten Mal beschrieben sind, für die Herstellung eines Medikaments zur Behandlung eines Zustandes, der durch Hemmung oder Verminderung der Wachstumshormonaktivität verbessert werden kann.

46. Verwendung eines rekombinanten DNA-Moleküls, umfassend ein Gen, das eine Sequenz einschließt, die ein Peptid oder Protein mit mindestens 11 Aminosäuren codiert, das Wachstumsinhibitoraktivität in einem Vertebraten aufweist und eine mutierte Alpha-Helix umfaßt, die im wesentlichen homolog, aber nicht identisch ist mit der dritten Alpha-Helix eines Vertebraten-Wachstumshormons, wobei die dritte Alpha-Helix der Alpha-Helix entspricht, die von den Aminosäuren an den Positionen 109 bis 126 der natürlich vorkommenden Form von Rinderwachstumshormon (bGH) gebildet wird, wobei die natürlich vorkommende Form des Rinderwachstumshormons identisch ist mit der Rinderwachstumshormon-Mutantensequenz, die in Figur 1 gezeigt ist, außer daß ein Glycin anstelle des Arginins, das in Position 119 der Mutantensequenz gezeigt ist, vorliegt, wobei die mutierte Alpha-Helix sich von der dritten Alpha-Helix des Vertebraten-Wachstumshormons in mindestens der Position unterscheidet, die der Position 119 der natürlich vorkommenden Form des Rinderwachstumshormons entspricht, für die Herstellung eines Mittels zur Behandlung eines Zustandes, der durch Hemmung oder Verminderung der Wachstumshormonaktivität verbessert werden kann.

47. Verwendung eines rekombinanten DNA-Moleküls nach einem der Ansprüche 28 bis 30 oder Verwendung eines rekombinanten DNA-Moleküls nach Anspruch 46, das ein Peptid oder Protein mit den Eigenschaften codiert, die in einem der Ansprüche 2 bis 27 zum ersten Mal beschrieben sind, für die Herstellung eines Mittels zur Behandlung eines Zustandes, der durch Hemmung oder Verminderung der Wachstumshormonaktivität verbessert werden kann.

48. Peptid oder Protein nach Anspruch 3, das mindestens 50 Aminosäuren aufweist.

49. Peptid oder Protein nach einem der Ansprüche 1 bis 27, das Wachstumsinhibitoraktivität in einem Menschen zeigt.

50. Peptid oder Protein nach Anspruch 1, wobei die Aminosäure an der Position, die der Position 119 der natürlich vorkommenden Form des Rinderwachstumshormons entspricht, eine hydrophobe Aminosäure ist, gemäß der Einteilung von Hopp und Woods, PNAS (USA) 78, 3824-28 (1981).

51. Peptid oder Protein nach Anspruch 1, wobei der amphiphile Charakter der mutierten Alpha-Helix, bestimmt gemäß dem Verfahren von Margalit et al., J. Immunol. 138, 2213-29 (1987), reduziert oder unverändert ist, bezogen auf den amphiphilen Charakter der dritten Alpha-Helix an den Positionen 109 bis 126 des Rinderwachstumshormons.

52. Peptid oder Protein nach Anspruch 50 oder 51 zur Verwendung als ein Medikament.

53. Verwendung des Peptids oder Proteins nach Anspruch 50 oder 51 für die Herstellung eines Medikaments zur Behandlung eines Zustandes, der durch Hemmung oder Verminderung der Wachstumshormonaktivität verbessert werden kann.

## Patentansprüche (Patentansprüche für folgende(n) Vertragsstaat(en): GR)

1. Rekombinantes DNA-Molekül, umfassend ein Gen, das eine Sequenz einschließt, die ein Peptid oder Protein mit mindestens 11 Aminosäuren codiert, das Wachstumsinhibitoraktivität in einem Vertebraten aufweist und eine mutierte Alpha-Helix umfaßt, die im wesentlichen homolog, aber nicht identisch ist mit der dritten Alpha-Helix eines Vertebraten-Wachstumshormons, wobei die dritte Alpha-Helix der Alpha-Helix entspricht, die von den Aminosäuren an den Positionen 109 bis 126 der natürlich vorkommenden Form des Rinderwachstumshormons (bGH) gebildet wird, wobei die natürlich vorkommende Form des Rinderwachstumshormons identisch ist mit der Rinderwachstumshormon-Mutantensequenz, die in Figur 1 gezeigt ist, außer daß ein Glycin anstelle des Arginins, das in Position 119 der Mutantensequenz gezeigt ist, vorliegt, wobei die mutierte Alpha-Helix sich von der dritten Alpha-Helix des Vertebraten-Wachstumshormons mindestens in der Position unterscheidet, die der Position 119 der natürlich vorkommenden Form des Rinderwachstumshormons entspricht,
mit der Maßgabe, daß das Protein oder Peptid weder
(a) das Rindersomatotropin ist, das die Aminosäuresequenz von Diagramm 1 der WO 91/00870 (D1) hat, wobei Glycin an Position 119 durch Prolin ersetzt ist; noch
(b) eine Mutante, hPRL (111-129), des vollständigen nativen menschlichen Wachstumshormons (bGH) ist mit der Sequenz, die in Figur 1 von Cunningham et al., Science 243, 1330-36 (1989) (D7) gezeigt ist, wobei die Mutante sich davon nur durch 12 Substitutionen unterscheidet, wie sie für hPRL (111-129) in Tabelle 1 von D7 gezeigt sind.

2. Molekül nach Anspruch 1, das die gleiche Größe hat wie oder größer ist als das Vertebraten-Wachstumshormon.

3. Rekombinantes DNA-Molekül, umfassend ein Gen, das eine Sequenz einschließt, die ein Peptid oder Protein mit mindestens 11 Aminosäuren codiert, das ein Fragment eines Proteins ist, das Wachstumsinhibitoraktivität in einem Vertebraten aufweist und eine mutierte Alpha-Helix umfaßt, die im wesentlichen homolog, aber nicht identisch ist mit der dritten Alpha-Helix eines Vertebraten-Wachstumshormons, wobei die dritte Alpha-Helix der Alpha-Helix entspricht, die von den Aminosäuren an den Positionen 109 bis 126 der natürlich vorkommenden Form von Rinderwachstumshormon (bGH) gebildet wird, wobei die natürlich vorkommende Form des Rinderwachstumshormons identisch ist mit der Rinderwachstumshormon-Mutantensequenz, die in Figur 1 gezeigt ist, außer daß ein Glycin anstelle des Arginins, das in Position 119 der Mutantensequenz gezeigt ist, vorliegt.

4. Molekül nach einem der Ansprüche 1 bis 3, wobei das Vertebraten-Wachstumshormon ein Säugerwachstumshormon ist.

5. Molekül nach einem der Ansprüche 1 bis 4, wobei das Vertebraten-Wachstumshormon Rinderwachstumshormon ist.

6. Molekül nach einem der Ansprüche 1 bis 4, wobei das Vertebraten-Wachstumshormon menschliches Wachstumshormon ist.

7. Molekül nach einem der Ansprüche 1 bis 3, wobei das Vertebraten-Wachstumshormon Hühnerwachstumshormon ist.

8. Molekül nach einem der Ansprüche 1 bis 4, wobei das Vertebraten-Wachstumshormon Ratten-, Schweine- oder Schafwachstumshormon ist.

9. Molekül nach einem der Ansprüche 1 bis 3, wobei das Vertebraten-Wachstumshormon Flunder-, Gelbschwanz-, Thunfisch- oder Lachswachstumshormon ist.

10. Molekül nach einem der Ansprüche 1 bis 3, wobei das Peptid oder Protein im wesentlichen homolog mit der natürlich vorkommenden Form von Rinderwachstumshormon ist.

11. Molekül nach einem der Ansprüche 1 bis 3, wobei das Peptid oder Protein im wesentlichen homolog mit einem Vertebraten-Wachstumshormon ist.

12. Molekül nach einem der Ansprüche 1 bis 3, wobei das Peptid oder Protein im wesentlichen homolog mit einem Säugerwachstumshormon ist.

13. Molekül nach einem der Ansprüche 1 bis 12, wobei die Aminosäure, die an der Position codiert ist, die der Position 119 der natürlich vorkommenden Form des Rinderwachstumshormons entspricht, nicht Alanin ist.

14. Molekül nach einem der Ansprüche 1 bis 13, wobei die Aminosäure, die an der Position codiert ist, die der Position 119 der natürlich vorkommenden Form des Rinderwachstumshormons entspricht, Arginin ist.

15. Molekül nach einem der Ansprüche 1 bis 13, wobei die Aminosäure, die an der Position codiert ist, die der Position 119 der natürlich vorkommenden Form des Rinderwachstumshormons entspricht, Prolin ist.

16. Molekül nach einem der Ansprüche 1 bis 13, wobei die Aminosäure, die an der Position codiert ist, die der Position 119 der natürlich vorkommenden Form des Rinderwachstumshormons entspricht, Lysin ist.

17. Molekül nach einem der Ansprüche 1 bis 13, wobei die Aminosäure, die an der Position codiert ist, die der Position 119 der natürlich vorkommenden Form des Rinderwachstumshormons entspricht, Leucin ist.

18. Molekül nach einem der Ansprüche 1 bis 13, wobei die Aminosäure, die an der Position codiert ist, die der Position 119 der natürlich vorkommenden Form des Rinderwachstumshormons entspricht, Serin ist.

19. Molekül nach einem der Ansprüche 1 bis 13, wobei die Aminosäure, die an der Position codiert ist, die der Position 119 der natürlich vorkommenden Form des Rinderwachstumshormons entspricht, Glutamin ist.

20. Molekül nach einem der Ansprüche 1 bis 13, wobei die Aminosäure, die an der Position codiert ist, die der Position 119 der natürlich vorkommenden Form des Rinderwachstumshormons entspricht, Glutaminsäure ist.

21. Molekül nach einem der Ansprüche 1 bis 13, wobei die Aminosäure, die an der Position codiert ist, die der Position 119 der natürlich vorkommenden Form des Rinderwachstumshormons entspricht, Tryptophan ist.

22. Molekül nach einem der Ansprüche 1 bis 13, wobei die Aminosäure, die an der Position codiert ist, die der Position 119 der natürlich vorkommenden Form des Rinderwachstumshormons entspricht, Methionin ist.

23. Molekül nach Anspruch 1, wobei das Peptid oder Protein ausgewählt ist aus der Gruppe
bGH (G119R),
bGH (G119R, E117L, A122D),
bGH (G119K),
bGH (G119L),
Fragmente dieser Proteine und
Fragmente von bGH (G119P),
die Wachstumsinhibitoraktivität in einem Vertebraten haben, wobei bGH die natürlich vorkommende Form von Rinderwachstumshormon (bGH) ist, das identisch ist mit der Rinderwachstumshormon-Mutantensequenz, die in Figur 1 gezeigt ist, bis auf das Vorhandensein eines Glycins anstelle des Arginins, das in Position 119 der Mutantensequenz gezeigt ist.

24. Molekül nach Anspruch 3, wobei dem Peptid oder Protein mindestens eine Alpha-Helix fehlt, ausgewählt aus Helices, die den ersten, zweiten oder vierten Helices der natürlich vorkommenden Form des Rinderwachstumshormons entsprechen.

25. Molekül nach einem der Ansprüche 1 bis 24, wobei das Peptid oder Protein sich weiterhin von dem Vertebraten-Wachstumshormon durch eine oder mehrere Substitutionen an Aminosäuren unterscheiden, die den Aminosäuren 115 oder 122 des natürlich vorkommenden Rinderwachstumshormons entsprechen, und/oder den Aminosäuren des natürlich vorkommenden Rinderwachstumshormons, die unter den Vertebraten-Wachstumshormonen nicht konserviert sind, und/oder durch Verkürzung der Aminosäuren, die den Aminosäuren 1 bis 95 und 134 bis 191 des natürlich vorkommenden Wachstumshormons entsprechen.

26. Molekül nach einem der Ansprüche 1 bis 25, wobei das Peptid oder Protein einen ED50-Wert hat, der niedriger ist als etwa 10mal der ED50-Wert des Wildtyp-Wachstumshormons in einem Test auf die Fähigkeit des Peptids oder Proteins radioaktiv markiertes Wildtyp-Wachstumshormon von einem Mausleber-Membranrezeptor zu ersetzen.

27. Molekül nach einem der Ansprüche 1 bis 26, wobei das Peptid oder Protein Wachstumsinhibitoraktivität in einem Säuger hat.

28. Molekül nach einem der Ansprüche 1 bis 27, das weiterhin einen Promotor umfaßt, der funktionell mit dem Gen verknüpft ist.

29. Molekül nach Anspruch 28, wobei der Promotor nicht vom Promotor eines Wachstumshormongens abgeleitet ist.

30. Zelle, umfassend das rekombinante DNA-Molekül nach einem der Ansprüche 1 bis 29.

31. Zelle nach Anspruch 30, wobei die Zelle eine Zelle ist, die ein chromosomales Wachstumshormongen exprimiert.

32. Isoliertes Peptid oder Protein mit mindestens 11 Aminosäuren, das Wachstumsinhibitoraktivität in einem Vertebraten aufweist und eine mutierte Alpha-Helix umfaßt, die im wesentlichen homolog, aber nicht identisch ist mit der dritten Alpha-Helix eines Vertebraten-Wachstumshormons, wobei die dritte Alpha-Helix der Alpha-Helix entspricht, die von den Aminosäuren an den Positionen 109 bis 126 der natürlich vorkommenden Form des Rinderwachstumshormons (bGH) gebildet wird, wobei die natürlich vorkommende Form des Rinderwachstumshormons identisch ist mit der Rinderwachstumshormon-Mutantensequenz, die in Figur 1 gezeigt ist, außer daß ein Glycin anstelle des Arginins, das an Position 119 der Mutantensequenz gezeigt ist, vorliegt,
wobei die mutierte Alpha-Helix sich von der dritten Alpha-Helix des Vertebraten-Wachstumshormons mindestens in der Position unterscheidet, die der Position 119 der natürlich vorkommenden Form des Rinderwachstumshormons entspricht,
mit der Maßgabe, daß das Protein oder Peptid weder
(a) das Rindersomatotropin ist, das die Aminosäuresequenz von Diagramm 1 der WO 91/00870 (D1) hat, wobei Glycin an Position 119 durch Prolin ersetzt ist; noch
(b) eine Mutante, hPRL (111-129), des vollständigen nativen menschlichen Wachstumshormons (hGH) ist mit der Sequenz, die in Figur 1 von Cunningham et al., Science 243, 1330-36 (1989) (D7) gezeigt ist, wobei die Mutante sich davon nur durch 12 Substitutionen unterscheidet, wie sie für hPRL (111-129) in Tabelle 1 von D7 gezeigt sind.

33. Peptid oder Protein nach Anspruch 32, wobei das Peptid oder Protein Eigenschaften hat, die zum ersten Mal in einem der Ansprüche 2 bis 27 beschrieben sind.

34. Nicht-menschliches transgenes Tier, umfassend eine Vielzahl von Zellen, die ein Gen enthalten, das funktionell mit einem Promotor verknüpft ist wobei das Gen eine Sequenz umfaßt, die ein Peptid oder Protein mit mindestens 11 Aminosäuren codiert, das Wachstumsinhibitoraktivität in einem Vertebraten aufweist und eine mutierte Alpha-Helix umfaßt, die im wesentlichen homolog, aber nicht identisch ist mit der dritten Alpha-Helix eines Vertebraten-Wachstumshormons, wobei die dritte Alpha-Helix der Alpha-Helix entspricht, die von den Aminosäuren an den Positionen 109 bis 126 der natürlich vorkommenden Form des Rinderwachstumshormons (bGH) gebildet wird, wobei die natürlich vorkommende Form des Rinderwachstumshormons identisch ist mit der Rinderwachstumshormon-Mutantensequenz, die in Figur 1 gezeigt ist, außer daß ein Glycin anstelle des Arginins, das in Position 119 der Mutantensequenz gezeigt ist, vorliegt,
wobei die mutierte Alpha-Helix sich von der dritten Alpha-Helix des Vertebraten-Wachstumshormons mindestens in der Position unterscheidet, die der Position 119 der natürlich vorkommenden Form der Rinderwachstumshormons entspricht.

35. Nicht-menschliches transgenes Tier nach Anspruch 34, wobei eine Vielzahl von Zellen ein rekombinantes DNA-Molekül nach einem der Ansprüche 1 bis 29 umfaßt.

36. Nicht-menschliches Tier nach Anspruch 34 oder 35, das eine reduzierte Wachstumsgeschwindigkeit verglichen mit Kontrolltieren zeigt.

37. Verfahren zur Herstellung eines Wachstumsinhibitorpeptids oder -Proteins, umfassend die Bereitstellung von Zellen, die ein Gen Wagen, das eine Sequenz einschließt, die ein Peptid oder Protein nach Anspruch 32 oder 33 codiert, unter Bedingungen, die förderlich sind für die Expression des Gens, wobei das Protein in einer verwendbaren oder gewinnbaren Form produziert wird.

38. Arzneimittel, umfassend das Peptid oder Protein nach Anspruch 32 oder 33 und einen pharmazeutisch verträglichen Träger.

39. Verfahren zur Herstellung eines Arzneimittels, umfassend das Mischen eines Peptids oder Proteins gemäß der Definition in Anspruch 32 oder 33 und eines pharmazeutisch verträglichen Trägers.

40. Rekombinantes DNA-Molekül, umfassend ein Gen, das eine Sequenz einschließt, die ein Peptid oder Protein mit mindestens 11 Aminosäuren codiert, das Wachstumsinhibitoraktivität in einem Vertebraten aufweist und eine mutierte Alpha-Helix umfaßt, die im wesentlichen homolog, aber nicht identisch ist mit der dritten Alpha-Helix eines Vertebraten-Wachstumshormons, wobei die dritte Alpha-Helix der Alpha-Helix entspricht, die von den Aminosäuren an den Positionen 109 bis 126 der natürlich vorkommenden Form des Rinderwachstumshormons (bGH) gebildet wird, wobei die natürlich vorkommende Form des Rinderwachstumshormons identisch ist mit der Rinderwachstumshormon-Mutantensequenz, die in Figur 1 gezeigt ist, außer daß ein Glycin anstelle des Arginins, das an Position 119 der Mutantensequenz gezeigt ist, vorliegt, wobei die mutierte Alpha-Helix sich von der dritten Alpha-Helix des Vertebraten-Wachstumshormons mindestens in der Position unterscheidet, die der Position 119 der natürlich vorkommenden Form des Rinderwachstumshormons entspricht, zur Verwendung als ein Medikament.

41. Rekombinantes DNA-Molekül nach einem der Ansprüche 1 bis 29 zur Verwendung als ein Medikament.

42. Rekombinantes DNA-Molekül nach Anspruch 40 oder 41 zur Hemmung oder Verminderung der Wachstumshormonaktivität in einem menschlichen oder tierischen Individuum.

43. Rekombinantes DNA-Molekül nach einem der Ansprüche 40 bis 42, wobei ein resultierendes genetisch modifiziertes menschliches oder tierisches Individuum fähig ist, das Protein oder Peptid nach einem der Ansprüche 1 bis 27 auf einem Wachstumsinhibitor-Niveau zu exprimieren.

44. Verwendung eines Peptids oder Proteins mit mindestens 11 Aminosäuren, das Wachstumsinhibitoraktivität in einem Vertebraten aufweist und eine mutierte Alpha-Helix umfaßt, die im wesentlichen homolog, aber nicht identisch ist mit der dritten Alpha-Helix eines Vertebraten-Wachstumshormons, wobei die dritte Alpha-Helix der Alpha-Helix entspricht, die von den Aminosäuren an den Positionen 109 bis 126 der natürlich vorkommenden Form des Rinderwachstumshormons (bGH) gebildet wird, wobei die natürlich vorkommende Form des Rinderwachstumshormons identisch ist mit der Rinderwachstumshormon-Mutantensequenz, die in Figur 1 gezeigt ist, außer daß ein Glycin anstelle des Arginins, das an Position 119 der Mutantensequenz gezeigt ist, vorliegt, wobei die mutierte Alpha-Helix sich von der dritten Alpha-Helix des Vertebraten-Wachstumshormons mindestens in der Position unterscheidet, die der Position 119 der natürlich vorkommenden Form des Rinderwachstumshormons entspricht, für die Herstellung eines Medikaments zur Behandlung eines Zustandes, der durch Hemmung oder Verminderung der Wachstumshormonaktivität verbessert werden kann.

45. Verwendung eines Peptids oder Proteins nach einem der Ansprüche 1 bis 27 für die Herstellung eines Medikaments zur Behandlung eines Zustandes, der durch Hemmung oder Verminderung der Wachstumshormonaktivität verbessert werden kann.

46. Verwendung eines rekombinanten DNA-Moleküls, umfassend ein Gen, das eine Sequenz einschließt, die ein Peptid oder Protein mit mindestens 11 Aminosäuren codiert, das Wachstumsinhibitoraktivität in einem Vertebraten aufweist und eine mutierte Alpha-Helix umfaßt, die im wesentlichen homolog, aber nicht identisch ist mit der dritten Alpha-Helix eines Vertebraten-Wachstumshormons, wobei die dritte Alpha-Helix der Alpha-Helix entspricht die von den Aminosäuren an den Positionen 109 bis 126 der natürlich vorkommenden Form von Rinderwachstumshormon (bGH) gebildet wird, wobei die natürlich vorkommende Form des Rinderwachstumshormons identisch ist mit der Rinderwachstumshormon-Mutantensequenz, die in Figur 1 gezeigt ist, außer daß ein Glycin anstelle des Arginins, das in Position 119 der Mutantensequenz gezeigt ist, vorliegt, wobei die mutierte Alpha-Helix sich von der dritten Alpha-Helix des Vertebraten-Wachstumshormons in mindestens der Position unterscheidet, die der Position 119 der natürlich vorkommenden Form des Rinderwachstumshormons entspricht, für die Herstellung eines Mittels zur Behandlung eines Zustandes, der durch Hemmung oder Verminderung der Wachstumshormonaktivität verbessert werden kann.

47. Verwendung eines rekombinanten DNA-Moleküls nach einem der Ansprüche 1 bis 29 für die Herstellung eines Mittels zur Behandlung eines Zustandes, der durch Hemmung oder Verminderung der Wachstumshormonaktivität verbessert werden kann.

48. Peptid oder Protein nach Anspruch 3, das mindestens 50 Aminosäuren aufweist.

49. Peptid oder Protein nach einem der Ansprüche 1 bis 27, das Wachstumsinhibitoraktivität in einem Menschen zeigt.

50. Peptid oder Protein nach Anspruch 1, wobei die Aminosäure an der Position, die der Position 119 der natürlich vorkommenden Form des Rinderwachstumshormons entspricht, eine hydrophobe Aminosäure ist, gemäß der Einteilung von Hopp und Woods, PNAS (USA) 78, 3824-28(1981).

51. Peptid oder Protein nach Anspruch 1, wobei der amphiphile Charakter der mutierten Alpha-Helix, bestimmt gemäß dem Verfahren von Margalit et al., J. Immunol. 138, 2213-29 (1987), reduziert oder unverändert ist, bezogen auf den amphiphilen Charakter der dritten Alpha-Helix an den Positionen 109 bis 126 des Rinderwachstumshormons.

52. Peptid oder Protein nach Anspruch 50 oder 51 zur Verwendung als ein Medikament.

53. Verwendung des Peptids oder Proteins nach Anspruch 50 oder 51 für die Herstellung eines Medikaments zur Behandlung eines Zustandes, der durch Hemmung oder Verminderung der Wachstumshormonaktivität verbessert werden kann.

54. Verfahren zur Herstellung eines Arzneimittels, umfassend das Mischen eines Peptids oder Proteins gemäß der Definition in Anspruch 50 oder 51 und eines pharmazeutisch verträglichen Trägers.

## Patentansprüche (Patentansprüche für folgende(n) Vertragsstaat(en): ES)

1. Verfahren zur Herstellung eines Wachstumsinhibitorpeptids oder -Proteins, umfassend die Bereitstellung von Zellen, die ein Gen tragen, das eine Sequenz einschließt, die ein Peptid oder Protein mit mindestens 11 Aminosäuren codiert, das Wachstumsinhibitoraktivität in einem Vertebraten aufweist und eine mutierte Alpha-Helix umfaßt, die im wesentlichen homolog, aber nicht identisch ist mit der dritten Alpha-Helix eines Vertebraten-Wachstumshormons, wobei die dritte Alpha-Helix der Alpha-Helix entspricht, die von den Aminosäuren an den Positionen 109 bis 126 der natürlich vorkommenden Form des Rinderwachstumshormons (bGH) gebildet wird, wobei die natürlich vorkommende Form des Rinderwachstumshormons identisch ist mit der Rinderwachstumshormon-Mutantensequenz, die in Figur 1 gezeigt ist, außer daß ein Glycin anstelle des Arginins, das in Position 119 der Mutantensequenz gezeigt ist, vorliegt, wobei die mutierte Alpha-Helix sich von der dritten Alpha-Helix des Vertebraten-Wachstumshormons mindestens in der Position unterscheidet, die der Position 119 der natürlich vorkommenden Form des Rinderwachstumshormons entspricht,
mit der Maßgabe, daß das Protein oder Peptid weder
(a) das Rindersomatotropin ist, das die Aminosäuresequenz von Diagramm 1 der WO 91/00870 (D1) hat, wobei Glycin an Position 119 durch Prolin ersetzt ist; noch
(b) eine Mutante, hPRL (111-129), des vollständigen nativen menschlichen Wachstumshormons (hGH) ist mit der Sequenz, die in Figur 1 von Cunningham et al., Science 243, 1330-36 (1989) (D7) gezeigt ist, wobei die Mutante sich davon nur durch 12 Substitutionen unterscheidet, wie sie für hPRL (111-129) in Tabelle 1 von D7 gezeigt sind,
unter Bedingungen, die förderlich sind für die Expression des Gens, wobei das Protein in einer verwendbaren oder gewinnbaren Form produziert wird.

2. Verfahren nach Anspruch 1, ferner umfassend die Herstellung der Zellen durch Transformation von Wirtszellen, so daß sie das Gen aufweisen.

3. Verfahren nach Anspruch 1 oder 2, wobei das Protein oder Peptid die gleiche Größe hat wie oder größer ist als das Vertebraten-Wachstumshormon.

4. Verfahren zur Herstellung eines Wachstumsinhibitorpeptids oder -Proteins, umfassend die Bereitstellung von Zellen, die ein Gen Wagen, das eine Sequenz einschließt, die ein Peptid oder Protein mit mindestens 11 Aminosäuren codiert, das ein Fragment eines Proteins ist, das Wachstumsinhibitoraktivität in einem Vertebraten aufweist und eine mutierte Alpha-Helix umfaßt, die im wesentlichen homolog, aber nicht identisch ist mit der dritten Alpha-Helix eines Vertebraten-Wachstumshormons, wobei die dritte Alpha-Helix der Alpha-Helix entspricht, die von den Aminosäuren an den Positionen 109 bis 126 der natürlich vorkommenden Form von Rinderwachstumshormon (bGH) gebildet wird, wobei die natürlich vorkommende Form des Rinderwachstumshormons identisch ist mit der Rinderwachstumshormon-Mutantensequenz, die in Figur 1 gezeigt ist, außer daß ein Glycin anstelle des Arginins, das in Position 119 der Mutantensequenz gezeigt ist, vorliegt, unter Bedingungen, die förderlich sind für die Expression des Gens, wobei das Protein in einer verwendbaren oder gewinnbaren Form produziert wird.

5. Verfahren nach einem der Ansprüche 1 bis 4, wobei das Vertebraten-Wachstumshormon ein Säugerwachstumshormon ist.

6. Verfahren nach einem der Ansprüche 1 bis 5, wobei das Vertebraten-Wachstumshormon Rinderwachstumshormon ist.

7. Verfahren nach einem der Ansprüche 1 bis 5, wobei das Vertebraten-Wachstumshormon menschliches Wachstumshormon ist.

8. Verfahren nach einem der Ansprüche 1 bis 4, wobei das Vertebraten-Wachstumshormon Hühnerwachstumshormon ist.

9. Verfahren nach einem der Ansprüche 1 bis 5, wobei das Vertebraten-Wachstumshormon Ratten-, Schweine- oder Schafwachstumshormon ist.

10. Verfahren nach einem der Ansprüche 1 bis 4, wobei das Vertebraten-Wachstumshormon Flunder-, Gelbschwanz-, Thunfisch- oder Lachswachstumshormon ist.

11. Verfahren nach einem der Ansprüche 1 bis 4, wobei das Peptid oder Protein im wesentlichen homolog mit der natürlich vorkommenden Form von Rinderwachstumshormon ist.

12. Verfahren nach einem der Ansprüche 1 bis 4, wobei das Peptid oder Protein im wesentlichen homolog mit einem Vertebraten-Wachstumshormon ist.

13. Verfahren nach einem der Ansprüche 1 bis 4, wobei das Peptid oder Protein im wesentlichen homolog mit einem Säugerwachstumshormon ist.

14. Verfahren nach einem der Ansprüche 1 bis 13, wobei die Aminosäure, die an der Position codiert ist, die der Position 119 der natürlich vorkommenden Form des Rinderwachstumshormons entspricht, nicht Alanin ist.

15. Verfahren nach einem der Ansprüche 1 bis 14, wobei die Aminosäure, die an der Position codiert ist, die der Position 119 der natürlich vorkommenden Form des Rinderwachstumshormons entspricht, Arginin ist.

16. Verfahren nach einem der Ansprüche 1 bis 14, wobei die Aminosäure, die an der Position codiert ist, die der Position 119 der natürlich vorkommenden Form des Rinderwachstumshormons entspricht, Prolin ist.

17. Verfahren nach einem der Ansprüche 1 bis 14, wobei die Aminosäure, die an der Position codiert ist, die der Position 119 der natürlich vorkommenden Form des Rinderwachstumshormons entspricht, Lysin ist.

18. Verfahren nach einem der Ansprüche 1 bis 14, wobei die Aminosäure, die an der Position codiert ist, die der Position 119 der natürlich vorkommenden Form des Rinderwachstumshormons entspricht, Leucin ist.

19. Verfahren nach einem der Ansprüche 1 bis 14, wobei die Aminosäure, die an der Position codiert ist, die der Position 119 der natürlich vorkommenden Form des Rinderwachstumshormons entspricht, Serin ist.

20. Verfahren nach einem der Ansprüche 1 bis 14, wobei die Aminosäure, die an der Position codiert ist, die der Position 119 der natürlich vorkommenden Form des Rinderwachstumshormons entspricht, Glutamin ist.

21. Verfahren nach einem der Ansprüche 1 bis 14, wobei die Aminosäure, die an der Position codiert ist, die der Position 119 der natürlich vorkommenden Form des Rinderwachstumshormons entspricht, Glutaminsäure ist.

22. Verfahren nach einem der Ansprüche 1 bis 14, wobei die Aminosäure, die an der Position codiert ist, die der Position 119 der natürlich vorkommenden Form des Rinderwachstumshormons entspricht, Tryptophan ist.

23. Verfahren nach einem der Ansprüche 1 bis 14, wobei die Aminosäure, die an der Position codiert ist, die der Position 119 der natürlich vorkommenden Form des Rinderwachstumshormons entspricht, Methionin ist.

24. Verfahren nach Anspruch 1, wobei das Peptid oder Protein ausgewählt ist aus der Gruppe
bGH (G119R),
bGH (G119R, E117L, A122D),
bGH (G119K),
bGH (G119L),
Fragmente dieser Proteine und
Fragmente von bGH (G119P),
die Wachstumsinhibitoraktivität in einem Vertebraten haben, wobei bGH die natürlich vorkommende Form von Rinderwachstumshormon (bGH) ist, das identisch ist mit der Rinderwachstumshormon-Mutantensequenz, die in Figur 1 gezeigt ist, bis auf das Vorhandensein eines Glycins anstelle des Arginins, das in Position 119 der Mutantensequenz gezeigt ist.

25. Verfahren nach Anspruch 4, wobei dem Peptid oder Protein mindestens eine Alpha-Helix fehlt, ausgewählt aus Helices, die den ersten, zweiten oder vierten Helices der natürlich vorkommenden Form des Rinderwachstumshormons entsprechen.

26. Verfahren nach einem der Ansprüche 1 bis 25, wobei das Peptid oder Protein sich weiterhin von dem Vertebraten-Wachstumshormon durch eine oder mehrere Substitutionen an Aminosäuren unterscheiden, die den Aminosäuren 115 oder 122 des natürlich vorkommenden Rinderwachstumshormons entsprechen, und/oder den Aminosäuren des natürlich vorkommenden Rinderwachstumshormons, die unter den Vertebraten-Wachstumshormonen nicht konserviert sind, und/oder durch Verkürzung der Aminosäuren, die den Aminosäuren 1 bis 95 und 134 bis 191 des natürlich vorkommenden Wachstumshormons entsprechen.

27. Verfahren nach einem der Ansprüche 1 bis 26, wobei das Peptid oder Protein einen ED50-Wert hat, der niedriger ist als etwa 10mal der ED50-Wert des Wildtyp-Wachstumshormons in einem Test auf die Fähigkeit des Peptids oder Proteins radioaktiv markiertes Wildtyp-Wachstumshormon von einem Mausleber-Membranrezeptor zu ersetzen.

28. Verfahren nach einem der Ansprüche 1 bis 27, wobei das Peptid oder Protein Wachstumsinhibitoraktivität in einem Säuger hat.

29. Verfahren zur Herstellung eines Arzneimittels, umfassend das Mischen eines Peptids oder Proteins gemäß der Definition in einem der Ansprüche 1 bis 28 und eines pharmazeutisch verträglichen Trägers.

30. Rekombinantes DNA-Molekül, umfassend ein Gen, das eine Sequenz einschließt, die ein Peptid oder Protein gemäß der Definition in einem der Ansprüche 1 bis 20 codiert.

31. Molekül nach Anspruch 30, das weiterhin einen Promotor umfaßt, der funktionell mit dem Gen verknüpft ist.

32. Molekül nach Anspruch 31, wobei der Promotor nicht vom Promotor eines Wachstumshormongens abgeleitet ist.

33. Zelle, umfassend das rekombinante DNA-Molekül nach einem der Ansprüche 30 bis 32.

34. Zelle nach Anspruch 33, wobei die Zelle eine Zelle ist, die ein chromosomales Wachstumshormongen exprimiert.

35. Nicht-menschliches transgenes Tier, umfassend eine Vielzahl von Zellen, die ein Gen enthalten, das funktionell mit einem Promotor verknüpft ist wobei das Gen eine Sequenz umfaßt, die ein Peptid oder Protein mit mindestens 11 Aminosäuren codiert, das Wachstumsinhibitoraktivität in einem Vertebraten aufweist und eine mutierte Alpha-Helix umfaßt, die im wesentlichen homolog, aber nicht identisch ist mit der dritten Alpha-Helix eines Vertebraten-Wachstumshormons, wobei die dritte Alpha-Helix der Alpha-Helix entspricht, die von den Aminosäuren an den Positionen 109 bis 126 der natürlich vorkommenden Form des Rinderwachstumshormons (bGH) gebildet wird,
wobei die natürlich vorkommende Form des Rinderwachstumshormons identisch ist mit der Rinderwachstumshormon-Mutantensequenz, die in Figur 1 gezeigt ist, außer daß ein Glycin anstelle des Arginins, das in Position 119 der Mutantensequenz gezeigt ist, vorliegt,
wobei die mutierte Alpha-Helix sich von der dritten Alpha-Helix des Vertebraten-Wachstumshormons mindestens in der Position unterscheidet, die der Position 119 der natürlich vorkommenden Form der Rinderwachstumshormons entspricht.

36. Nicht-menschliches transgenes Tier nach Anspruch 35, wobei eine Vielzahl von Zellen ein rekombinantes DNA-Molekül nach einem der Ansprüche 30 bis 32 umfaßt.

37. Nicht-menschliches Tier nach Anspruch 35 oder 36, das eine reduzierte Wachstumsgeschwindigkeit verglichen mit Kontrolltieren zeigt.

38. Arzneimittel, umfassend ein Peptid oder Protein gemäß der Definition in einem der Ansprüche 1 bis 28 und einen pharmazeutisch verträglichen Träger.

39. Peptid oder Protein gemäß der Definition in einem der Ansprüche 1 bis 29 zur Verwendung als ein Medikament.

40. Rekombinantes DNA-Molekül, umfassend ein Gen, das eine Sequenz einschließt, die ein Peptid oder Protein mit mindestens 11 Aminosäuren codiert, das Wachstumsinhibitoraktivität in einem Vertebraten aufweist und eine mutierte Alpha-Helix umfaßt, die im wesentlichen homolog, aber nicht identisch ist mit der dritten Alpha-Helix eines Vertebraten-Wachstumshormons, wobei die dritte Alpha-Helix der Alpha-Helix entspricht, die von den Aminosäuren an den Positionen 109 bis 126 der natürlich vorkommenden Form des Rinderwachstumshormons (bGH) gebildet wird, wobei die natürlich vorkommende Form des Rinderwachstumshormons identisch ist mit der Rinderwachstumshormon-Mutantensequenz, die in Figur 1 gezeigt ist, außer daß ein Glycin anstelle des Arginins, das an Position 119 der Mutantensequenz gezeigt ist, vorliegt, wobei die mutierte Alpha-Helix sich von der dritten Alpha-Helix des Vertebraten-Wachstumshormons mindestens in der Position unterscheidet, die der Position 119 der natürlich vorkommenden Form des Rinderwachstumshormons entspricht, zur Verwendung als ein Medikament.

41. Rekombinantes DNA-Molekül nach einem der Ansprüche 30 bis 32 zur Verwendung als ein Medikament.

42. Rekombinantes DNA-Molekül nach Anspruch 40 oder 41 zur Hemmung oder Verminderung der Wachstumshormonaktivität in einem menschlichen oder tierischen Individuum.

43. Rekombinantes DNA-Molekül nach einem der Ansprüche 40 bis 42, wobei ein resultierendes genetisch modifiziertes menschliches oder tierisches Individuum fähig ist, das Protein oder Peptid nach einem der Ansprüche 1 bis 27 auf einem Wachstumsinhibitor-Niveau zu exprimieren.

44. Verfahren zur Herstellung eines Medikaments zur Behandlung eines Zustandes, der durch Hemmung oder Verminderung der Wachstumshormonaktivität verbessert werden kann, gekennzeichnet durch die Verwendung eines Peptids oder Proteins mit mindestens 11 Aminosäuren, das Wachstumsinhibitoraktivität in einem Vertebraten aufweist und eine mutierte Alpha-Helix umfaßt, die im wesentlichen homolog, aber nicht identisch ist mit der dritten Alpha-Helix eines Vertebraten-Wachstumshormons, wobei die dritte Alpha-Helix der Alpha-Helix entspricht, die von den Aminosäuren an den Positionen 109 bis 126 der natürlich vorkommenden Form des Rinderwachstumshormons (bGH) gebildet wird, wobei die natürlich vorkommende Form des Rinderwachstumshormons identisch ist mit der Rinderwachstumshormon-Mutantensequenz, die in Figur 1 gezeigt ist, außer daß ein Glycin anstelle des Arginins, das an Position 119 der Mutantensequenz gezeigt ist, vorliegt, wobei die mutierte Alpha-Helix sich von der dritten Alpha-Helix des Vertebraten-Wachstumshormons mindestens in der Position unterscheidet, die der Position 119 der natürlich vorkommenden Form des Rinderwachstumshormons entspricht.

45. Verfahren zur Herstellung eines Medikaments zur Behandlung eines Zustandes, der durch Hemmung oder Verminderung der Wachstumshormonaktivität verbessert werden kann, gekennzeichnet durch die Verwendung eines Peptids oder Proteins nach einem der Ansprüche 1 bis 28.

46. Verwendung eines Peptids oder Proteins mit mindestens 11 Aminosäuren, das Wachstumsinhibitoraktivität in einem Vertebraten aufweist und eine mutierte Alpha-Helix umfaßt, die im wesentlichen homolog, aber nicht identisch ist mit der dritten Alpha-Helix eines Vertebraten-Wachstumshormons, wobei die dritte Alpha-Helix der Alpha-Helix entspricht, die von den Aminosäuren an den Positionen 109 bis 126 der natürlich vorkommenden Form von Rinderwachstumshormon (bGH) gebildet wird, wobei die natürlich vorkommende Form des Rinderwachstumshormons identisch ist mit der Rinderwachstumshormon-Mutantensequenz, die in Figur 1 gezeigt ist, außer daß ein Glycin anstelle des Arginins, das in Position 119 der Mutantensequenz gezeigt ist, vorliegt, wobei die mutierte Alpha-Helix sich von der dritten Alpha-Helix des Vertebraten-Wachstumshormons in mindestens der Position unterscheidet, die der Position 119 der natürlich vorkommenden Form des Rinderwachstumshormons entspricht, für die Herstellung eines Medikaments zur Behandlung eines Zustandes, der durch Hemmung oder Verminderung der Wachstumshormonaktivität verbessert werden kann.

47. Verfahren zur Herstellung eines Mittels zur Behandlung eines Zustandes, der durch Hemmung oder Verminderung der Wachstumshormonaktivität verbessert werden kann, gekennzeichnet durch die Verwendung eines rekombinanten DNA-Moleküls nach einem der Ansprüche 30 bis 32.

48. Verwendung eines rekombinanten DNA-Moleküls, umfassend ein Gen, das eine Sequenz einschließt, die ein Peptid oder Protein mit mindestens 11 Aminosäuren codiert, das Wachstumsinhibitoraktivität in einem Vertebraten aufweist und eine mutierte Alpha-Helix umfaßt, die im wesentlichen homolog, aber nicht identisch ist mit der dritten Alpha-Helix eines Vertebraten-Wachstumshormons, wobei die dritte Alpha-Helix der Alpha-Helix entspricht, die von den Aminosäuren an den Positionen 109 bis 126 der natürlich vorkommenden Form von Rinderwachstumshormon (bGH) gebildet wird, wobei die natürlich vorkommende Form des Rinderwachstumshormons identisch ist mit der Rinderwachstumshormon-Mutantensequenz, die in Figur 1 gezeigt ist, außer daß ein Glycin anstelle des Arginins, das in Position 119 der Mutantensequenz gezeigt ist, vorliegt, wobei die mutierte Alpha-Helix sich von der dritten Alpha-Helix des Vertebraten-Wachstumshormons in mindestens der Position unterscheidet, die der Position 119 der natürlich vorkommenden Form des Rinderwachstumshormons entspricht, für die Herstellung eines Mittels zur Behandlung eines Zustandes, der durch Hemmung oder Verminderung der Wachstumshormonaktivität verbessert werden kann.

49. Isoliertes Peptid oder Protein mit mindestens 11 Aminosäuren, das Wachstumsinhibitoraktivität in einem Vertebraten aufweist und eine mutierte Alpha-Helix umfaßt, die im wesentlichen homolog, aber nicht identisch ist mit der dritten Alpha-Helix eines Vertebraten-Wachstumshormons, wobei die dritte Alpha-Helix der Alpha-Helix entspricht, die von den Aminosäuren an den Positionen 109 bis 126 der natürlich vorkommenden Form des Rinderwachstumshormons (bGH) gebildet wird, wobei die natürlich vorkommende Form des Rinderwachstumshormons identisch ist mit der Rinderwachstumshormon-Mutantensequenz, die in Figur 1 gezeigt ist, außer daß ein Glycin anstelle des Arginins, das an Position 119 der Mutantensequenz gezeigt ist, vorliegt,
wobei die mutierte Alpha-Helix sich von der dritten Alpha-Helix des Vertebraten-Wachstumshormons mindestens in der Position unterscheidet, die der Position 119 der natürlich vorkommenden Form des Rinderwachstumshormons entspricht,
mit der Maßgabe, daß das Protein oder Peptid weder
(a) das Rindersomatotropin ist, das die Aminosäuresequenz von Diagramm 1 der WO 91/00870 (D1) hat, wobei Glycin an Position 119 durch Prolin ersetzt ist; noch
(b) eine Mutante, hPRL (111-129), des vollständigen nativen menschlichen Wachstumshormons (hGH) ist mit der Sequenz, die in Figur 1 von Cunningham et al., Science 243, 1330-36 (1989) (D7) gezeigt ist, wobei die Mutante sich davon nur durch 12 Substitutionen unterscheidet, wie sie für hPRL (111-129) in Tabelle 1 von D7 gezeigt sind.

50. Peptid oder Protein nach Anspruch 49, wobei das Peptid oder Protein Eigenschaften hat, die zum ersten Mal in einem der Ansprüche 2 bis 28 beschrieben sind.

51. Peptid oder Protein nach Anspruch 1, wobei die Aminosäure an der Position, die der Position 119 der natürlich vorkommenden Form des Rinderwachstumshormons entspricht, eine hydrophobe Aminosäure ist, gemäß der Einteilung von Hopp und Woods, PNAS (USA) 78, 3824-28 (1981).

52. Peptid oder Protein nach Anspruch 1, wobei der amphiphile Charakter der mutierten Alpha-Helix, bestimmt gemäß dem Verfahren von Margalit et al., J. Immunol. 138, 2213-29 (1987), reduziert oder unverändert ist, bezogen auf den amphiphilen Charakter der dritten Alpha-Helix an den Positionen 109 bis 126 des Rinderwachstumshormons.

53. Peptid oder Protein nach Anspruch 51 oder 52, zur Verwendung als ein Medikament.

54. Verwendung des Peptids oder Proteins nach Anspruch 51 oder 52 für die Herstellung eines Medikaments zur Behandlung eines Zustandes, der durch Hemmung oder Verminderung der Wachstumshormonaktivität verbessert werden kann.

55. Verfahren zur Herstellung eines Arzneimittels, umfassend das Mischen eines Peptids oder Proteins gemäß der Definition in Anspruch 51 oder 52 und eines pharmazeutisch verträglichen Trägers.

## Revendications (Revendications pour l'(les) Etat(s) contractant(s) suivant(s): AT, BE, CH, LI, DE, DK, GB, IT, LU, NL, SE)

1. Peptide ou protéine isolé ayant au moins 11 acides aminés, ayant une activité inhibitrice de croissance chez un vertébré, et comprenant une hélice alpha mutante, qui est essentiellement homologue de la troisième hélice alpha de l'hormone de croissance d'un vertébré, mais n'y est pas identique, ladite troisième hélice alpha correspondant à l'hélice alpha formée par les acides aminés sur les positions 109 à 126 de la forme naturelle de l'hormone de croissance bovine (bGH), ladite forme naturelle de l'hormone de croissance bovine étant identique à la séquence mutante de l'hormone de croissance bovine décrite sur la Figure 1, à l'exception de la présence d'une glycine au lieu de l'arginine présentée sur la position 119 de la séquence mutante,
où l'hélice alpha mutante diffère de la troisième hélice alpha de l'hormone de croissance du vertébré, au moins sur la position correspondant à la position 119 de ladite forme naturelle de l'hormone de croissance bovine,
à la condition que la protéine ou le peptide ne soit
(a) ni la somatotrophine bovine ayant la séquence d'acides aminés du Tableau 1 de la demande WO 91/00870 (= D1), mais dont une glycine sur la position 119 est remplacée par une proline ;
(b) ni un mutant, hPRL(111-129), de l'hormone de croissance humaine native complète (hGH) ayant la séquence présentée sur la Figure 1 de Cunningham et al., Science 243:1330-1336, 1989 (= D7), ce mutant n'en différant que par les 12 substitutions indiquées pour le hPRL(111-129) du Tableau 1 de D7.

2. Peptide ou protéine selon la revendication 1, qui a la même taille ou est plus grand que ladite hormone de croissance de vertébré.

3. Peptide ou protéine isolé ayant au moins 11 acides aminés, qui est un fragment d'un protéine ayant une activité inhibitrice de croissance chez un vertébré et comprenant une hélice alpha mutante, qui est essentiellement homologue de la troisième hélice alpha de l'hormone de croissance d'un vertébré, mais n'y est pas identique, ladite troisième hélice alpha correspondant à l'hélice alpha formée par les acides aminés sur les positions 109 à 126 de la forme naturelle de l'hormone de croissance bovine (bGH), ladite forme naturelle de l'hormone de croissance bovine étant identique à la séquence mutante de l'hormone de croissance bovine décrite sur la Figure 1, à l'exception de le présence d'une glycine au lieu de l'arginine présentée sur la position 119 de la séquence mutante,
où l'hélice alpha mutante diffère de la troisième hélice alpha de l'hormone de croissance du vertébré, au moins sur la position correspondant à la position 119 de ladite forme naturelle de l'hormone de croissance bovine,
et dans lequel ledit peptide ou protéine isolé a une séquence d'acides aminés qui est tronquée par rapport à celle de ladite hormone de croissance de vertébré.

4. Peptide ou protéine selon l'une quelconque des revendications 1 à 3, dans lequel l'hormone de croissance de vertébré est une hormone de croissance de mammifère.

5. Peptide ou protéine selon l'une quelconque des revendications 1 à 4, dans lequel l'hormone de croissance de vertébré est l'hormone de croissance bovine.

6. Peptide ou protéine selon l'une quelconque des revendications 1 à 4, dans lequel l'hormone de croissance de vertébré est l'hormone de croissance humaine.

7. Peptide ou protéine selon l'uns quelconque des revendications 1 à 3, dans lequel l'hormone de croissance de vertébré est l'hormone de croissance de poulet.

8. Peptide ou protéine selon l'une quelconque des revendications 1 à 4, dans lequel l'hormone de croissance de vertébré est l'hormone de croissance de rat, porcine ou ovine.

9. Peptide ou protéine selon l'une quelconque des revendications 1 à 3, dans lequel l'hormone de croissance de vertébré est l'hormone de croissance de plie grise, de limande à queue jaune, de thon ou de saumon.

10. Peptide ou protéine selon l'une quelconque des revendications 1 à 3, qui est essentiellement homologue de ladite forme naturelle de l'hormone de croissance bovine.

11. Peptide ou protéine selon l'une quelconque des revendications 1 à 3, qui est essentiellement homologue d'une hormone de croissance de vertébré.

12. Peptide ou protéine selon l'une quelconque des revendications 1 à 3, qui est essentiellement homologue d'une hormone de croissance de mammifère.

13. Peptide ou protéine selon l'une quelconque des revendications 1 à 12, dans lequel l'acide aminé sur la position correspondant à la position 119 de ladite forme naturelle de l'hormone de croissance bovine n'est pas l'alanine.

14. Peptide ou protéine selon l'une quelconque des revendications 1 à 13, dans lequel l'acide aminé sur la position correspondant à la position 119 de ladite forme naturelle de l'hormone de croissance bovine est l'arginine.

15. Peptide ou protéine selon l'une quelconque des revendications 1 à 13 dans lequel l'acide aminé sur la position correspondant à la position 119 de ladite forme naturelle de l'hormone de croissance bovine est la proline.

16. Peptide ou protéine selon l'une quelconque des revendications 1 à 13, dans lequel l'acide aminé sur la position correspondant à la position 119 de ladite forme naturelle de l'hormone de croissance bovine est la lysine.

17. Peptide ou protéine selon l'une quelconque des revendications 1 à 13, dans lequel l'acide aminé sur la position correspondant à la position 119 de ladite forme naturelle de l'hormone de croissance bovine est la leucine.

18. Peptide ou protéine selon l'une quelconque des revendications 1 à 13, dans lequel l'acide aminé sur la position correspondant à la position 119 de ladite forme naturelle de l'hormone de croissance bovins est la sérine.

19. Peptide ou protéine selon l'une quelconque des revendications 1 à 13, dans lequel l'acide aminé sur la position correspondant à la position 119 de ladite forme naturelle de l'hormone de croissance bovine est la glutamine.

20. Peptide ou protéine selon l'une quelconque des revendications 1 à 13, dans lequel l'acide aminé sur la position correspondant à la position 119 de ladite forme naturelle de l'hormone de croissance bovine est l'acide glutamique.

21. Peptide ou protéine selon l'une quelconque des revendications 1 à 13, dans lequel l'acide aminé sur la position correspondant à la position 119 de ladite forme naturelle de l'hormone de croissance bovine est le tryptophane.

22. Peptide ou protéine selon l'une quelconque des revendications 1 à 13, dans lequel l'acide aminé sur la position correspondant à la position 119 de ladite forme naturelle de l'hormone de croissance bovine est la méthionine.

23. Protéine isolée choisie dans l'ensemble comprenant :
bGH (G119R),
bGH (G119R E117L, A122D),
bGH (G119K),
bGH (G119L),
les fragments de ces protéines, et
les fragments de bGH (G119P)
ayant une activité inhibitrice de croissance chez un vertébré, où bGH est la forme naturelle de l'hormone de croissance bovine (bGH) qui est identique à la séquence mutante de l'hormone de croissance bovine présentée sur la Figure 1, mais avec présence d'une glycine au lieu de l'arginine présentée sur la position 119 de la séquence mutante.

24. Peptide ou protéine selon la revendication 3, qui manque d'au moins une hélice alpha choisie dans l'ensemble comprenant les hélices correspondant à la première, à la deuxième ou à la quatrième hélice de ladite forme naturelle de l'hormone de croissance bovine.

25. Peptide ou protéine selon l'une quelconque des revendications 1 à 24, qui diffère encore de ladite hormone de croissance de vertébré par une ou plusieurs substitutions au niveau d'acides aminés correspondant aux acides aminés 115 ou 122 de ladite hormone de croissance bovine naturelle, et/ou à des acides aminés de ladite hormone de croissance bovine naturelle qui ne sont pas conservés parmi les hormones de croissance de vertébré, et par une troncation d'acides aminés correspondant aux acides aminés 1-95 et 134-191 de ladite hormone de croissance bovine naturelle.

26. Peptide ou protéine selon l'une quelconque des revendications 1 à 25, dans lequel le peptide ou la protéine a une DE₅₀ qui est inférieure à environ 10 fois la DE₅₀ de l'hormone de croissance de type sauvage, dans un essai destiné à déterminer l'aptitude du peptide ou de la protéine à déplacer, à partir d'un récepteur de membrane de foie de souris, l'hormone de croissance de type sauvage radiomarquée.

27. Peptide ou protéine selon l'une quelconque des revendications 1 à 26, dans lequel le peptide ou la protéine a une activité inhibitrice de croissance chez un mammifère.

28. Molécule d'ADN recombinant comprenant un gène contenant une séquence codant pour le peptide ou la protéine selon l'une quelconque des revendications 1 à 27.

29. Molécule selon la revendication 28, qui comprend en outre un promoteur lié d'une manière opérationnelle audit gène.

30. Molécule selon la revendication 29, dans laquelle ledit promoteur ne dérive pas du promoteur d'un gène d'hormone de croissance.

31. Cellule comprenant la molécule d'ADN recombinant selon l'une quelconque des revendications 28 à 30.

32. Cellule selon la revendication 31, dans laquelle ladite cellule est une cellule qui n'exprime pas un gène d'hormone de croissance chromosomique.

33. Animal transgénique non-humain comprenant une pluralité de cellules comprenant un gène lié d'uns manière opérationnelle à un promoteur, le gène contenant une séquence codant pour un peptide ou une protéine ayant au moins 11 acides aminés et ayant une activité inhibitrice de croissance chez un mammifère, et comprenant une hélice alpha mutante, qui est essentiellement homologue de la troisième hélice alpha de l'hormone de croissance d'un vertébré, mais n'y est pas identique, ladite troisième
hélice alpha correspondant à l'hélice alpha formée par les acides aminés sur les positions 109 à 126 de la forme naturelle de l'hormone de croissance bovine (bGH), ladite forme naturelle de l'hormone de croissance bovine étant identique à la séquence mutante de l'hormone de croissance bovine décrite sur la Figure 1, à l'exception de la présence d'une glycine au lieu de l'arginine présentée sur la position 119 de la séquence mutante,
où l'hélice alpha mutante diffère de la troisième hélice alpha de l'hormone de croissance du vertébré, au moins sur la position correspondant à la position 119 de ladite forme naturelle de l'hormone de croissance bovine.

34. Animal transgénique non-humain selon la revendication 33, dans lequel une pluralité de cellules comprennent un gène contenant une séquence codant pour une protéine ou un peptide selon l'une quelconque des revendications 1 à 27.

35. Animal non-humain selon la revendication 33 ou 34, qui présente une vitesse de croissance réduits par comparaison à des animaux témoins.

36. Procédé de production d'un peptide ou d'une protéine inhibiteur de croissance, qui consiste à mettre à disposition des cellules portant un gène contenant une séquence codant pour un peptide ou une protéine selon l'une quelconque des revendications 1 à 27, dans des conditions conduisant à l'expression dudit gène, ladite protéine étant ainsi produite sous une forme utilisable ou récupérable.

37. Composition pharmaceutique comprenant le peptide ou la protéine selon l'une quelconque des revendications 1 à 27 et un excipient acceptable d'un point de vue pharmaceutique.

38. Peptide ou protéine ayant au moins 11 acides aminés, ayant une activité inhibitrice de croissance chez un vertébré, et comprenant une hélice alpha mutante, qui est essentiellement homologue de la troisième hélice alpha de l'hormone de croissance d'un vertébré, mais n'y est pas identique, ladite troisième hélice alpha correspondant à l'hélice alpha formée par les acides aminés sur les positions 109 à 126 de la forme naturelle de l'hormone de croissance bovine (bGH), ladite forme naturelle de l'hormone de croissance bovine étant identique à la séquence mutante de l'hormone de croissance bovine décrite sur la Figure 1, à l'exception de la présence d'une glycine au lieu de l'arginine présentée sur la position 119 de la séquence mutante,
où l'hélice alpha mutante diffère de la troisième hélice alpha de l'hormone de croissance du vertébré, au moins sur la position correspondant à la position 119 de ladite forme naturelle de l'hormone de croissance bovine,
pour utilisation an tant que médicament,
à la condition que la protéine ou le peptide ne soit
(a) ni la somatotrophine bovins ayant la séquence d'acides aminés du Tableau 1 de la demande WO 91/00870 (= D1), mais dont une glycine sur la position 119 est remplacée par une proline ;
(b) ni un mutent, hPRL(111-129), de l'hormone de croissance humaine native complète (hGH) ayant la séquence présentés sur la Figure 1 de Cunningham et al., Science 243:1330-1336, 1989 (= D7), ce mutant n'en différant que par les 12 substitutions indiquées pour le hPRL(111-129) du Tableau 1 de D7.

39. Peptide ou protéine selon l'une quelconque des revendications 1 à 27, ou peptide ou protéine selon la revendication 38, ayant les propriétés mentionnées an premier dans l'une quelconque des revendications 2 à 27, pour utilisation en tant que médicament.

40. Molécule d'ADN recombinant comprenant un gène contenant une séquence codant pour un peptide ou une protéine ayant au moins 11 acides aminés et ayant une activité inhibitrice de croissance chez un vertébré, et comprenant une hélice alpha mutante, qui est essentiellement homologue de la troisième hélice alpha de l'hormone de croissance d'un vertébré, mais n'y est pas identique, ladite troisième hélice alpha correspondant à l'hélice alpha formée par les acides aminés sur les positions 109 à 126 de la forme naturelle de l'hormone de croissance bovine (bGH), ladite forme naturelle de l'hormone de croissance bovine étant identique à la séquence mutante de l'hormone de croissance bovine décrite sur la Figure 1, à l'exception de la présence d'une glycine au lieu de l'arginine présentée sur la position 119 de la séquence mutante,
où l'hélice alpha mutante diffère de la troisième hélice alpha de l'hormone de croissance du vertébré, au moins sur la position correspondant à la position 119 de ladite forme naturelle de l'hormone de croissance bovine,
pour utilisation en tant que médicament.

41. Molécule d'ADN recombinant selon l'une quelconque des revendications 28 à 30, ou molécule d'ADN recombinant selon la revendication 40, codant pour un peptide ou une protéine ayant les propriétés mentionnées en premier dans l'une quelconque des revendications 2 à 27, pour utilisation en tant que médicament.

42. Molécule recombinante selon la revendication 40 ou 41, pour inhiber ou réduire l'activité d'hormone de croissance chez un sujet humain ou animal.

43. Molécule d'ADN recombinant selon l'une quelconque des revendications 40 à 42, dans lequel le sujet humain ou animal génétiquement modifié obtenu est capable d'exprimer la protéine ou le peptide selon l'une quelconque des revendications 1 à 27 ou 38, à un niveau inhibiteur de croissance.

44. Utilisation d'un peptide ou d'une protéine ayant au moins 11 acides aminés et ayant une activité inhibitrice Ce croissance chez un vertébré, et comprenant une hélice alpha mutante, qui est essentiellement homologue de la troisième hélice alpha de l'hormone de croissance d'un vertébré, mais n'y est pas identique, ladite troisième hélice alpha correspondant à l'hélice alpha formée par les acides aminés sur les positions 109 à 126 de la forme naturelle de l'hormone de croissance bovine (bGH), ladite forme naturelle de l'hormone de croissance bovine étant identique à la séquence mutante de l'hormone de croissance bovine décrite sur la Figure 1, à l'exception de la présence d'une glycine au lieu de l'arginine présentée sur la position 119 de la séquence mutante,
où l'hélice alpha mutante diffère de la troisième hélice alpha de l'hormone de croissance du vertébré, au moins sur la position correspondant à la position 119 de ladite forme naturelle de l'hormone de croissance bovine,
pour préparer un médicament destiné au traitement d'un état pouvant être amélioré par inhibition ou réduction de l'activité de l'hormone de croissance.

45. Utilisation d'un peptide ou d'une protéine selon l'une quelconque des revendications 1 à 27, ou utilisation d'un peptide ou d'une protéine selon la revendication 44 ayant les propriétés mentionnées en premier dans l'une quelconque des revendications 2 à 27, pour préparer un médicament destiné au traitement d'un état pouvant être amélioré par inhibition ou réduction de l'activité de l'hormone de croissance.

46. Utilisation d'une molécule d'ADN recombinant comprenant un gène contenant une séquence codant pour un peptide ou une protéine ayant au moins 11 acides aminés et ayant une activité inhibitrice de croissance chez un vertébré, et comprenant une hélice alpha mutante, qui est essentiellement homologue de la troisième hélice alpha de l'hormone de croissance d'un vertébré, mais n'y est pas identique, ladite troisième hélice alpha correspondant à l'hélice alpha formée par les acides aminés sur les positions 109 à 126 de la forme naturelle de l'hormone de croissance bovine (bGH), ladite forme naturelle de l'hormone de croissance bovine étant identique à la séquence mutante de l'hormone de croissance bovine décrite sur la Figure 1, à l'exception de la présence d'une glycine au lieu de l'arginine présentée sur la position 119 de la séquence mutante,
où l'hélice alpha mutante diffère de la troisième hélice alpha de l'hormone de croissance du vertébré, au moins sur la position correspondant à la position 119 de ladite forme naturelle de l'hormone de croissance bovine,
pour préparer une composition destinée au traitement d'un état pouvant être amélioré par inhibition ou réduction de l'activité de l'hormone de croissance.

47. Utilisation d'une molécule d'ADN recombinant selon l'une quelconque des revendications 28 à 30, ou utilisation d'une molécule d'ADN recombinant selon la revendication 46, codant pour un peptide ou une protéine ayant les propriétés mentionnées en premier dans l'une quelconque des revendications 2 à 27, pour préparer une composition destinée au traitement d'un état pouvant être amélioré par inhibition ou réduction de l'activité de l'hormone de croissance.

48. Peptide ou protéine selon la revendication 3, qui a au moins 50 acides aminés.

49. Peptide ou protéine selon l'une quelconque des revendications 1 à 27, qui a une activité inhibitrice de croissance chez l'homme.

50. Peptide ou protéine selon la revendication 1, dans lequel l'acide aminé sur la position correspondant à la position 119 de ladite forme naturelle de l'hormone de croissance bovine est un acide aminé hydrophobe, selon l'échelle de Hopp et Woods, PNAS (USA) 78:3824-3828 (1981).

51. Peptide ou protéine selon la revendication 1, dans lequel le caractère amphiphile de l'hélice alpha mutante, tel que déterminé par la méthode de Margalit et al., J. Immunol. 138:2213-2229 (1987) est réduit ou inchangé par rapport au caractère amphiphile de la troisième hélice alpha sur les positions 109 à 126 de ladite hormone de croissance bovine.

52. Peptide ou protéine selon la revendication 50 ou 51, pour utilisation an tant que médicament.

53. Utilisation du peptide ou de la protéine selon la revendication 50 ou 51 pour préparer un médicament destiné au traitement d'un état pouvant être amélioré par inhibition ou réduction de l'activité de l'hormone de croissance.

## Revendications (Revendications pour l'(les) Etat(s) contractant(s) suivant(s): GR)

1. Molécule d'ADN recombinant comprenant un gène contenant une séquence codant pour un peptide ou protéine isolé ayant au moins 11 acides aminés, ayant une activité inhibitrice de croissance chez un vertébré, et comprenant une hélice alpha mutante, qui est essentiellement homologue de la troisième hélice alpha de l'hormone de croissance d'un vertébré, mais n'y est pas identique, ladite troisième hélice alpha correspondant à l'hélice alpha formée par les acides aminés sur les positions 109 à 126 de la forme naturelle de l'hormone de croissance bovine (bGH), ladite forme naturelle de l'hormone de croissance bovine étant identique à la séquence mutante de l'hormone de croissance bovine décrite sur la Figure 1, à l'exception de la présence d'une glycine au lieu de l'arginine présentée sur la position 119 de la séquence mutante,
où l'hélice alpha mutante diffère de la troisième hélice alpha de l'hormone de croissance du vertébré, au moins sur la position correspondant à la position 119 de ladite forme naturelle de l'hormone de croissance bovine,
à la condition que la protéine ou le peptide ne soit
(a) ni la somatotrophine bovine ayant la séquence d'acides aminés du Tableau 1 de la demande WO 91/00870 (= D1), mais dont une glycine sur la position 119 est remplacée par une proline ;
(b) ni un mutant, hPRL(111-129), de l'hormone de croissance humaine native complète (hGH) ayant la séquence présentée sur la Figure 1 de Cunningham et al., Science 243:1330-1336, 1989 (= D7), ce mutant n'en différant que par les 12 substitutions indiquées pour le hPRL(111-129) du Tableau 1 de D7.

2. Molécule selon la revendication 1, qui a la même taille ou est plus grand que ladite hormone de croissance de vertébré.

3. Molécule d'ADN recombinant comprenant un gène contenant une séquence codant pour un peptide ou protéine isolé ayant au moins 11 acides aminés, qui est un fragment d'un protéine ayant une activité inhibitrice de croissance chez un vertébré, et comprenant une hélice alpha mutante, qui est essentiellement homologue de la troisième hélice alpha de l'hormone de croissance d'un vertébré, mais n'y est pas identique, ladite troisième hélice alpha correspondant à l'hélice alpha formée par les acides aminés sur les positions 109 à 126 de la forme naturelle de l'hormone de croissance bovine (bGH), ladite forme naturelle de l'hormone de croissance bovine étant identique à la séquence mutante de l'hormone de croissance bovine décrite sur la Figure 1, à l'exception de la présence d'une glycine au lieu de l'arginine présentée sur la position 119 de la séquence mutante.

4. Molécule selon l'une quelconque des revendications 1 à 3, dans laquelle l'hormone de croissance de vertébré est une hormone de croissance de mammifère.

5. Molécule selon l'une quelconque des revendications 1 à 4, dans laquelle l'hormone de croissance de vertébré est l'hormone de croissance bovine.

6. Molécule selon l'une quelconque des revendications 1 à 4, dans laquelle l'hormone de croissance de vertébré est l'hormone de croissance humaine.

7. Molécule selon l'une quelconque des revendications 1 à 3, dans laquelle l'hormone de croissance de vertébré est l'hormone de croissance de poulet.

8. Molécule selon l'une quelconque des revendications 1 à 4, dans laquelle l'hormone de croissance de vertébré est l'hormone de croissance de rat, porcine ou ovine.

9. Molécule selon l'une quelconque des revendications 1 à 3, dans laquelle l'hormone de croissance de vertébré est l'hormone de croissance de plie grise, de limande à queue jaune, de thon ou de saumon.

10. Molécule selon l'une quelconque des revendications 1 à 3, dans laquelle le peptide ou la protéine est essentiellement homologue de ladite forme naturelle de l'hormone de croissance bovine.

11. Molécule selon l'une quelconque des revendications 1 à 3, dans laquelle le peptide ou la protéine est essentiellement homologue d'une hormone de croissance de vertébré.

12. Molécule selon l'une quelconque des revendications 1 à 3, dans laquelle le peptide ou la protéine est essentiellement homologue d'une hormone de croissance de mammifère.

13. Molécule selon l'une quelconque des revendications 1 à 12, dans laquelle l'acide aminé sur la position correspondant à la position 119 de ladite forme naturelle de l'hormone de croissance bovine n'est pas l'alanine.

14. Molécule selon l'une quelconque des revendications 1 à 13, dans laquelle l'acide aminé sur la position correspondant à la position 119 de ladite forme naturelle de l'hormone de croissance bovine est l'arginine.

15. Molécule selon l'une quelconque des revendications 1 à 13, dans laquelle l'acide aminé sur la position correspondant à la position 119 de ladite forme naturelle de l'hormone de croissance bovine est la proline.

16. Molécule selon l'une quelconque des revendications 1 à 13, dans laquelle l'acide aminé sur la position correspondant à la position 119 de ladite forme naturelle de l'hormone de croissance bovine est la lysine.

17. Molécule selon l'une quelconque des revendications 1 à 13, dans laquelle l'acide aminé sur la position correspondant à la position 119 de ladite forme naturelle de l'hormone de croissance bovine est la leucine.

18. Molécule selon l'une quelconque des revendications 1 à 13, dans laquelle l'acide aminé sur la position correspondant à la position 119 de ladite forme naturelle de l'hormone de croissance bovine est la sérine.

19. Molécule selon l'une quelconque des revendications 1 à 13, dans laquelle l'acide aminé sur la position correspondant à la position 119 de ladite forme naturelle de l'hormone de croissance bovine est la glutamine.

20. Molécule selon l'une quelconque des revendications 1 à 13, dans laquelle l'acide aminé sur la position correspondant à la position 119 de ladite forme naturelle de l'hormone de croissance bovine est l'acide glutamique.

21. Molécule selon l'une quelconque des revendications 1 à 13, dans laquelle l'acide aminé sur la position correspondant à la position 119 de ladite forme naturelle de l'hormone de croissance bovine est le tryptophane.

22. Molécule selon l'une quelconque des revendications 1 à 13, dans laquelle l'acide aminé sur la position correspondant à la position 119 de ladite forme naturelle de l'hormone de croissance bovine est la méthionine.

23. Molécule selon la revendication 1, dans laquelle le peptide ou la protéine est choisi dans l'ensemble comprenant :
bGH (G119R),
bGH (G119R, E117L, A122D),
bGH (G119K),
bGH (G119L),
les fragments de ces protéines, et
les fragments de bGH (G119P)
ayant une activité inhibitrice de croissance chez un vertébré, où bGH est la forme naturelle de l'hormone de croissance bovine (bGH) qui est identique à la séquence mutante de l'hormone de croissance bovine présentée sur la Figure 1, mais avec présence d'une glycine au lieu de l'arginine présentée sur la position 119 de la séquence mutante.

24. Molécule selon la revendication 3, dans laquelle le peptide ou la protéine manque d'au moins une hélice alpha choisie dans l'ensemble comprenant les hélices correspondant à la première, à la deuxième ou à la quatrième hélice de ladite forme naturelle de l'hormone de croissance bovine.

25. Molécule selon l'une quelconque des revendications 1 à 24, dans laquelle le peptide ou la protéine diffère an outre de ladite hormone de croissance de vertébré par une ou plusieurs substitutions au niveau d'acides aminés correspondant aux acides aminés 115 ou 122 de ladite hormone de croissance bovine naturelle, et/ou à des acides aminés de ladite hormone de croissance bovine naturelle qui ne sont pas conservés parmi les hormones de croissance de vertébré, et par une troncation d'acides aminés correspondant aux acides aminés 1-95 et 134-191 de ladite hormone de croissance bovine naturelle.

26. Molécule salon l'une quelconque des revendications 1 à 25, dans laquelle le peptide ou la protéine a une DE₅₀ inférieure à environ 10 fois la DE₅₀ de l'hormone de croissance de type sauvage, dans un essai destiné à déterminer l'aptitude du peptide ou de la protéine à déplacer, à partir d'un récepteur de membrane de foie de souris, l'hormone de croissance de type sauvage radiomarquée.

27. Molécule selon l'une quelconque des revendications 1 à 26, dans laquelle le peptide ou la protéine a une activité inhibitrice de croissance chez un mammifère.

28. Molécule selon l'une quelconque des revendications 1 à 27, qui comprend en outre un promoteur lié d'une manière opérationnelle audit gène.

29. Molécule selon la revendication 28, dans laquelle ledit promoteur ne dérive pas du promoteur d'un gène d'hormone de croissance.

30. Cellule comprenant la molécule d'ADN recombinant selon l'une quelconque des revendications 1 à 29.

31. Cellule selon la revendication 30, dans laquelle ladite cellule est une cellule qui n'exprime pas un gène d'hormone de croissance chromosomique.

32. Peptide ou protéine isolé ayant au moins 11 acides aminés, ayant une activité inhibitrice de croissance chez un vertébré, et comprenant une hélice alpha mutante, qui est essentiellement homologue de la troisième hélice alpha de l'hormone de croissance d'un vertébré, mats n'y est pas identique, ladite troisième hélice alpha correspondant à l'hélice alpha formée par les acides aminés sur les positions 109 à 126 de la forme naturelle de l'hormone de croissance bovine (bGH), ladite forme naturelle de l'hormone de croissance bovine étant identique à la séquence mutante de l'hormone de croissance bovine décrite sur la Figure 1, à l'exception de la présence d'une glycine au lieu de l'arginine présentée sur la position 119 de la séquence mutante,
où l'hélice alpha mutante diffère de la troisième hélice alpha de l'hormone de croissance du vertébré, au moins sur la position correspondant à la position 119 de ladite forme naturelle de l'hormone de croissance bovine,
à la condition que la protéine ou le peptide ne soit
(a) ni la somatotrophine bovine ayant la séquence d'acides aminés du Tableau 1 de la demande WO 91/00870 (= D1), mais dont une glycine sur la position 119 est remplacée par une proline ;
(b) ni un mutant, hPRL(111-129), de l'hormone de croissance humaine native complète (hGH) ayant la séquence présentée sur la Figure 1 de Cunningham et al., Science 243:1330-1336, 1989 (= D7), ce mutant n'en différant que par les 12 substitutions indiquées pour le hPRL(111-129) du Tableau 1 de D7.

33. Peptide ou protéine selon la revendication 32, dans lequel le peptide ou la protéine a les propriétés mentionnées en premier dans l'une quelconque des revendications 2 à 27.

34. Animal transgénique non-humain comprenant une pluralité de cellules comprenant un gène lié d'une manière opérationnelle à un promoteur, le gène contenant une séquence codant pour un peptide ou une protéine ayant au moins 11 acides aminés et ayant une activité inhibitrice de croissance chez un mammifère, et comprenant une hélice alpha mutante, qui est essentiellement homologue de la troisième hélice alpha de l'hormone de croissance d'un vertébré, mais n'y est pas identique, ladite troisième hélice alpha correspondant à l'hélice alpha formée par les acides aminés sur les positions 109 à 126 de la forme naturelle de l'hormone de croissance bovine (bGH), ladite forme naturelle de l'hormone de croissance bovine étant identique à la séquence mutante de l'hormone de croissance bovine décrite sur la Figure 1, à l'exception de la présence d'une glycine au lieu de l'arginine présentée sur la position 119 de la séquence mutante,
où l'hélice alpha mutante diffère de la troisième hélice alpha de l'hormone de croissance du vertébré, au moins sur la position correspondant à la position 119 de ladite forme naturelle de l'hormone de croissance bovine.

35. Animal transgénique non-humain selon la revendication 34, dans lequel une pluralité de cellules comprennent une molécule d'ADN recombinant selon l'une quelconque des revendications 1 à 29.

36. Animal non-humain selon la revendication 34 ou 35, qui présente une vitesse de croissance réduite par comparaison à des animaux témoins.

37. Procédé de production d'un peptide ou d'une protéine inhibiteur de croissance, qui consiste à mettre à disposition des cellules portant un gène contenant une séquence codant pour un peptide ou une protéine selon la revendication 32 ou 33, dans des conditions conduisant à l'expression dudit gène, ladite protéine étant ainsi produite sous une forme utilisable ou récupérable.

38. Composition pharmaceutique comprenant le peptide ou la protéine selon la revendication 32 ou 33 et un excipient acceptable d'un point de vue pharmaceutique.

39. Procédé de préparation d'une composition pharmaceutique, qui consiste à mettre en mélange un peptide ou une protéine selon la revendication 32 ou 33 et un excipient acceptable d'un point de vue pharmaceutique.

40. Molécule d'ADN recombinant comprenant un gène contenant une séquence codant pour un peptide ou une protéine ayant au moins 11 acides aminés et ayant une activité inhibitrice de croissance chez un vertébré, et comprenant une hélice alpha mutante, qui est essentiellement homologue de la troisième hélice alpha de l'hormone de croissance d'un vertébré, mais n'y est pas identique, ladite troisième hélice alpha correspondant à l'hélice alpha formée par les acides aminés sur les positions 109 à 126 de la forme naturelle de l'hormone de croissance bovine (bGH), ladite forme naturelle de l'hormone de croissance bovine étant identique à la séquence mutante de l'hormone de croissance bovine décrite sur la Figure 1, à l'exception de la présence d'une glycine au lieu de l'arginine présentée sur la position 119 de la séquence mutante,
où l'hélice alpha mutante diffère de la troisième hélice alpha de l'hormone de croissance du vertébré, au moins sur la position correspondant à la position 119 de ladite forme naturelle de l'hormone de croissance bovine,
pour utilisation en tant que médicament.

41. Molécule d'ADN recombinant selon l'une quelconque des revendications 1 à 29, pour utilisation en tant que médicament.

42. Molécule d'ADN recombinant selon la revendication 40 ou 41, pour inhiber ou réduire l'activité d'hormone de croissance chez un sujet humain ou animal.

43. Molécule d'ADN recombinant selon l'une quelconque des revendications 40 à 42, grâce à laquelle un sujet humain ou animal génétiquement modifié tel qu'obtenu est capable d'exprimer la protéine ou le peptide selon l'une quelconque des revendications 1 à 27 a un niveau inhibiteur de croissance.

44. Utilisation d'un peptide ou d'une protéine ayant au moins 11 acides aminés et ayant une activité inhibitrice de croissance chez un vertébré, et comprenant une hélice alpha mutante, qui est essentiellement homologue de la troisième hélice alpha de l'hormone de croissance d'un vertébré, mais n'y est pas identique, ladite troisième hélice alpha correspondant à l'hélice alpha formée par les acides aminés sur les positions 109 à 126 de la forme naturelle de l'hormone de croissance bovine (bGH), ladite forme naturelle de l'hormone de croissance bovine étant identique à la séquence mutante de l'hormone de croissance bovine décrite sur la Figure 1, à l'exception de la présence d'une glycine au lieu de l'arginine présentée sur la position 119 de la séquence mutante,
où l'hélice alpha mutante diffère de la troisième hélice alpha de l'hormone de croissance du vertébré, au moins sur la position correspondant à la position 119 de ladite forme naturelle de l'hormone de croissance bovine,
pour préparer un médicament destiné au traitement d'un état pouvant être amélioré par inhibition ou réduction de l'activité de l'hormone de croissance.

45. Utilisation d'un peptide ou d'une protéine selon l'une quelconque des revendications 1 à 27 pour préparer un médicament destiné au traitement d'un état pouvant être amélioré par inhibition ou réduction de l'activité de l'hormone de croissance.

46. Utilisation d'une molécule d'ADN recombinant comprenant un gène contenant une séquence codant pour un peptide ou une protéine ayant au moins 11 acides aminés et ayant une activité inhibitrice de croissance chez un vertébré, et comprenant une hélice alpha mutante, qui est essentiellement homologue de la troisième hélice alpha de l'hormone de croissance d'un vertébré, mais n'y est pas identique, ladite troisième hélice alpha correspondant à l'hélice alpha formée par les acides aminés sur les positions 109 à 126 de la forme naturelle de l'hormone de croissance bovine (bGH), ladite forme naturelle de l'hormone de croissance bovine étant identique à la séquence mutante de l'hormone de croissance bovine décrite sur la Figure 1, à l'exception de la présence d'une glycine au lieu de l'arginine présentée sur la position 119 de la séquence mutante,
où l'hélice alpha mutante diffère de la troisième hélice alpha de l'hormone de croissance du vertébré, au moins sur la position correspondant à la position 119 de ladite forme naturelle de l'hormone de croissance bovine,
pour préparer une composition destinée au traitement d'un état pouvant être amélioré par inhibition ou réduction de l'activité de l'hormone de croissance,

47. Utilisation d'une molécule d'ADN recombinant selon l'une quelconque des revendications 1 à 29 pour préparer uns composition destinée au traitement d'un état pouvant être amélioré par inhibition ou réduction de l'activité de l'hormone de croissance.

48. Peptide ou protéine selon la revendication 3, qui a au moins 50 acides aminés.

49. Peptide ou protéine selon l'une quelconque des revendications 1 à 27, qui a une activité inhibitrice de croissance chez l'homme.

50. Peptide ou protéine selon la revendication 1, dans lequel l'acide aminé sur la position correspondant à la position 119 de ladite forme naturelle de l'hormone de croissance bovine est un acide aminé hydrophobe, selon l'échelle de Hopp et Woods, PNAS (USA) 78:3824-3828 (1981).

51. Peptide ou protéine selon la revendication 1, dans lequel le caractère amphiphile de l'hélice alpha mutante, tel que déterminé par la méthode de Margalit et al., J. Immunol. 138:2213-2229 (1987) est réduit ou inchangé par rapport au caractère amphiphile de la troisième hélice alpha sur les positions 109 à 126 de ladite hormone de croissance bovine.

52. Peptide ou protéine selon la revendication 50 ou 51, pour utilisation an tant que médicament.

53. Utilisation du peptide ou de la protéine selon la revendication 50 ou 51 pour préparer un médicament destiné au traitement d'un état pouvant être amélioré par inhibition ou réduction de l'activité de l'hormone de croissance.

54. Procédé de préparation d'une composition pharmaceutique, qui consiste à mettre en mélange un peptide ou une protéine selon la revendication 50 ou 51 et un excipient acceptable d'un point de vue pharmaceutique.

## Revendications (Revendications pour l'(les) Etat(s) contractant(s) suivant(s): ES)

1. Procédé de production d'un peptide ou d'une protéine inhibiteur de croissance, qui consiste à mettre à disposition des cellules portant un gène, lequel gène contient une séquence codant pour un peptide ou une protéine ayant au moins 11 acides aminés et ayant une activité inhibitrice de croissance chez un vertébré, et comprenant une hélice alpha mutante, qui est essentiellement homologue de la troisième hélice alpha de l'hormone de croissance d'un vertébré, mais n'y est pas identique, ladite troisième hélice alpha correspondant à l'hélice alpha formée par les acides aminés sur les positions 109 à 126 de la forme naturelle de l'hormone de croissance bovine (bGH), ladite forme naturelle de l'hormone de croissance bovine étant identique à la séquence mutante de l'hormone de croissance bovine décrite sur la Figure 1, à l'exception de la présence d'une glycine au lieu de l'arginine présentée sur la position 119 de la séquence mutante,
où l'hélice alpha mutante diffère de la troisième hélice alpha de l'hormone de croissance du vertébré, au moins sur la position correspondant à la position 119 de ladite forme naturelle de l'hormone de croissance bovine,
à la condition que la protéine ou le peptide ne soit
(a) ni la somatotrophine bovine ayant la séquence d'acides aminés du Tableau 1 de la demande WO 91/00870 (= D1), mais dont une glycine sur la position 119 est remplacée par une proline ;
(b) ni un mutent, hPRL(111-129), de l'hormone de croissance humaine native complète (hGH) ayant la séquence présentée sur la Figure 1 de Cunningham et al., Science 243:1330-1336, 1989 (= D7), ce mutant n'en différant que par les 12 substitutions indiquées pour le hPRL(111-129) du Tableau 1 de D7,
dans des conditions conduisant à l'expression dudit gène, ce qui conduit à la production de ladite protéine sous une forme utilisable ou récupérable.

2. Procédé selon la revendication 1, qui comprend en outre l'étape de préparation des cellules par transformation de cellules hôtes pour les amener à porter ledit gène.

3. Procédé selon la revendication 1 ou 2, dans lequel le peptide ou la protéine a la même taille ou est plus grand que ladite hormone de croissance de vertébré.

4. Procédé de production d'un peptide ou d'une protéine inhibiteur de croissance, qui consiste à mettre à disposition des cellules portant un gène, lequel gène contient une séquence codant pour un peptide ou une protéine ayant au moins 11 acides aminés, qui est un fragment d'une protéine ayant une activité inhibitrice de croissance chez un vertébré, et comprenant une hélice alpha mutante, qui est essentiellement homologue de la troisième hélice alpha de l'hormone de croissance d'un vertébré, mais n'y est pas identique, ladite troisième hélice alpha correspondant à l'hélice alpha formée par les acides aminés sur les positions 109 à 126 de la forme naturelle de l'hormone de croissance bovine (bGH), ladite forme naturelle de l'hormone de croissance bovine étant identique à la séquence mutante de l'hormone de croissance bovine décrite sur la Figure 1, à l'exception de la présence d'une glycine au lieu de l'arginine présentée sur la position 119 de la séquence mutante,
dans des conditions conduisant à l'expression dudit gène, ce qui conduit à la production de ladite protéine sous une forme utilisable ou récupérable.

5. Procédé selon l'une quelconque des revendications 1 à 4, dans lequel l'hormone de croissance de vertébré est une hormone de croissance de mammifère.

6. Procédé selon l'une quelconque des revendications 1 à 5, dans lequel l'hormone de croissance de vertébré est l'hormone de croissance bovine.

7. Procédé selon l'une quelconque des revendications 1 à 5, dans lequel l'hormone de croissance de vertébré est l'hormone de croissance humaine.

8. Procédé selon l'une quelconque des revendications 1 à 4, dans lequel l'hormone de croissance de vertébré est l'hormone de croissance de poulet.

9. Procédé selon l'une quelconque des revendications 1 à 5, dans lequel l'hormone de croissance de vertébré est l'hormone de croissance de rat, porcine ou ovine.

10. Procédé selon l'une quelconque des revendications 1 à 4, dans lequel l'hormone de croissance de vertébré est l'hormone de croissance de plie grise, de limande à queue jaune, de thon ou de saumon.

11. Procédé selon l'une quelconque des revendications 1 à 4, dans lequel le peptide ou la protéine est essentiellement homologue de ladite forme naturelle de l'hormone de croissance bovine.

12. Procédé selon l'une quelconque des revendications 1 à 4, dans lequel le peptide ou la protéine est essentiellement homologue d'une hormone de croissance de vertébré.

13. Procédé selon l'une quelconque des revendications 1 à 4, dans lequel le peptide ou la protéine est essentiellement homologue d'une hormone de croissance de mammifère.

14. Procédé selon l'une quelconque des revendications 1 à 13, dans lequel l'acide aminé sur la position correspondant à la position 119 de ladite forme naturelle de l'hormone de croissance bovine n'est pas l'alanine.

15. Procédé selon l'une quelconque des revendications 1 à 14, dans lequel l'acide aminé sur la position correspondant à la position 119 de ladite forme naturelle de l'hormone de croissance bovine est l'arginine.

16. Procédé selon l'une quelconque des revendications 1 à 14, dans lequel l'acide aminé sur la position correspondant à la position 119 de ladite forme naturelle de l'hormone de croissance bovine est la proline.

17. Procédé selon l'une quelconque des revendications 1 à 14, dans lequel l'acide aminé sur la position correspondant à la position 119 de ladite forme naturelle de l'hormone de croissance bovine est la lysine.

18. Procédé selon l'une quelconque des revendications 1 à 14, dans lequel l'acide aminé sur la position correspondant à la position 119 de ladite forme naturelle de l'hormone de croissance bovine est la leucine.

19. Procédé selon l'une quelconque des revendications 1 à 14, dans lequel l'acide aminé sur la position correspondant à la position 119 de ladite forme naturelle de l'hormone de croissance bovine est la sérine.

20. Procédé selon l'une quelconque des revendications 1 à 14, dans lequel l'acide aminé sur la position correspondant à la position 119 de ladite forme naturelle de l'hormone de croissance bovine est la glutamine.

21. Procédé selon l'une quelconque des revendications 1 à 14, dans lequel l'acide aminé sur la position correspondant à la position 119 de ladite forme naturelle de l'hormone de croissance bovine est l'acide glutamique.

22. Procédé selon l'une quelconque des revendications 1 à 14, dans lequel l'acide aminé sur la position correspondant à la position 119 de ladite forme naturelle de l'hormone de croissance bovine est le tryptophane.

23. Procédé selon l'une quelconque des revendications 1 à 14, dans lequel l'acide aminé sur la position correspondant à la position 119 de ladite forme naturelle de l'hormone de croissance bovine est la méthionine.

24. Procédé selon la revendication 1, dans lequel le peptide ou la protéine est choisi dans l'ensemble comprenant :
bGH (G119R),
bGH (G119R, E117L, A122D),
bGH (G119K),
bGH (G119L),
les fragments de ces protéines, et
les fragments de bGH (G119P)
ayant une activité inhibitrice de croissance chez un vertébré, où bGH est la forme naturelle de l'hormone de croissance bovine (bGH) qui est identique à la séquence mutante de l'hormone de croissance bovine présentée sur la Figure 1, mais avec présence d'une glycine au lieu de l'arginine présentée sur la position 119 de la séquence mutante.

25. Procédé selon la revendication 4, dans lequel le peptide ou la protéine manque d'au moins une hélice alpha choisie dans l'ensemble comprenant les hélices correspondant à la première, à la deuxième ou à la quatrième hélice de ladite forme naturelle de l'hormone de croissance bovine.

26. Procédé selon l'une quelconque des revendications 1 à 25, dans lequel le peptide ou protéine diffère an outre de ladite hormone de croissance de vertébré par une ou plusieurs substitutions au niveau d'acides aminés correspondant aux acides aminés 115 ou 122 de ladite hormone de croissance bovine naturelle, et/ou à des acides aminés de ladite hormone de croissance bovine naturelle qui ne sont pas conservés parmi les hormones de croissance de vertébré, et par une troncation d'acides aminés correspondant aux acides aminés 1-95 et 134-191 de ladite hormone de croissance bovine naturelle.

27. Procédé selon l'une quelconque des revendications 1 à 26, dans lequel le peptide ou la protéine a une DE₅₀ qui est inférieure à environ 10 fois la DE₅₀ de l'hormone de croissance de type sauvage, dans un essai destiné à déterminer l'aptitude du peptide ou de la protéine à déplacer, à partir d'un récepteur de membrane de foie de souris, l'hormone de croissance de type sauvage radiomarquée.

28. Procédé selon l'une quelconque des revendications 1 à 27, dans lequel le peptide ou la protéine a une activité inhibitrice de croissance chez un mammifère.

29. Procédé de préparation d'une composition pharmaceutique, qui consiste à mettre en mélange un peptide ou une protéine selon l'une quelconque des revendications 1 à 28 et un excipient acceptable d'un point de vue pharmaceutique.

30. Molécule d'ADN recombinant comprenant un gène contenant une séquence codant pour le peptide ou la protéine selon l'une quelconque des revendications 1 à 20.

31. Molécule selon la revendication 30, qui comprend an outre un promoteur lié d'une manière opérationnelle audit gène.

32. Molécule selon la revendication 31, dans laquelle ledit promoteur ne dérive pas du promoteur d'un gène d'hormone de croissance.

33. Cellule comprenant la molécule d'ADN recombinant selon l'une quelconque des revendications 30 à 32.

34. Cellule selon la revendication 33, dans laquelle ladite cellule est une cellule qui n'exprime pas un gène d'hormone de croissance chromosomique.

35. Animai transgénique non-humain comprenant une pluralité de cellules comprenant un gène lié d'une manière opérationnelle à un promoteur, le gène contenant une séquence codant pour un peptide ou une protéine ayant au moins 11 acides aminés et ayant une activité inhibitrice de croissance chez un mammifère, et comprenant une hélice alpha mutante, qui est essentiellement homologue de la troisième hélice alpha de l'hormone de croissance d'un vertébré, mais n'y est pas identique, ladite troisième hélice alpha correspondant à l'hélice alpha formée par les acides aminés sur les positions 109 à 126 de la forme naturelle de l'hormone de croissance bovine (bGH), ladite forme naturelle de l'hormone de croissance bovine étant identique à la séquence mutante de l'hormone de croissance bovine décrite sur la Figure 1, à l'exception de la présence d'une glycine au lieu de l'arginine présentée sur la position 119 de la séquence mutants,
où l'hélice alpha mutante diffère de la troisième hélice alpha de l'hormone de croissance du vertébré, au moins sur la position correspondant à la position 119 de ladite forme naturelle de l'hormone de croissance bovine.

36. Animal transgénique non-humain selon la revendication 35, dans lequel une pluralité de cellules comprennent une molécule d'ADN recombinant selon l'une quelconque des revendications 30 à 32.

37. Animal non-humain selon la revendication 35 ou 36, qui présente une vitesse de croissance réduite par comparaison à des animaux témoins.

38. Composition pharmaceutique comprenant un peptide ou une protéine tels que définis dans l'une quelconque des revendications 1 à 28 et un excipient acceptable d'un point de vue pharmaceutique.

39. Peptide ou protéine selon l'uns quelconque des revendications 1 à 29, pour utilisation en tant que médicament.

40. Molécule d'ADN recombinant comprenant un gène contenant une séquence codent pour un peptide ou une protéine ayant au moins 11 acides aminés et ayant une activité inhibitrice de croissance chez un vertébré, et comprenant une hélice alpha mutante, qui est essentiellement homologue de la troisième hélice alpha de l'hormone de croissance d'un vertébré, mais n'y est pas identique, ladite troisième hélice alpha correspondant à l'hélice alpha formée par les acides aminés sur les positions 109 à 126 de la forme naturelle de l'hormone de croissance bovine (bGH), ladite forme naturelle de l'hormone de croissance bovine étant identique à la séquence mutante de l'hormone de croissance bovine décrite sur la Figure 1, à l'exception de la présence d'une glycine au lieu de l'arginine présentée sur la position 119 de la séquence mutante,
où l'hélice alpha mutante diffère de la troisième hélice alpha de l'hormone de croissance du vertébré, au moins sur la position correspondant à la position 119 de ladite forme naturelle de l'hormone de croissance bovine,
pour utilisation en tant que médicament.

41. Molécule d'ADN recombinant selon l'une quelconque des revendications 30 à 32, pour utilisation en tant que médicament.

42. Molécule d'ADN recombinant selon la revendication 40 ou 41, pour inhiber ou réduire l'activité d'hormone de croissance chez un sujet humain ou animal.

43. Molécule d'ADN recombinant selon l'une quelconque des revendications 40 à 42, grâce à laquelle un sujet humain ou animal génétiquement modifié tel qu'obtenu est capable d'exprimer la protéine ou le peptide selon l'une quelconque des revendications 1 à 27 à un niveau inhibiteur de croissance.

44. Procédé de préparation d'un médicament destiné au traitement d'un état pouvant être amélioré par inhibition ou réduction de l'activité de l'hormone de croissance, caractérisé par l'utilisation d'un peptide ou d'une protéine ayant au moins 11 acides aminés et ayant une activité inhibitrice de croissance chez un vertébré, et comprenant une hélice alpha mutante, qui est essentiellement homologue de la troisième hélice alpha de l'hormone de croissance d'un vertébré, mais n'y est pas identique, ladite troisième hélice alpha correspondant à l'hélice alpha formée par les acides aminés sur les positions 109 à 126 de la forme naturelle de l'hormone de croissance bovine (bGH), ladite forme naturelle de l'hormone de croissance bovine étant identique à la séquence mutante de l'hormone de croissance bovine décrits sur la Figure 1, à l'exception de la présence d'une glycine au lieu de l'arginine présentée sur la position 119 de la séquence mutante,
où l'hélice alpha mutante diffère de la troisième hélice alpha de l'hormone de croissance du vertébré, au moins sur la position correspondant à la position 119 de ladite forme naturelle de l'hormone de croissance bovins,

45. Procédé de préparation d'un médicament destiné au traitement d'un état pouvant être amélioré par inhibition ou réduction de l'activité de l'hormone de croissance, caractérisé par utilisation d'un peptide ou d'une protéine selon l'une quelconque des revendications 1 à 28.

46. Utilisation d'un peptide ou d'une protéine ayant au moins 11 acides aminés et ayant une activité inhibitrice de croissance chez un vertébré, et comprenant une hélice alpha mutante, qui est essentiellement homologue de la troisième hélice alpha de l'hormone de croissance d'un vertébré, mais n'y est pas identique, ladite troisième hélice alpha correspondant à l'hélice alpha formée par les acides aminés sur les positions 109 à 126 de la forme naturelle de l'hormone de croissance bovine (bGH), ladite forme naturelle de l'hormone de croissance bovine étant identique à la séquence mutante de l'hormone de croissance bovine décrite sur la Figure 1, à l'exception de la présence d'une glycine au lieu de l'arginine présentée sur la position 119 de la séquence mutante,
où l'hélice alpha mutante diffère de la troisième hélice alpha de l'hormone de croissance du vertébré, au moins sur la position correspondant à la position 119 de ladite forme naturelle de l'hormone de croissance bovine,
pour préparer un médicament destiné au traitement d'un état pouvant être amélioré par inhibition ou réduction de l'activité de l'hormone de croissance.

47. Procédé de préparation d'une composition destinée au traitement d'un état pouvant être amélioré par inhibition ou réduction de l'activité de l'hormone de croissance, caractérisé par l'utilisation d'une molécule d'ADN recombinant selon l'une quelconque des revendications 30 à 32.

48. Utilisation d'une molécule d'ADN recombinant comprenant un gène contenant une séquence codant pour un peptide ou une protéine ayant au moins 11 acides aminés et ayant une activité inhibitrice de croissance chez un vertébré, et comprenant une hélice alpha mutante, qui est essentiellement homologue de la troisième hélice alpha de l'hormone de croissance d'un vertébré, mais n'y est pas identique, ladite troisième hélice alpha correspondant à l'hélice alpha formée par les acides aminés sur les positions 109 à 126 de la forme naturelle de l'hormone de croissance bovine (bGH), ladite forme naturelle de l'hormone de croissance bovine étant identique à la séquence mutante de l'hormone de croissance bovine décrite sur la Figure 1, à l'exception de la présence d'une glycine au lieu de l'arginine présentée sur la position 119 de la séquence mutante,
où l'hélice alpha mutante diffère de la troisième hélice alpha de l'hormone de croissance du vertébré, au moins sur la position correspondant à la position 119 de ladite forme naturelle de l'hormone de croissance bovine,
pour préparer une composition destinée au traitement d'un état pouvant être amélioré par inhibition ou réduction de l'activité de l'hormone de croissance.

49. Peptide ou protéine isolé ayant au moins 11 acides aminés, ayant une activité inhibitrice de croissance chez un vertébré, et comprenant une hélice alpha mutante, qui est essentiellement homologue de la troisième hélice alpha de l'hormone de croissance d'un vertébré, mats n'y est pas identique, ladite troisième hélice alpha correspondant à l'hélice alpha formée par les acides aminés sur les positions 109 à 126 de la forme naturelle de l'hormone de croissance bovine (bGH), ladite forme naturelle de l'hormone de croissance bovine étant identique à la séquence mutante de l'hormone de croissance bovine décrite sur la Figure 1, à l'exception de la présence d'une glycine au lieu de l'arginine présentée sur la position 119 de la séquence mutante,
où l'hélice alpha mutante diffère de la troisième hélice alpha de l'hormone de croissance du vertébré, au moins sur la position correspondant à la position 119 de ladite forme naturelle de l'hormone de croissance bovine,
à la condition que la protéine ou le peptide ne soit
(a) ni la somatotrophine bovine ayant la séquence d'acides aminés du Tableau 1 de la demanda WO 91/00870 (= D1), mais dont une glycine sur la position 119 est remplacée par une proline ;
(b) ni un mutant, hPRL(111-129), de l'hormone de croissance humaine native complète (hGH) ayant la séquence présentée sur la Figure 1 de Cunningham et al., Science 243:1330-1336, 1989 (= D7), ce mutant n'en différant que par les 12 substitutions indiquées pour le hPRL(111-129) du Tableau 1 de D7.

50. Peptide ou protéine selon la revendication 49, dans lequel le peptide ou la protéine a les propriétés présentées an premier dans l'une quelconque des revendications 2 à 28.

51. Peptide ou protéine selon la revendication 1, dans lequel l'acide aminé sur la position correspondant à la position 119 de ladite forme naturelle de l'hormone de croissance bovine est un acide aminé hydrophobe, selon l'échelle de Hopp et Woods, PNAS (USA) 78:3824-3828 (1981).

52. Peptide ou protéine selon la revendication 1, dans lequel le caractère amphiphile de l'hélice alpha mutante, tel que déterminé par la méthode de Margalit et al., J Immunol. 138:2213-2229 (1987) est réduit ou inchangé par rapport au caractère amphiphile de la troisième hélice alpha sur les positions 109 à 126 de ladite hormone de croissante bovine.

53. Peptide ou protéine selon la revendication 51 ou 52, pour utilisation en tant que médicament.

54. Utilisation du peptide ou de la protéine selon la revendication 51 ou 52 pour préparer un médicament destiné au traitement d'un état pouvant être amélioré par inhibition ou réduction de l'activité de l'hormone de croissance.

55. Procédé de préparation d'une composition pharmaceutique, qui consiste à mettre en mélange un peptide ou une protéine selon la revendication 51 ou 52 et un excipient acceptable d'un point de vue pharmaceutique.
